# EUROPEAN PATENT SPECIFICATION

(11) **EP 0 780 122 B1**
(45) Date of publication and mention of the grant of the patent: **22.11.2006**
(21) Application number: 95930716.6
(22) Date of filing: 07.09.1995
(51) Int. Cl.: A61K 31/12, A01N 31/06, C08F 2/38, C08K 5/08, C09K 3/00, C09K 15/08, C11D 3/20, C07C 49/603

(54) **DEPRESSANT OF FUNCTIONS DEVELOPED BY MOLECULE**
MITTEL ZUM HERABSETZEN VON MOLEKÜLFUNKTIONEN
DEPRESSEUR DE FONCTIONS DEVELOPPEES PAR UNE MOLECULE

(30) Priority: 09.09.1994 JP 25266094
(43) Date of publication of application: 25.06.1997
(73) Proprietor: Koyama, Shozo, Nagano 390-02 (JP)
(72) Inventor: KOYAMA, Shozo, Matsumoto-shi, Nagano 390-02 (JP); YAMAGUCHI, Yoshihiro, Matsumoto-shi Nagano 390 (JP)
(74) Representative: Liedl, Christine
(86) International application number: PCT/JP1995/001783
(87) International publication number: WO 1996/007403

(56) References cited:
- EP-A- 0 282 643
- EP-A- 0 613 680
- GB-A- 2 180 847
- JP-A- 1 135 711
- JP-A- 1 172 437
- JP-A- 3 255 446
- JP-A- 6 186 545
- JP-A- 6 194 790
- JP-A- 6 220 489
- JP-A- 6 336 599
- JP-A- 7 010 728
- JP-A- 51 054 639
- JP-A- 57 100 423
- JP-A- 62 172 033
- JP-A- 62 289 525
- JP-B- 36 009 993
- JP-T- 5 502 243
- JP-T- 5 507 300
- JP-T- 58 502 215
- JP-T- 63 501 011
- SU-A- 1 363 752
- US-A- 4 481 277
- BEILSTEIN INFORMATION SERVICE FILE: CROSSFIRE, XP002059402
- J. JANSSEN: "Synthese von 4,4,4',4'-Tetramethyl..." CHEM. BERICHTE, vol. 115, no. 3, 1982, pages 1234-1243, XP002059403
- SH. KOYAMA ET AL.: "A new Substance (Yoshixol) with interesting...." GENERAL PHARMACOLOGY, vol. 28, no. 5, 1997, pages 797-804, XP002059404
- SH. KOYAMA ET AL.: "Apoptosis-like ..." GENERAL PHARMACOLOGY, vol. 28, no. 5, 1997, pages 805-811, XP002059405
- BEILSTEIN INFORMATION SERVICE: CROSSFIRE, XP002072082
- CHEM. BER., Vol. 115, (1982), p. 1234-1243.
- FARMAKOL. TOKSIKOL., (Moscow), Vol. 29, No. 5, (1966), p. 597-600.

## Description

The present invention is related to the use of substituted 4,4'-dimethyl-2-cyclohexene-1-one compounds corresponding to the following general formulas (2), (3-a) and (3-b) for the manufacture of a preparation to be used in the treatment as one ore more agent(s) each having a distinctive function:

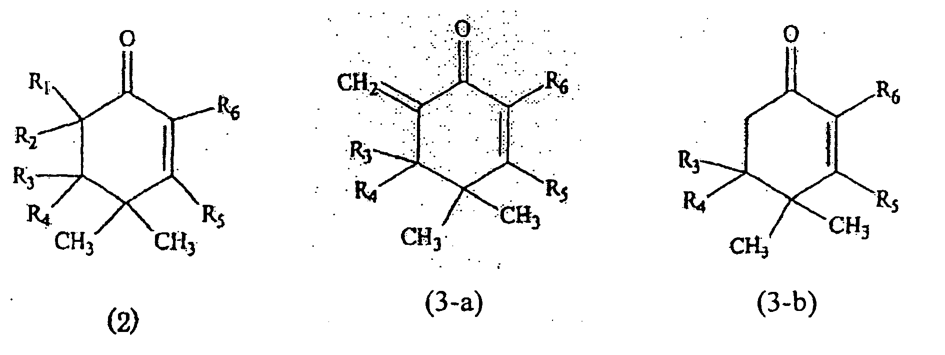

Details of the substituents R₁ to R₆ may be taken from the claim structure.

Especially, the present invention is concerned with the use of 4,4-dimethyl-6-methylene-2-cyclohexene-1-one having a structure according to the following general formula (3-a-1) for the manufacture of a preparation to be used in the treatment as one or more agent(s) each having a distinctive function. This compound (3-a-1) is also termed -"Yoshixol".

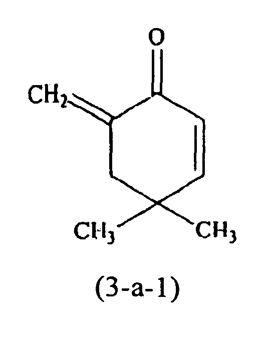

4,4-Dimethyl-6-methylene-2-cyclohexene-1-one and its manufacture is known from a scientific essay authored by Johann Janssen and Wolfgang Lüttke and published in Chem. Ber. 115, 1234 - 1244, especially page 1241 (1981). This essay does not mention specific functions and properties of said compound.

The non-substituted compound 4,4-dimethyl-2-cyclohexene-1-one corresponding to general formula (3-b) when all the substituents R3, R4, R5 and R6 are representing hydrogen is disclosed in several documents such as Japanese patent S 50-105841, Japanese patent S 51-105038, Japanese patent H 4-316531, US patent 4 081 458, US patent 5 169 993 and Swiss patent 603 071; here, function and use of this compound as antifungal agent, as antiandrogen agent, as fragrant agent and reagents having optical activity is mentioned.

Further, compounds having the same but substituted basic 4,4-dimethyl-2-cyclohexene-1-one structure are for example disclosed in document GB 2 193 091 A, here compound no. 16 on page 6, or in document EP 0 613 680 A1, here compound I according to general formula (I) on page 2, or in document WO 92/04432, here compounds having a structure according to general formulas IV, V, VI and VII of claim 1, on page 34.

The inventors as denominated to the present patent have searched for and have found specific functions and properties of 4,4-dimethyl-2-cyclohexene-1-one ("Yoshixol") and related compounds. These results have led to the present invention.

Accordingly, it is the technical problem of the present invention to state beneficial kinds of use of substituted 4,4-dimethyl-6-methylene-2-cyclohexene-1-one compounds corresponds to the above-stated general formulas (2), (3-a), (3-a-1) and (3-b) when used for the manufacture of a preparation to be used in the treatment as one or more agent(s) each having a distinctive function.

The solution of the above-stated technical problem according to the present invention is the use as stated in anyone of the claims 1 to 4.

In the following, the present invention is explained in more detail.

A first aspect of the present invention is related to the use of a substituted 4,4-dimethyl-2-cyclohexene-1-one compound represented by the following general formula (2) for the manufacture of a preparation to be used in the treatment as an antibacterial agent, an antifungal agent, an antiviral agent, a bactericidal and/or sterilizing agent, an anticancer agent and/or antitumor agent, an anticoagulant and/or antifibrinolytic agent, a thrombolytic agent, a metabolism (lipids, sugar, proteins) - improving agent, a promoting agent for wound healing and epithelialization-promoting agent (including hair restoration effect), against arteriosclerosis, against antigen-antibody reaction, and/or to be used in an organ and tissue preservative, an antiseptic and/or preservative, a labeled reagent that has a labeled substance in at least one substituent, which labelled substance indicates a targeted position of generating function of molecule, a reductant, a free radical scavenger, a desulfurization agent, a depolymerization agent, an agent for improving functional and/or physical properties of surfactants, a spermatocidal agent or contraceptive agent for external use, a confirmation altering agent of saccharide chains, a phase transition agent or a phase transition-improving agent, an improving agent of microphase separation structure, a plasticizer or plasticity and/or elasticity-improving agent, a copolymerization agent or a copolymerization-improving agent, a polymerization regulator or an improving agent of polymerization adjustment, a stabilizer or a stabilization-improving agent, an antioxidant or an oxidation-preventing agent, an improving agent of crystallized materials and/or amorphous materials, a fluidability-improving agent, a softener or an improving agent of softeners and/or flexibility improving agent, a modulation and/or improving agent fluorescent wavelength and/or excitation wavelength pigments, coating materials, paints or colorants, an improving agent of functional and/or physical properties of low molecular substances, an improving agent of physical properties and functions of macromolecular substances, and/or a physical property-improving agent of macromolecular composite materials and functional macromolecular composite materials: 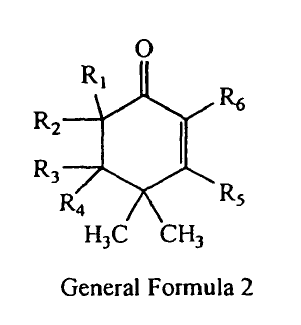 wherein
(i) R1 and R2 both together represent a methylene group and/or
   R1, R2, R3, R4, R5 and R6 represent independently hydrogen atom; halogen atom; C1-C6 alkyl group; amidino group; C3-C8 cycloalkyl group; C1-C6 alkoxy C1-C6 alkyl group; aryl group; allyl group; aralkyl group in which one or more C1-C6 alkyl groups are bound to an aromatic ring selected from the group consisting of benzene, naphthalene and anthracene ring; C1-C6 alkylene group; benzoyl group; cinnamyl group; cinnamoyl group or furoyl group;
(ii) one or more of R1, R2, R3 and R4, and/or one or more of R5 and R6 may be substituted or non-substituted cyclopentyl group; substituted or non-substituted cyclohexyl group; or substituted or non-substituted naphthyl group;
(iii) R5 and R6 may form a ring by binding with another condensation polycyclic hydrocarbon compound or heterocyclic compound;
(iv) one or more of R3, R4, R5 and R6 may be substituted by one or more of substituents selected from the group consisting of halogen atom, cyano group, protected or non-protected carboxyl group, protected or non-protected hydroxyl group, protected or non-protected amino group, C1-C6 alkyl group, C1-C6 alkoxy group, C1-C7 alkoxy carbonyl group, aryl group, C3-C6 cycloalkyl group, C1-C6 acylamino group, C1-C6 acyloxy group, C2-C6 alkenyl group, C1-C6 trihalogenoalkyl group, C1-C6 alkylamino group, and C1-C6 dialkylamino group;
(v) R2 and/or R5 may be substituted by one or more substituents selected from the group consisting of halogen atom, C1-C6 alkyl group, protected or non-protected carboxyl group, protected or non-protected hydroxyl group, protected or non-protected amino group, protected or non-protected C1-C6 alkylamino group, protected or non-protected C1-C6 aminoalkyl group, protected or non-protected C1-C6 alkylamino C1-C6 alkyl group, protected or non-protected hydroxyalkyl group, and C3-C6 cycloalkylamino group;
   wherein
(vi) when one or more of R3, R4, R5 and R6 are alkyl groups, terminal end(s) of the alkyl group(s) may be substituted by C3-C8 cycloalkyl group);
   said aryl group in (i) and (iv) is phenyl, tolyl, xylyl or naphthyl group; said substituted cyclopentyl group in (ii) is cyclopentylamino group or cyclopentylcarbinol group; said substituted cyclohexyl group in (ii) is cyclohexylamino group, cyclohexylaldehyde group or cyclohexyl acetic acid group; and said substituted naphthyl group in (ii) is naphthylamino group or naphthylamino sulfonic acid group; and said condensation polycyclic hydrocarbon compound in (iii) is pentalene, indene, naphthalene, azulene, heptalene, biphenylene, indacene, acenaphthylene, fluorene, phenalene, phenanthrene, anthracene, pentacene, hexacene, dibenzophenanthrene, 1H-cyclopentacyclooctene or benzocyclooctene, and the heterocyclic compound may be furan, thiophene, pyrrole, γ-pyran, γ-thiopyran, pyridine, thiazole, imidazole pyrimidine, indole or quinoline.

A second aspect of the present invention is related to the use of a substituted 4,4-dimethyl-2-cyclohexene-1-one compound represented by the following general formula (3-a), and a third aspect of the present invention is related to the use of a substituted 4,4-dimethyl-2-cyclohexene-1-one compound represented by the following general formula (3-b), both, for the manufacture of a preparation to be used in the treatment as an antibacterial agent, an antifungal agent, an antiviral agent, a bactericidal and/or sterilizing agent, an anticancer agent and/or antitumor agent, an anticoagulant and/or antifibrinolytic agent, a thrombolytic agent, a metabolism (lipids, sugar, proteins)- improving agent , a promoting agent for wound healing epithelialization-promoting agent (including hair restoration effect), against arteriosclerosis , against antigen-antibody reaction, and/or to be used in an organ and tissue preservative, an antiseptic and/or preservative, a labeled reagent that has a labeled substance in at least one substituent, which labeled substance indicates a targeted position of generating function of molecule, a reductant, a free radical scavenger, a desulfurization agent, a depolymerization agent, an agent for improving functional and/or physical properties of surfactants, a spermatocidal agent or contraceptive agent for external use, a conformation altering agent of saccharide chains, a phase transition agent or a phase transition-improving agent, an improving agent of microphase separation structure, a plasticizer or plasticity and/or elasticity-improving agent, a copolymerization agent or a copolymerization-improving agent, a polymerization regulator or an improving agent of polymerization adjustment, a stabilizer or a stabilization-improving agent, an antioxidant or an oxidation-preventing agent, an improving agent of crystallized materials and/or amorphous materials, a fluidability-improving agent, a softener or an improving agent of softeners and/or flexibility improving agent, a modulation and/or improving agent fluorescent Wavelength and/or excitation wavelength pigments, coating materials, paints or colorants, an improving agent of functional and/or physical properties of low molecular substances, an improving agent of physical properties and functions of macromolecular substances, and/or a physical property-improving agent of macromolecular composite materials and functional macromolecular composite materials: 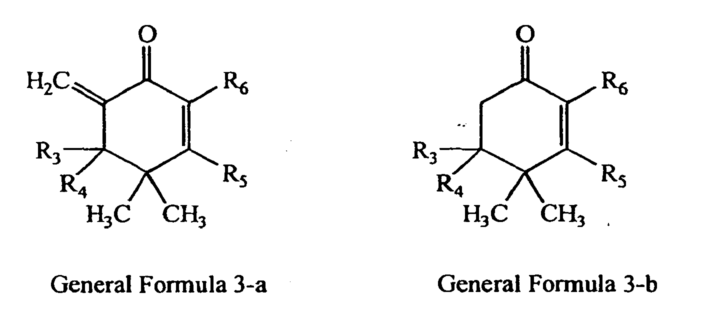 wherein
(i) R3, R4, R5 and R6 represent independently hydrogen atom; halogen atom; C1-C6 alkyl group; amidino group; C3-C8 cycloalkyl group; C1-C6 alkoxy C1-C6 alkyl group; aryl group; allyl group; aralkyl group in which one or more C1-C6 alkyl groups are bound to an aromatic ring selected from the group consisting of benzene, naphthalene and anthracene ring; C1-C6 alkylene group; benzoyl group; cinnamyl group; cinnamoyl group or furoyl group;
(ii) one or more of R3 and R4, and/or one or more of R5 and R6 may be substituted or non-substituted cyclopentyl group; substituted or non-substituted cyclohexyl group; or substituted or non-substituted naphthyl group;
(iii) R5 and R6 may form a ring by binding with another condensation polycyclic hydrocarbon compound or heterocyclic compound;
(iv) one or more of R3, R4, R5 and R6 may be substituted by one or more of substituents selected from the group consisting of halogen atom, cyano group, protected or non-protected carboxyl group, protected or non-protected hydroxyl group, protected or non-protected amino group, C1-C6 alkyl group, C1-C6 alkoxy group, C1-C7 alkoxy carbonyl group, aryl group, C3-C6 cycloalkyl group, C1-C6 acylamino group, C1-C6 acyloxy group, C2-C6 alkenyl group, C1-C6 trihalogenoalkyl group, C1-C6 alkylamino group, and C1-C6 dialkylamino group;
(v) R5 may be substituted by one or more substituents selected from the group consisting of halogen atom, C1-C6 alkyl group, protected or non-protected carboxyl group, protected or non-protected hydroxyl group, protected or non-protected amino group, protected or non-protected C1-C6 alkylamino group, protected or non-protected C1-C6 aminoalkyl group, protected or non-protected C1-C6 alkylamino C1-C6 alkyl group, protected or non-protected hydroxyalkyl group, and C3-C6 cycloalkylamino group;
(vi) when one or more of R3, R4, R5 and R6 are alkyl groups, terminal end(s) of the alkyl group(s) may be substituted by C3-C8 cycloalkyl group);
   wherein
   said aryl group in (i) and (iv) is phenyl, tolyl, xylyl or naphthyl group;
   said substituted cyclopentyl group in (ii) is cyclopentylamino group or cyclopentylcarbinol group, and said substituted cyclohexyl group in (ii) is cyclohexylamino group, cyclohexylaldehyde group or cyclohexyl acetic acid group; and said substituted naphthyl group in (ii) is naphthylamino group or naphthylamino sulfonic acid group;
   said and condensation polycyclic hydrocarbon compound in (iii) is pentalene, indene, naphthalene, azulene, heptalene, biphenylene, indacene, acenaphthylene, fluorene, phenalene, phenanthrene, anthracene, pentacene, hexacene, dibenzophenanthrene, 1H-cyclopentacyclooctene or benzocyclooctene, and the heterocyclic compound may be furan, thiophene, pyrrole, γ-pyran, γ-thiopyran, pyridine, thiazole, imidazole pyrimidine, indole or quinoline.

A fourth aspect of the present invention is related to the use of a substituted 4,4-dimethyl-2-cyclohexene-1-one compound represented by the following formula (3-a-1) for the manufacture of a preparation to be used in the treatment as an antibacterial agent, an antifungal agent, an antiviral agent, a bactericidal and/or sterilizing agent, an anticancer agent and/or antitumor agent, an anticoagulant and/or antifibrinolytic agent, a thrombolytic agent, a metabolism (lipids, sugar, proteins)- improving agent, a promoting agent for wound healing epithelialization-promoting agent (including hair restoration effect), against arteriosclerosis , against antigen-antibody reaction, and/or to be used in an organ and tissue preservative, an antiseptic and/or preservative, a labeled reagent that has a labeled substance in at least one substituent, which labeled substance indicates a targeted position of generating function of molecule, a reductant, a free radical scavenger, a desulfurization agent, a depolymerization agent, an agent for improving functional and/or physical properties of surfactants, a spermatocidal agent or contraceptive agent for external use, a conformation altering agent of saccharide chains, a phase transition agent or a phase transition-improving agent, an improving agent of microphase separation structure, a plasticizer or plasticity and/or elasticity-improving agent, a copolymerization agent or a copolymerization-improving agent, a polymerization regulator or an improving agent of polymerization adjustment, a stabilizer or a stabilization-improving agent, an antioxidant or an oxidation-preventing agent, an improving agent of crystallized materials and/or amorphous materials, a fluidability-improving agent, a softener or an improving agent of softeners and/or flexibility improving agent, a modulation and/or improving agent fluorescent wavelength and/or excitation wavelength pigments, coating materials, paints or colorants, an improving agent of functional and/or physical properties of low molecular substances, an improving agent of physical properties and functions of macromolecular substances, and/or a physical property-improving agent of macromolecular composite materials and functional macromolecular composite materials:

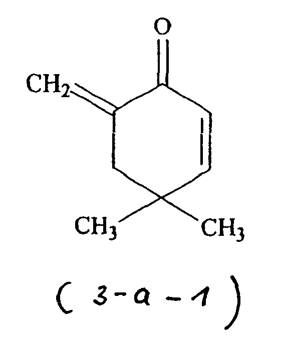

In the present specification, unless otherwise specified, the term "halogen atom" means, for example, fluorine atom, chlorine atom, bromine atom or iodine atom; the term "alkyl group" means Cl-10 alkyl group such as methyl group, ethyl group, n-propyl group, isopropyl group, n-butyl group, isobutyl group, sec-butyl group, tort-butyl group, benzyl group, hexyl group, octyl group or the like; the term "lower alkyl group" means C1-5 alkyl group among the alkyl groups mentioned above; the term "alkoxy group" means -O-alkyl group (alkyl group is C1-10 alkyl group mentioned above); the term "lower alkylamino group" means C1-5 alkylamino group such as methylamino group, ethylamino group, propylamino group or the like; the term "di-lower alkylamino group" means C1-5 dialkylamino group such as dimethylamino group; the term "lower alkenyl group" means C2-5 alkenyl group such as vinyl group, allyl group, 1-propenyl group, 1-butenyl group or the like; the term "cycloalkyl group" means C3-6 cycloalkyl group such as cyclopropyl, cyclobutyl, cyclopentyl, cyclohexyl or the like; the term "aryl group" means, for example, phenyl group or naphthyl group; the term "alkoxy carbonyl group" means -COO-alkyl group (alkyl group means C1-10 alkyl group above mentioned); the term "hydroxy lower alkyl group" means hydroxy-C1-5 alkyl group such as hydroxy methyl group, hydroxy ethyl group, hydroxy propyl group or the like; the term "amino lower alkyl group" means amino Cl-5 alkyl group such as aminomethyl group, aminoethyl group, aminopropyl group or the like; the term "lower alkylamino lower alkyl group" means C1-5 alkylamino C1-5 alkyl group such as methylaminomethyl group, ethylaminomethyl group, ethylaminoethyl group or the like; the term "di-lower alkylamino lower alkyl group" means C1-5 dialkylamino C1-5 alkyl group such as dimethylaminomethyl group or diethylaminomethyl group; the term "cyclic amino group" means cyclic amino group with 4-10 membered ring such as piperazinyl group, morpholinyl group, 1,4-diazabicyclo (3,2,1) octyl group or the like; the term "cyclic amino lower alkyl group" means C1-5 alkyl group attached to cyclic amino group with 4-6 membered ring such as 1-piperazinylmethyl group, 1-pyrrolidinylmethyl group, 1-azethydinylmethyl group, 1-morpholinylmethyl group or the like; the term "acylamino group" means C1-4 acylamino group such as formylamino group, acetylamino group, propionylamino group, butyrylamino group or the like; the term "acyloxy group" means C1-4 acyloxy group such as formyloxy group, acetyloxy group, propionyloxy group, butyryloxy group or the like; the term "trihalogeno-lower alkyl group" means trihalogeno C1-5 alkyl group such as trichloromethyl group, trifluoromethyl group or the like; the term "heterocyclic group" means 5 membered ring, 6 membered ring or those condensation rings (such as furyl, propyl, thienyl, oxazolyl, imidazolyl, thiazolyl, 1-pyrrolinyl, benzofuryl, benzothiazolyl, pyridyl, quinolyl, pyrimidinyl or morpholinyl group as an example) which has one or more atoms selected from the group consisting of oxygen atom, nitrogen atom and sulfate atom.

The alkyl group represented by R3, R4, R5 or R6 in each general formula may be either straight or branched alkyl group such as, for example, methyl group, ethyl group, propyl group, isopropyl group, butyl isobutyl group, sec-butyl group, 1-butyl group, pentyl group, isopentyl group, neopentyl group or hexyl group which are lower alkyl groups (C1-4). In addition, terminal end of these alkyl groups can be bound to lower cycloalkyl group (C3-4) such as cyclopropyl methyl group, cyclobutyl ethyl group, cyclopentyl methyl group or the like.

Lower cycloalkyl groups (C3-4) included in the cycloalkyl group represented by R3, R4, R5 and R6 in each general formula may be, for example, cyclopropyl group, cyclobutyl group, cyclopentyl group or cyclohexyl group. The alkoxyalkyl group may be, for example, ethyl group, methyl group, methoxyethyl group, ethoxyethyl group, propoxyethyl group, isopropoxyethyl group, butoxyethyl group, methoxypropyl group, 2-ethoxy-1-methyl ethyl group or the like.

Straight or branched alkylene group represented by R3, R4, R5 or R6 in each general formula may be, for example, methylene group, ethylene group, trimethylene group, tetramethylene group, 1,2-dimethylethylene group or the like.

Each substituent of R3, R4, R5 and R6 in each general formula or the methylene group in the general formula 3-a may be at least one substituent selected from the group consisting of halogen atom, cyano group, protected or non-protected carboxyl group, protected or non-protected hydroxyl group, protected or non-protected amino group, alkyl group, alkoxy group, alkoxy carbonyl group, aryl group, cycloalkyl group, acylamino group, acyloxy group, lower alkenyl group, trihalogeno-lower alkyl group, lower alkylamino group, di-lower alkylamino group and the like; R2 and/or R5 may be substituted by at least one substituent selected from the group consisting of halogen atom, lower alkyl group, protected or non-protected carboxyl group, protected or non-protected hydroxyl group, protected or non-protected amino group, protected or non-protected lower alkylamino group, protected or non-protected amino lower alkyl group, protected or non-protected lower alkylamino lower alkyl group, protected or non-protected hydroxy lower alkyl group, di-lower alkylamino group, di-lower alkylamino lower alkyl group or cyclic amino lower alkyl group.

Examples of the protecting substituents of carboxyl group include pharmaceutically acceptable protecting groups of carboxyl group, such as an ester-forming group which is easily detached in an organism.

Moreover, protecting substituent of amino group, amino lower alkyl group; lower alkylamino group, and lower alkylamino lower alkyl group may be a pharmaceutically acceptable amino-protecting group which is easily detached in an organism.

In addition, protecting substituent of hydroxyl group and hydroxy lower alkyl group may be a pharmaceutically acceptable protecting group of hydroxyl group which is easily detached in an organism.

Though halogen compounds can be added to the compositions according to this invention, it is necessary to give heed to an existence of toxicity which the combined compounds may have. By adding halogen compounds, coloring due to light can be prevented. Halogen compounds which can be applied to the compositions according to this invention, for example, are halogenated alkali metals such as potassium bromide, sodium bromide, potassium chloride, sodium chloride, potassium iodide and sodium iodide, and halogenated alkaline-earth metal such as calcium bromide, magnesium bromide, calcium chloride and magnesium chloride, and halogenated zinc such as zinc bromide and zinc chloride.

In addition, in the present specification, unless otherwise specified, or except for the case which is clear from a context, the term "alkyl group" includes straight alkyl group as well as branched one. Similarly, the alkyl group in "alkoxy group", "aralkyl group" and "alkylamino group" which has alkyl group includes straight alkyl group as well as branched one "Cycloalkyl group" is also similar to the above, and includes branched groups such as ethyl cyclopentyl group and methyl cyclohexyl group.

The compounds mentioned in this document may be directly applied on the surface of infected wound such as burn and decubitus. And, those compounds may be used by combining with carrier substances which are acceptable for pharmaceutical use.

The carrier substances which are acceptable for pharmaceutical include biologically acceptable carrier substances such as polyoxyalkylenealkyl ether, polyoxyethylene sorbitan fatty acid ester, polyvinylpyrrolidone, hydrocarbon, paraffin, alcohol, polyvalent alcohol, alcohol ester, polyalcohol ester, fatty acid and metal salts of fatty acid; moreover, chitosan, polyethylene glycol, polyethylene glycerin fatty acid ester (caprylic acid, capric acid, lauric acid) can be exemplified.

In addition, when the compounds mentioned in this document are used as combined substances common with the pharmaceutically acceptable carrier substances these may be applied in many kinds such as cream agents, ointments, pastes, poultices, milky lotions, suspensions, liniments, lotions, aerosol agents, solutions and tapes corresponding to prospected treatments. Also, it is allowed to add solvent supporting agents, isotonic changing agents, pH adjusters, deodorants, antiseptics or odorants. The here mentioned compounds may be used alone, or may be used together with acid addition salts, emulsifiers, ester agents or polymerization agents, unless electric charge distribution and electric charge density of the molecules are changed drastically. It can be used in the following form as an example; non-toxic and pharmaceutically acceptable acid addition salts of the compounds which are provided in chemical formula (2), (3-a) and (3-b) including salts of inorganic acids such as hydrochloric acid, hydrobromic acid, phosphoric acid or sulfuric acid and further salts of organic acids such as acetic acid, citric acid, tartaric acid, lactic acid, succinic acid, fumaric acid, maleic acid or methasulfonic acid.

Representative compounds corresponding to the above-stated general formula (2) include for example:
(15) 4,4,6-trimethyl-2-cyclohexen-1-one,
(16) 4,4-dimethyl-6-ethyl-2-cyclohexen-1-one,
(17) 4,4-dimethyl-6-propyl-2-cyclohexen-1-one,
(18) 4,4-dimethyl-6-isopropyl-2-cyclohexen-1-one,
(19) 6-butyl-4,4-dimethyl-2-cyclohexen-1-one,
(20) 4,4-dimethyl-6-isobutyl-2-cyclohexen-1-one,
(21) 6-benzyl-4,4-dimethyl-2-cyclohexen-1-one,
(22) 4,4-dimethyl-6-hexyl-2-cyclohexen-1-one,
(23) 4,4-dimethyl-6-octyl-2-cyclohexen-1-one,
(24) 6-pentyloxy-4,4-dimethyl-2-cyclohexen-1-one,
(25) 4,4-dimethyl-6-hexyloxy-2-cyclohexen-1-one,
(26) 4,4-dimethyl-6-methylamino-2-cyclohexen-1-one,
(27) 4,4-dimethyl-6-ethylamino-2-cyclohexen-1-one,
(28) 4,4-dimethyl-6-propylamino-2-cyclohexen-1-one,
(29) 4,4-dimethyl-6-dimethylamino-2-cyclohexen-1-one,
(30) 6-vinyl-4,4-dimethyl-2-cyclohexen-1-one,
(31) 6-allyl-4,4-dimethyl-2-cyclohexen-1-one,
(32) 4,4-dimethyl-6-isopropenyl-2-cyclohexen-1-one,
(32.1) 6-cyclopropyl-4,4-dimethyl-2-cyclohexen-1-one,
(32.2) 6-cyclobutyl-4,4-dimethyl-2-cyclohexen-1-one,
(32.3) 6-cyclopentyl-4,4-dimethyl-2-cyclohexen-1-one,
(32.4) 6-cyclohexyl-4,4-dimethyl-2-cyclohexen-1-one,
(32.5) 4,4-dimethyl-6-phenyl-2-cyclohexen-1-one,
(32.6) 4,4-dimethyl-6-naphthyl-2-cyclohexen-1-one,
(33) 4,4-dimethyl-6-hydroxymethyl-2-cyclohexen-1-one,
(34) 4,4-dimethyl-6-hydroxyethyl-2-cyclohexen-1-one,
(35) 4,4-dimethyl-6-hydroxypropyl-2-cyclohexen-1-one,
(36) 6-aminomethyl-4,4-dimethyl-2-cyclohexen-1-one,
(37) 6-aminoethyl-4,4-dimethyl-2-cyclohexen-1-one,
(38) 6-aminopropyl-4,4-dimethyl-2-cyclohexen-1-one,
(39) 4,4-dimethyl-6-methylaminomethyl-2-cyclohexen-1-one,
(40) 4,4-dimethyl-6-ethylaminomethyl-2-cyclohexen-1-one,
(41) 4,4-dimethyl-6-ethylaminoethyl-2-cyclohexen-1-one,
(42) 4,4-dimethyl-6-dimethylaminomethyl-2-cyclohexen-1-one,
(43) 4,4-dimethyl-6-diethylaminomethyl-2-cyclohexen-1-one,
(44) 4,4-dimethyl-6-piperazinyl-2-cyclohexen-1-one,
(45) 4,4-dimethyl-6-morpholinyl-2-cyclohexen-1-one,
(46) 4,4-dimethyl-6-piperazinylethyl-2-cyclohexen-1-one,
(47) 4,4-dimethyl-6-pyrrolinylmethyl-2-cyclohexen-1-one,
(48) 6-azethydinylmethyl-4,4-dimethyl-2-cyclohexen-1-one,
(49) 4,4-dimethyl-6-morpholinylmethyl-2-cyclohexen-1-one,
(50) 4,4-dimethyl-6-formylamino-2-cyclohexen-1-one,
(51) 6-acetylamino-4,4-dimethyl-2-cyclohexen-1-one,
(52) 4,4-dimethyl-6-propionylamino-2-cyclohexen-1-one,
(53) 6-butyrylamino-9,4-dimethyl-2-cyclohexen-1-one,
(54) 4,4-dimethyl-6-formyloxy-2-cyclohexen-1-one,
(55) 6-acetyloxy-4,4-dimethyl-2-cyclohexen-1-one,
(56) 4,4-dimethyl-6-propionyloxy-2-cyclohexen-1-one
(57) 6-butyryloxy-4,4-dimethyl-2-cyclohexen-1-one,
(58) 4,4-dimethyl-6-trichloromethyl-2-cyclohexen-1-one,
(59) 4,4-dimethyl-6-trifluoromethyl-2-cyclohexen-1-one,
(60) 4,4-dimethyl-6-furyl-2-cyclohexen-1-one,
(61) 4,4-dimethyl-6-propyl-2-cyclohexen-1-one,
(62) 4,4-dimethyl-6-thienyl-2-cyclohexen-1-one,
(63) 4,4-dimethyl-6-isoxazolyl-2-cyclohexen-1-one,
(64) 4,4-dimethyl-6-imidazolyl-2-cyclohexen-1-one,
(65) 4,4-dimethyl-6-thiazolyl-2-cyclohexen-1-one,
(66) 4,4-dimethyl-6-pyrrolinyl-2-cyclohexen-1-one,
(67) 6-benzofuryl-4,4-dimethyl-2-cyclohexen-1-one,
(68) 6-benzothiazolyl-4,4-dimethyl-2-cyclohexen-1-one,
(69) 6-pyridyl-4,4-dimethyl-2-cyclohexen-1-one,
(70) 4,4-dimethyl-6-quinolyl-2-cyclohexen-1-one,
(71) 4,4-dimethyl-6-pyrimidinyl-2-cyclohexen-1-one.
(72) 4,4-dimethyl-6-morpholinyl-2-cyclohexen-1-one, and the acid addition salts thereof.

Representative compounds corresponding to the above-stated general formula (3-a) include for example:
(73) 6-methylene-4,4,5-trimethyl-2-cyclohexen-1-one,
(74) 4,4-dimethyl-5-ethyl-6-methylene-2-cyclohexen-1-one,
(75) 4,4-dimethyl-5-propyl-6-methylene-2-cyclohexen-1-one,
(76) 4,4-dimethyl-5-isopropyl-6-methylene-2-cyclohexen-1-one,
(77) 5-butyl-4,4-dimethyl-6-methylene-2-cyclohexen-1-one,
(78) 4,4-dimethyl-5-isobutyl-6-methylene-2-cyclohexen-1-one,
(79) 5-benzyl-4,4-dimethyl-6-methylene-2-cyclohexen-1-one,
(80) 4,4-dimethyl-5-hexyl-6-methylene-2-cyclohexen-1-one,
(81) 4,4-dimethyl-5-octyl-6-methylene-2-cyclohexen-1-one,
(82) 5-pentyloxy-4,4-dimethyl-6-methylene-2-cyclohexen-1-one,
(83) 4,4-dimethyl-5-hexyloxy-6-methylene-2-cyclohexen-1-one,
(84) 4,4-dimethyl-5-methylamino-6-methylene-2-cyclohexen-1-one,
(85) 4,4-dimethyl-5-ethylamino-6-methylene-2-cyclohexen-1-one,
(86) 4,4-dimethyl-5-propylamino-6-methylene-2-cyclohexen-1-one,
(87) 4,4-dimethyl-5-dimethylamino-6-methylene-2-cyclohexen-1-one,
(88) 5-vinyl-4,4-dimethyl-6-methylene-2-cyclohexen-1-one,
(89) 5-allyl-4,4-dimethyl-6-methylene-2-cyclohexen-1-one,
(90) 4,4-dimethyl-5-isopropenyl-6-methylene-2-cyclohexen-1-one,
(91) 5-cyclopropyl-4,4-dimethyl-6-methylene-2-cyclohexen-1-one,
(92) 5-cyclobutyl-4,4-dimethyl-6-methylene-2-cyclohexen-1-one,
(93) 5-cyclopentyl-4,4-dimethyl-6-methylene-2-cyclohexen-1-one,
(94) 5-cyclohexyl-4,4-dimethyl-6-methylene-2-cyclohexen-1-one,
(95) 4,4-dimethyl-5-phenyl-6-methylene-2-cyclohexen-1-one,
(96) 4,4-dimethyl-5-naphthyl-6-methylene-2-cyclohexen-1-one,
(98) 4,4-dimethyl-5-hydroxy methyl-6-methylene-2-cyclohexen-1-one,
(99) 4,4-dimethyl-5-hydroxy ethyl-6-methylene-2-cyclohexen-1-one,
(100) 4,4-dimethyl-5-hydroxy propyl-6-methylene-2-cyclohexen-1-one,
(101) 5-aminomethyl-4,4-dimethyl-6-methylene-2-cyclohexen-1-one,
(102) 5-aminoethyl-4,4-dimethyl-6-methylene-2-cyclohexen-1-one,
(103) 5-aminopropyl-4,4-dimethyl-6-methylene-2-cyclohexen-1-one,
(104) 4,4-dimethyl-5-methylaminomethyl-6-methylene-2-cyclohexen-1-one,
(105) 4,4-dimethyl-5-ethylaminomethyl-6-methylene-2-cyclohexen-1-one,
(106) 4,4-dimethyl-5-ethylaminoethyl-6-methylene-2-cyclohexen-1-one,
(107) 4,4-dimethyl-5-dimethylaminomethyl-6-methylene-2-cyclohexen-1-one,
(108) 4,4-dimethyl-5-diethylaminomethyl-6-methylene-2-cyclohexen-1-one,
(109) 4,4-dimethyl-5-piperazinyl-6-methylene-2-cyclohexen-1-one,
(110) 4,4-dimethyl-5-morpholinyl-6-methylene-2-cyclohexen-1-one,
(111) 4,4-dimethyl-5-piperazinyl ethyl-6-methylene-2-cyclohexen-1-one,
(112) 4,4-dimethyl-5-pyrrolinyl methyl-6-methylene-2-cyclohexen-1-one,
(113) 5-azethydinylmethyl-4,4-dimethyl-6-methylene-2-cyclohexen-1-one,
(114) 4,4-dimethyl-5-morpholinylmethyl-6-methylene-2-cyclohexen-1-one,
(115) 4,4-dimethyl-5-formylamino-6-methylene-2-cyclohexen-1-one,
(116) 5-acetylamino-4,4-dimethyl-6-methylene-2-cyclohexen-1-one,
(117) 4,4-dimethyl-5-propionylamino-6-methylene-2-cyclohexen-1-one,
(118) 5-butyrylamino-4,4-dimethyl-6-methylene-2-cyclohexen-1-one,
(119) 4,4-dimethyl-5-formyloxy-6-methylene-2-cyclohexen-1-one,
(120) 5-acetyloxy-4,4-dimethyl-6-methylene-2-cyclohexen-1-one,
(121) 4,4-dimethyl-5-propionyloxy-6-methylene-2-cyclohexen-1-one,
(122) 5-butyryloxy-4,4-dimethyl-6-methylene-2-cyclohexen-1-one,
(123) 4,4-dimethyl-5-trichloromethyl-6-methylene-2-cyclohexen-1-one,
(124) 4,4-dimethyl-5-trifluoromethyl-6-methylene-2-cyclohexen-1-one,
(125) 4,4-dimethyl-5-furyl-6-methylene-2-cyclohexen-1-one,
(126) 4,4-dimethyl-5-propyl-6-methylene-2-cyclohexen-1-one,
(127) 4,4-dimethyl-5-thienyl-6-methylene-2-cyclohexen-1-one,
(128) 4,4-dimethyl-5-isoxazolyl-6-methylene-2-cyclohexen-1-one,
(129) 4,4-dimethyl-5-imidazolyl-6-methylene-2-cyclohexen-1-one,
(130) 4,4-dimethyl-5-thiazolyl-6-methylene-2-cyclohexen-1-one,
(131) 4,4-dimethyl-5-pyrrolinyl-6-methylene-2-cyclohexen-1-one,
(132) 5-benzofuryl-4,4-dimethyl-6-methylene-2-cyclohexen-1-one,
(133) 5-benzothiazolyl-4,4-dimethyl-6-methylene-2-cyclohexen-1-one,
(134) 5-pyridyl-4,4-dimethyl-6-methylene-2-cyclohexen-1-one,
(135) 4,4-dimethyl-5-quinolyl-6-methylene-2-cyclohexen-1-one,
(136) 4,4-dimethyl-5-pyrimidinyl-6-methylene-2-cyclohexen-1-one,
(137) 4,4-dimethyl-5-morpholinyl-6-methylene-2-cyclohexen-1-one,
(138) 3,4,4-trimethyl-6-methylene-2-cyclohexen-1-one,
(139) 4,4-dimethyl-3-ethyl-6-methylene-2-cyclohexen-1-one,
(140) 4,4-dimethyl-6-methylene-3-propyl-2-cyclohexen-1-one,
(141) 4,4-dimethyl-3-isopropyl-6-methylene-2-cyclohexen-1-one,
(142) 3-butyl-4,4-dimethyl-6-methylene-2-cyclohexen-1-one,
(143) 4,4-dimethyl-3-isobutyl-6-methylene-2-cyclohexen-1-one,
(144) 3-benzyl-4,4-dimethyl-6-methylene-2-cyclohexen-1-one,
(145) 4,4-dimethyl-3-hexyl-6-methylene-2-cyclohexen-1-one,
(146) 4,4-dimethyl-6-methylene-3-octyl-2-cyclohexen-1-one,
(147) 3-pentyloxy-4,4-dimethyl-6-methylene-2-cyclohexen-1-one,
(148) 4,4-dimethyl-3-hexyloxy-6-methylene-2-cyclohexen-1-one,
(149) 4,4-dimethyl-3-methylamino-6-methylene-2-cyclohexen-1-one,
(150) 4,4-dimethyl-3-ethylamino-6-methylene-2-cyclohexen-1-one,
(151) 4,4-dimethyl-6-methylene-3-propylamino-2-cyclohexen-1-one,
(152) 4,4-dimethyl-3-dimethylamino-6-methylene-2-cyclohexen-1-one,
(153) 3-vinyl-4,4-dimethyl-6-methylene-2-cyclohexen-1-one
(154) 3-allyl-4,4-dimethyl-6-methylene-2-cyclohexen-1-one,
(155) 4,4-dimethyl-3-isopropenyl-6-methylene-2-cyclohexen-1-one,
(156) 3-cyclopropyl-4,4-dimethyl-6-methylene-2-cyclohexen-1-one,
(157) 3-cyclobutyl-4,4-dimethyl-6-methylene-2-cyclohexen-1-one
(158) 3-cyclopentyl-4,4-dimethyl-6-methylene-2-cyclohexen-1-one,
(159) 3-cyclohexyl-4,4-dimethyl-6-methylene-2-cyclohexen-1-one,
(160) 4,4-dimethyl-3-phenyl-6-methylene-2-cyclohexen-1-one,
(161) 4,4-dimethyl-6-methylene-3-naphthyl-2-cyclohexen-1-one,
(163) 4,4-dimethyl-3-hydroxy methyl-6-methylene-2-cyclohexen-1-one,
(164) 4,4-dimethyl-3-hydroxy ethyl-6-methylene-2-cyclohexen-1-one,
(165) 4,4-dimethyl-3-hydroxy propyl-6-methylene-2-cyclohexen-1-one,
(166) 3-aminomethyl-4,4-dimethyl-6-methylene-2-cyclohexen-1-one,
(167) 3-aminoethyl-4,4-dimethyl-6-methylene-2-cyclohexen-1-one,
(168) 3-aminopropyl-4,4-dimethyl-6-methylene-2-cyclohexen-1-one,
(169) 4,4-dimethyl-3-methylaminomethyl-6-methylene-2-cyclohexen-1-one,
(170) 4,4-dimethyl-3-ethylaminomethyl-6-methylene-2-cyclohexen-1-one,
(171) 4,4-dimethyl-3-ethylaminoethyl-6-methylene-2-cyclohexen-1-one,
(172) 3-dimethylaminomethyl-4,4-dimethyl-6-methylene-2-cyclohexen-1-one,
(173) 3-diethylaminomethyl-4,4-dimethyl-6-methylene-2-cyclohexen-1-one,
(174) 4,4-dimethyl-6-methylene-3-piperazinyl-2-cyclohexen-1-one,
(175) 4,4-dimethyl-6-methylene-3-morpholinyl-2-cyclohexen-1-one,
(176) 4,4-dimethyl-6-methylene-3-piperazinyl ethyl-2-cyclohexen-1-one,
(177) 4,4-dimethyl-6-methylene-3-pyrrolinyl methyl-2-cyclohexen-1-one,
(178) 3-azethydinylmethyl-4,4-dimethyl-6-methylene-2-cyclohexen-1-one,
(179) 4,4-dimethyl-6-methylene-3-morpholinylmethyl-2-cyclohexen-1-one,
(180) 4,4-dimethyl-3-formylamino-6-methylene-2-cyclohexen-1-one,
(181) 3-acetylamino-4,4-dimethyl-6-methylene-2-cyclohexen-1-one,
(182) 4,4-dimethyl-6-methylene-3-propionylamino-2-cyclohexen-1-one,
(183) 3-butyrylamino-4,4-dimethyl-6-methylene-2-cyclohexen-1-one,
(184) 4,4-dimethyl-3-formyloxy-6-methylene-2-cyclohexen-1-one,
(185) 3-acetyloxy-4,4-dimethyl-6-methylene-2-cyclohexen-1-one,
(186) 4,4-dimethyl-6-methylene-3-propionyloxy-2-cyclohexen-1-one,
(187) 3-butyryloxy-4,4-dimethyl-6-methylene-2-cyclohexen-1-one,
(188) 4,4-dimethyl-6-methylene-3-trichloromethyl-2-cyclohexen-1-one,
(189) 4,4-dimethyl-6-methylene-3-trifluoromethyl-2-cyclohexen-1-one,
(190) 4,4-dimethyl-3-furyl-6-methylene-2-cyclohexen-1-one,
(191) 4,4-dimethyl-6-methylene-3-propyl-2-cyclohexen-1-one,
(192) 4,4-dimethyl-6-methylene-3-thienyl-2-cyclohexen-1-one,
(193) 4,4-dimethyl-3-isoxazolyl-6-methylene-2-cyclohexen-1-one,
(194) 4,4-dimethyl-3-imidazolyl-6-methylene-2-cyclohexen-1-one,
(195) 4,4-dimethyl-6-methylene-3-thiazolyl-2-cyclohexen-1-one,
(196) 4,4-dimethyl-6-methylene-3-pyrrolinyl-2-cyclohexen-1-one,
(197) 3-benzofuryl-4,4-dimethyl-6-methylene-2-cyclohexen-1-one,
(198) 3-benzothiazolyl-4,4-dimethyl-6-methylene-2-cyclohexen-1-one,
(199) 3-pyridyl-4,4-dimethyl-6-methylene-2-cyclohexen-1-one,
(200) 4,4-dimethyl-6-methylene-3-quinolyl-2-cyclohexen-1-one,
(201) 4,4-dimethyl-6-methylene-3-pyrimidinyl-2-cyclohexen-1-one,
(202) 4,4-dimethyl-6-methylene-3-morpholinyl-2-cyclohexen-1-one,
(203) 6-methylene-2,4,4-trimethyl-2-cyclohexen-1-one,
(204) 4,9-dimethyl-2-ethyl-6-methylene-2-cyclohexen-1-one,
(205) 4,4-dimethyl-6-methylene-2-propyl-2-cyclohexen-1-one,
(206) 4,4-dimethyl-2-isopropyl-6-methylene-2-cyclohexen-1-one
(207) 2-butyl-4,4-dimethyl-6-methylene-2-cyclohexen-1-one,
(208) 4,4-dimethyl-2-isobutyl-6-methylene-2-cyclohexen-1-one,
(209) 2-benzyl-4,4-dimethyl-6-methylene-2-cyclohexen-1-one,
(210) 4,4-dimethyl-2-hexyl-6-methylene-2-cyclohexen-1-one,
(211) 4,4-dimethyl-6-methylene-2-octyl-2-cyclohexen-1-one,
(212) 2-pentyloxy-4,4-dimethyl-6-methylene-2-cyclohexen-1-one,
(213) 4,4-dimethyl-2-hexyloxy-6-methylene-2-cyclohexen-1-one,
(214) 4,4-dimethyl-2-methylamino-6-methylene-2-cyclohexen-1-one,
(215) 4,4-dimethyl-2-ethylamino-6-methylene-2-cyclohexen-1-one,
(216) 4,4-dimethyl-6-methylene-2-propylamino-2-cyclohexen-1-one,
(217) 2-dimethylamino-4,4-dimethyl-6-methylene-2-cyclohexen-1-one,
(218) 2-vinyl-4,4-dimethyl-6-methylene-2-cyclohexen-1-one,
(219) 2-allyl-4,4-dimethyl-6-methylene-2-cyclohexen-1-one,
(220) 4,4-dimethyl-2-isopropenyl-6-methylene-2-cyclohexen-1-one,
(221) 2-cyclopropyl-4,4-dimethyl-6-methylene-2-cyclohexen-1-one,
(222) 2-cyclobutyl-4,4-dimethyl-6-methylene-2-cyclohexen-1-one,
(223) 2-cyclopentyl-4,4-dimethyl-6-methylene-2-cyclohexen-1-one,
(224) 2-cyclohexyl-4,4-dimethyl-6-methylene-2-cyclohexen-1-one,
(225) 4,4-dimethyl-2-phenyl-6-methylene-2-cyclohexen-1-one,
(226) 4,4-dimethyl-6-methylene-2-naphthyl-2-cyclohexen-1-one,
(228) 4,4-dimethyl-2-hydroxy methyl-6-methylene-2-cyclohexen-1-one,
(229) 4,4-dimethyl-2-hydroxy ethyl-6-methylene-2-cyclohexen-1-one,
(230) 4,4-dimethyl-2-hydroxy propyl-6-methylene-2-cyclohexen-1-one,
(231) 2-aminomethyl-4,4-dimethyl-6-methylene-2-cyclohexen-1-one,
(232) 2-aminoethyl-4,4-dimethyl-6-methylene-2-cyclohexen-1-one,
(233) 2-aminopropyl-4,4-dimethyl-6-methylene-2-cyclohexen-1-one,
(234) 4,4-dimethyl-2-methylaminomethyl-6-methylene-2-cyclohexen-1-one,
(235) 4,4-dimethyl-2-ethylaminomethyl-6-methylene-2-cyclohexen-1-one,
(236) 4,4-dimethyl-2-ethylaminoethyl-6-methylene-2-cyclohexen-1-one,
(237) 2-dimethylaminomethyl-4,4-dimethyl-6-methylene-2-cyclohexen-1-one,
(238) 2-diethylaminomethyl-4,4-dimethyl-6-methylene-2-cyclohexen-1-one,
(239) 4,4-dimethyl-6-methylene-2-piperazinyl-2-cyclohexen-1-one,
(240) 4,4-dimethyl-6-methylene-2-morpholinyl-2-cyclohexen-1-one,
(241) 4,4-dimethyl-6-methylene-2-piperazinyl ethyl-2-cyclohexen-1-one,
(242) 4,4-dimethyl-6-methylene-2-pyrrolinyl methyl-2-cyclohexen-1-one,
(243) 2-azethydinylmethyl-4,4-dimethyl-6-methylene-2-cyclohexen-1-one,
(244) 4,4-dimethyl-6-methylene-2-morpholinylmethyl-2-cyclohexen-1-one,
(245) 4,4-dimethyl-2-formylamino-6-methylene-2-cyclohexen-1-one,
(246) 2-acetylamino-4,4-dimethyl-6-methylene-2-cyclohexen-1-one,
(247) 4,4-dimethyl-6-methylene-2-propionylamino-2-cyclohexen-1-one,
(248) 2-butyrylamino-4,4-dimethyl-6-dimethylene-2-cyclohexen-1-one,
(249) 4,4-dimethyl-2-formyloxy-6-methylene-2-cyclohexen-1-one,
(250) 2-acetyloxy-4,4-dimethyl-6-methylene-2-cyclohexen-1-one,
(251) 4,4-dimethyl-6-methylene-2-propionyloxy-2-cyclohexen-1-one,
(252) 2-butyryloxy-4,4-dimethyl-6-methylene-2-cyclohexen-1-one,
(253) 4,4-dimethyl-6-methylene-2-trichloromethyl-2-cyclohexen-1-one,
(254) 4,4-dimethyl-6-methylene-2-trifluoromethyl-2-cyclohexen-1-one,
(255) 4,4-dimethyl-2-furyl-6-methylene-2-cyclohexen-1-one, (256) 4,4-dimethyl-6-methylene-2-propyl-2-cyclohexen-1-one,
(257) 4,4-dimethyl-6-methylene-2-thienyl-2-cyclohexen-1-one,
(258) 4,4-dimethyl-2-isoxazolyl-6-methylene-2-cyclohexen-1-one,
(259) 4,4-dimethyl-2-imidazolyl-6-methylene-2-cyclohexen-1-one,
(260) 4,4-dimethyl-6-methylene-2-thiazolyl-2-cyclohexen-1-one,
(261) 4,4-dimethyl-6-methylene-2-pyrrolinyl-2-cyclohexen-1-one,
(262) 2-benzofuryl-4,4-dimethyl-6-methylene-2-cyclohexen-1-one,
(263) 2-benzothiazolyl-4,4-dimethyl-6-methylene-2-cyclohexen-1-one,
(264) 2-pyridyl-4,4-dimethyl-6-methylene-2-cyclohexen-1-one,
(265) 4,4-dimethyl-6-methylene-2-quinolyl-2-cyclohexen-1-one,
(267) 4,4-dimethyl-6-methylene-2-pyrimidinyl-2-cyclohexen-1-one
(268) 4,4-dimethyl-6-methylene-2-morpholinyl-2-cyclohexen-1-one,
(269) 5H-4-dimethyl-6-methylene-7-oxo-indene,
(270) 4-dimethyl-2-methylene-1-oxo-tetralin,
(271) 3H-4-dimethyl-2-methylene-1-oxo-anthracene,
(272) 5H-4-dimethyl-6-methylene-7-oxo-benzothiophene,
(273) 5H-4-dimethyl-6-methylene-7-oxo-benzofuran,
(274) 5H-4-dimethyl-6-methylene-7-oxo-indole,
(275) 6H-5-dimethyl-7-methylene-8-oxo-quinoline,
(276) 6H-5-dimethyl-7-methylene-8-oxo-quinoxaline,
(277) 6H-5-dimethyl-7-methylene-8-oxo-cinnoline,
(278) 5H-5-dimethyl-7-methylene-8-oxo-1,4 dithianaphthalene,
(279) 3H-4-dimethyl-2-methylene-1-oxo-thianthrene, and the acid addition salts thereof

Representative compounds corresponding to the above-stated general formula (3-b) include the basic compound 4,4-dimethyl-2-cyclohexen-1-one wherein all of the substituent R3, R4, R5 and R6 represent hydrogen atoms and include further other representatives such as for example:
(280) 4,4,5-trimethyl-2-cyclohexen-1-one,
(281) 4,4-dimethyl-5-ethyl-2-cyclohexen-1-one,
(282) 4,4-dimethyl-5-propyl-2-cyclohexen-1-one,
(283) 4,4-dimethyl-5-isopropyl-2-cyclohexen-1-one,
(284) 5-butyl-4,4-dimethyl-2-cyclohexen-1-one,
(285) 4,4-dimethyl-5-isobutyl-2-cyclohexen-1-one,
(286) 5-benzyl-4,4-dimethyl-2-cyclohexen-1-one,
(287) 4,4-dimethyl-5-hexyl-2-cyclohexen-1-one,
(288) 4,4-dimethyl-5-octyl-2-cyclohexen-1-one,
(289) 5-pentyloxy-4,4-dimethyl-2-cyclohexen-1-one,
(290) 4,4-dimethyl-5-hexyloxy-2-cyclohexen-1-one,
(291) 4,4-dimethyl-5-methylamino-2-cyclohexen-1-one,
(292) 4,4-dimethyl-5-ethylamino-2-cyclohexen-1-one,
(293) 4,4-dimethyl-5-propylamino-2-cyclohexen-1-one,
(294) 4,4-dimethyl-5-dimethylamino-2-cyclohexen-1-one,
(295) 5-vinyl-4,4-dimethyl-2-cyclohexen-1-one,
(296) 5-allyl-4,4-dimethyl-2-cyclohexen-1-one,
(297) 4,4-dimethyl-5-isopropenyl-2-cyclohexen-1-one,
(298) 5-cyclopropyl-4,4-dimethyl-2-cyclohexen-1-one,
(299) 5-cyclobutyl-4,4-dimethyl-2-cyclohexen-1-one,
(300) 5-cyclopentyl-4,4-dimethyl-2-cyclohexen-1-one,
(301) 5-cyclohexyl-4,4-dimethyl-2-cyclohexen-1-one,
(302) 4,4-dimethyl-5-phenyl-2-cyclohexen-1-one,
(303) 4,4-dimethyl-5-naphthyl-2-cyclohexen-1-one,
(305) 4,4-dimethyl-5-hydroxy methyl-2-cyclohexen-1-one,
(306) 4,4-dimethyl-5-hydroxy ethyl-2-cyclohexen-1-one,
(307) 4,4-dimethyl-5-hydroxy propyl-2-cyclohexen-1-one,
(308) 5-aminomethyl-4,4-dimethyl-2-cyclohexen-1-one,
(309) 5-aminoethyl-4,4-dimethyl-2-cyclohexen-1-one,
(310) 5-aminopropyl-4,4-dimethyl-2-cyclohexen-1-one,
(311) 4,4-dimethyl-5-methylaminomethyl-2-cyclohexen-1-one,
(312) 4,4-dimethyl-5-ethylaminomethyl-2-cyclohexen-1-one,
(313) 4,4-dimethyl-5-ethylaminoethyl-2-cyclohexen-1-one,
(314) 4,4-dimethyl-5-dimethylaminomethyl-2-cyclohexen-1-one,
(315) 4,4-dimethyl-5-diethylaminomethyl-2-cyclohexen-1-one,
(316) 4,4-dimethyl-5-piperazinyl-2-cyclohexen-1-one,
(317) 4,4-dimethyl-5-morpholinyl-2-cyclohexen-1-one,
(318) 4,4-dimethyl-5-piperazinyl ethyl-2-cyclohexen-1-one,
(319) 4,4-dimethyl-5-pyrrolinyl methyl-2-cyclohexen-1-one,
(320) 5-azethydinylmethyl-4,4-dimethyl-2-cyclohexen-1-one,
(321) 4,4-dimethyl-5-morpholinylmethyl-2-cyclohexen-1-one,
(322) 4,4-dimethyl-5-formylamino-2-cyclohexen-1-one,
(323) 5-acetylamino-4,4-dimethyl-2-cyclohexen-1-one,
(324) 4,4-dimethyl-5-propionylamino-2-cyclohexen-1-one,
(325) 5-butyrylamino-4,4-dimethyl-2-cyclohexen-1-one,
(326) 4,4-dimethyl-5-formyloxy-2-cyclohexen-1-one,
(327) 5-acetyloxy-4,4-dimethyl-2-cyclohexen-1-one,
(328) 4,4-dimethyl-5-propionyloxy-2-cyclohexen-1-one,
(329) 5-butyryloxy-4,4-dimethyl-2-cyclohexen-1-one,
(330) 4,4-dimethyl-5-trichloromethyl-2-cyclohexen-1-one,
(331) 4,4-dimethyl-5-trifluoromethyl-2-cyclohexen-1-one,
(332) 4,4-dimethyl-5-furyl-2-cyclohexen-1-one,
(333) 4,4-dimethyl-5-propyl-2-cyclohexen-1-one,
(334) 4,4-dimethyl-5-thienyl-2-cyclohexen-1-one,
(335) 4,4-dimethyl-5-isoxazolyl-2-cyclohexen-1-one,
(336) 4,4-dimethyl-5-imidazolyl-2-cyclohexen-1-one,
(337) 4,4-dimethyl-5-thiazolyl-2-cyclohexen-1-one,
(338) 4,4-dimethyl-5-pyrrolinyl-2-cyclohexen-1-one,
(339) 5-benzofuryl-4,4-dimethyl-2-cyclohexen-1-one,
(340) 5-benzothiazolyl-4,4-dimethyl-2-cyclohexen-1-one,
(341) 5-pyridyl-4,4-dimethyl-2-cyclohexen-1-one,
(342) 4,4-dimethyl-5-quinolyl-2-cyclohexen-1-one,
(343) 4,4-dimethyl-5-pyrimidinyl-2-cyclohexen-1-one,
(344) 4,4-dimethyl-5-morpholinyl-2-cyclohexen-1-one,
(345) 3,4,4-trimethyl-2-cyclohexen-1-one,
(346) 4,4-dimethyl-3-ethyl-2-cyclohexen-1-one,
(347) 4,4-dimethyl-3-propyl-2-cyclohexen-1-one,
(348) 4,4-dimethyl-3-isopropyl-2-cyclohexen-1-one,
(349) 3-butyl-4,4-dimethyl-2-cyclohexen-1-one,
(350) 4,4-dimethyl-3-isobutyl-2-cyclohexen-1-one,
(351) 3-benzyl-4,4-dimethyl-2-cyclohexen-1-one,
(352) 4,4-dimethyl-3-hexyl-2-cyclohexen-1-one,
(353) 4, 4-dimethyl-3-octyl-2-cyclohexen-1-one,
(354) 3-pentyloxy-4,4-dimethyl-2-cyclohexen-1-one,
(355) 4,4-dimethyl-3-hexyloxy-2-cyclohexen-1-one,
(356) 4,4-dimethyl-3-methylamino-2-cyclohexen-1-one,
(357) 4,4-dimethyl-3-ethylamino-2-cyclohexen-1-one,
(358) 4,4-dimethyl-3-propylamino-2-cyclohexen-1-one,
(359) 3-dimethylamino-4,4-dimethyl-2-cyclohexen-1-one,
(360) 3-vinyl-4,4-dimethyl-2-cyclohexen-1-one,
(361) 3-allyl-4,4-dimethyl-2-cyclohexen-1-one,
(362) 4,4-dimethyl-3-isopropenyl-2-cyclohexen-1-one,
(363) 3-cyclopropyl-4,4-dimethyl-2-cyclohexen-1-one,
(364) 3-cyclobutyl-4,4-dimethyl-2-cyclohexen-1-one,
(365) 3-cyclopentyl-4,4-dimethyl-2-cyclohexen-1-one,
(366) 3-cyclohexyl-4,4-dimethyl-2-cyclohexen-1-one,
(367) 4,4-dimethyl-3-phenyl-2-cyclohexen-1-one,
(368) 4,4-dimethyl-3-naphthyl-2-cyclohexen-1-one,
(370) 4,4-dimethyl -3-hydroxy methyl-2-cyclohexen-1-one,
(371) 4,4-dimethyl -3-hydroxy ethyl-2-cyclohexen-1-one,
(372) 4,4-dimethyl -3-hydroxy propyl-2-cyclohexen-1-one,
(373) 3-aminomethyl-4,4-dimethyl-2-cyclohexen-1-one,
(374) 3-aminoethyl-4,4-dimethyl-2-cyclohexen-1-one,
(375) 3-aminopropyl-4,4-dimethyl-2-cyclohexen-1-one,
(376) 4,4-dimethyl-3-methylaminomethyl-2-cyclohexen-1-one,
(377) 4,4-dimethyl-3-ethylaminomethyl-2-cyclohexen-1-one,
(378) 4,4-dimethyl-3-ethylaminoethyl-2-cyclohexen-1-one,
(379) 3-dimethylaminomethyl-4,4-dimethyl-2-cyclohexen-1-one,
(380) 3-diethylaminomethyl-4,4-dimethyl-2-cyclohexen-1-one,
(381) 4,4-dimethyl-3-piperazinyl-2-cyclohexen-1-one,
(382) 4,4-dimethyl-3-morpholinyl-2-cyclohexen-1-one,
(383) 4,4-dimethyl-3-piperazinylethyl-2-cyclohexen-1-one,
(384) 4,4-dimethyl-3-pyrrolinylmethyl-2-cyclohexen-1-one,
(385) 3-azethydinylmethyl-4,4-dimethyl-2-cyclohexen-1-one,
(386) 4,4-dimethyl-3-morpholinylmethyl-2-cyclohexen-1-one,
(387) 4,4-dimethyl-3-formylamino-2-cyclohexen-1-one,
(388) 3-acetylamino-4,4-dimethyl-2-cyclohexen-1-one,
(389) 4,4-dimethyl-3-propionylamino-2-cyclohexen-1-one,
(390) 3-butyrylamino-4,4-dimethyl-2-cyclohexen-1-one,
(391) 4,4-dimethyl-3-formyloxy-2-cyclohexen-1-one,
(392) 3-acetyloxy-4,4-dimethyl-2-cyclohexen-1-one,
(393) 4,4-dimethyl-3-propionyloxy-2-cyclohexen-1-one,
(394) 3-butyryloxy-4,9-dimethyl-2-cyclohexen-1-one,
(395) 4,4-dimethyl-3-trichloromethyl-2-cyclohexen-1-one,
(396) 4,4-dimethyl-3-trifluoromethyl-2-cyclohexen-1-one,
(397) 4,4-dimethyl-3-furyl-2-cyclohexen-1-one,
(398) 4,4-dimethyl-3-propyl-2-cyclohexen-1-one,
(399) 4,4'dimethyl-3-thienyl-2-cyclohexen-1-one,
(400) 4,4-dimethyl-3-isoxazolyl-2-cyclohexen-1-one,
(401) 4,4-dimethyl-3-imidazolyl-2-cyclohexen-1-one,
(402) 4,4-dimethyl-3-thiazolyl-2-cyclohexen-1-one,
(403) 4,4-dimethyl-3-pyrrolinyl-2-cyclohexen-1-one,
(404) 3-benzofuryl-4,4-dimethyl-2-cyclohexen-1-one,
(405) 3-benzothiazolyl-4,4-dimethyl-2-cyclohexen-1-one,
(406) 3-pyridyl-4,4-dimethyl-2-cyclohexen-1-one,
(407) 4,4-dimethyl-3-quinolyl-2-cyclohexen-1-one,
(408) 4,4-dimethyl-3-pyrimidinyl-2-cyclohexen-1-one,
(409) 4,4-dimethyl-3-morpholinyl-2-cyclohexen-1-one,
(410) 2,4,4-trimethyl-2-cyclohexen-1-one,
(411) 4,4-dimethyl-2-ethyl-2-cyclohexen-1-one,
(412) 4,4-dimethyl-2-propyl-2-cyclohexen-1-one,
(413) 4,4-dimethyl-2-isopropyl-2-cyclohexen-1-one,
(414) 2-butyl-4,4-dimethyl-2-cyclohexen-1-one,
(415) 4,4-dimethyl-2-isobutyl-2-cyclohexen-1-one,
(416) 2-benzyl-4,4-dimethyl-2-cyclohexen-1-one,
(417) 4,4-dimethyl-2-hexyl-2-cyclohexen-1-one,
(418) 4,4-dimethyl-2-octyl-2-cyclohexen-1-one,
(419) 2-pentyloxy-4,4-dimethyl-2-cyclohexen-1-one,
(420) 4,4-dimethyl-2-hexyloxy-2-cyclohexen-1-one,
(421) 4,4-dimethyl-2-methylamino-2-cyclohexen-1-one,
(422) 4,4-dimethyl-2-ethylamino-2-cyclohexen-1-one,
(423) 4,4-dimethyl-2-propylamino-2-cyclohexen-1-one,
(424) 2-dimethylamino-4,4-dimethyl-2-cyclohexen-1-one,
(425) 2-vinyl-4,4-dimethyl-2-cyclohexen-1-one,
(426) 2-allyl-4,4-dimethyl-2-cyclohexen-1-one,
(427) 4,4-dimethyl-2-isopropenyl-2-cyclohexen-1-one,
(428) 2-cyclopropyl-4,4-dimethyl-2-cyclohexen-1-one,
(429) 2-cyclobutyl-4,4-dimethyl-2-cyclohexen-1-one,
(430) 2-cyclopentyl-4,4-dimethyl-2-cyclohexen-1-one,
(431) 2-cyclohexyl-4,4-dimethyl-2-cyclohexen-1-one,
(432) 4,4-dimethyl-2-phenyl-2-cyclohexen-1-one,
(433) 4,4-dimethyl-2-naphthyl-2-cyclohexen-1-one,
(435) 4,4-dimethyl-2-hydroxy methyl-2-cyclohexen-1-one,
(436) 4,4-dimethyl -2-hydroxy ethyl-2-cyclohexen-1-one,
(437) 4,4-dimethyl -2-hydroxy propyl-2-cyclohexen-1-one,
(438) 2-aminomethyl-4,4-dimethyl-2-cyclohexen-1-one,
(439) 2-aminoethyl-4,4-dimethyl-2-cyclohexen-1-one,
(940) 2-aminopropyl-4,4-dimethyl-2-cyclohexen-1-one,
(441) 4,4-dimethyl-2-methylaminomethyl-2-cyclohexen-1-one,
(442) 4,4-dimethyl-2-ethylaminomethyl-2-cyclohexen-1-one,
(443) 4,4-dimethyl-2-ethylaminoethyl-2-cyclohexen-1-one,
(444) 2-dimethylaminomethyl-4,4-dimethyl-2-cyclohexen-1-one,
(445) 2-diethylaminomethyl-4,4-dimethyl-2-cyclohexen-1-one,
(446) 9,4-dimethyl-2-piperazinyl-2-cyclohexen-1-one,
(447) 4,4-dimethyl-2-morpholinyl-2-cyclohexen-1-one,
(448) 4,4-dimethyl-2-piperazinylethyl-2-cyclohexen-1-one,
(449) 4,4-dimethyl-2-pyrrolinylmethyl-2-cyclohexen-1-one,
(450) 2-azethydinylmethyl-4,4-dimethyl-2-cyclohexen-1-one,
(451) 4,4-dimethyl-2-morpholinylmethyl-2-cyclohexen-1-one,
(452) 4,4-dimethyl-2-formylamino-2-cyclohexen-1-one,
(453) 2-acetylamino-4,4-dimethyl-2-cyclohexen-1-one,
(454) 4,4-dimethyl-2-propionylamino-2-cyclohexen-1-one,
(455) 2-butyrylamino-4,4-dimethyl-2-cyclohexen-1-one,
(456) 4,4-dimethyl-2-formyloxy-2-cyclohexen-1-one,
(457) 2-acetyloxy-4,4-dimethyl-2-cyclohexen-1-one,
(458) 4,4-dimethyl-2-propionyloxy-2-cyclohexen-1-one,
(459) 2-butyryloxy-4,4-dimethyl-2-cyclohexen-1-one,
(460) 4,4-dimethyl-2-trichloromethyl-2-cyclohexen-1-one,
(461) 4,4-dimethyl-2-trifluoromethyl-2-cyclohexen-1-one,
(462) 4,4-dimethyl-2-furyl-2-cyclohexen-1-one,
(463) 4,4-dimethyl-2-propyl-2-cyclohexen-1-one,
(464) 4,4-dimethyl-2-thienyl-2-cyclohexen-1-one,
(465) 4,4-dimethyl-2-isoxazolyl-2-cyclohexen-1-one,
(466) 4,4-dimethyl-2-imidazolyl-2-cyclohexen-1-one,
(467) 4,4-dimethyl-2-thiazolyl-2-cyclohexen-1-one,
(468) 4,4-dimethyl-2-pyrrolinyl-2-cyclohexen-1-one,
(469) 2-benzofuryl-4,4-dimethyl-2-cyclohexen-1-one,
(470) 2-benzothiazolyl-4,4-dimethyl-2-cyclohexen-1-one,
(471) 2-pyridyl-4,4-dimethyl-2-cyclohexen-1-one,
(472) 4,4-dimethyl-2-quinolyl-2-cyclohexen-1-one,
(473) 4,4-dimethyl-2-pyrimidinyl-2-cyclohexen-1-one,
(474) 4,4-dimethyl-2-morpholinyl-2-cyclohexen-1-one,
(475) 5H-4-dimethyl-7-oxo-indene,
(476) 4-dimethyl-1-oxo-tetralin,
(477) 3H-4-dimethyl-1-oxo-anthracene,
(478) 5H-4-dimethyl-7-oxo-benzothiophene,
(479) 5H-4-dimethyl-7-oxo-benzofuran,
(480) 5H-4-dimethyl-7-oxo-indole,
(481) 6H-5-dimethyl-8-oxo-quinoline,
(482) 6H-5-dimethyl-8-oxo-quinoxaline,
(483) 6H-5-dimethyl-8-oxo-cinnoline,
(484) 5H-5-dimethyl-8-oxo-1,4-dithianaphthalene,
(485) 3H-4-dimethyl-1-oxo-thianthrene,
and the acid addition salts thereof.

The compounds according to the above-stated general formula (2), (3-a) (3-a-1) and (3-b) may be synthesized by the known processing manners of organic synthesis with conventional organic chemical compounds and/or natural plant oils. When the acid addition salts of those compounds are used it is possible to administer those acid addition salts in form of a single agent or in form of a combined agent along with a carrier substance which is acceptable for drugs. These compositions are dependent on routes and/or planning of administration. When used as drugs the compounds corresponding to the above-stated general formula (2), (3-a) (3-a-1) and (3-b) or the acid addition salts thereof may be administered orally or non-orally as medicament compositions such as powders, granules, tablets, capsules, injection solutions by suitably mixing with adequate components such as carrier substances, excipients or attenuants which are pharmaceutically acceptable. Also, an effect of the compounds can be expected when administered in vapor form.

As an example of solid carriers, capsules made from usual gelatin may be mentioned. The following substances may be used as the excipients: gelatin, lactose, sugars such as glucose, cone, wheat, rice, starches such as corn starch, fatty acids such as stearic acid, fat bases such as calcium stearic acid and magnesium stearic acid, talc, vegetable oil, alcohol such as stearylalcohol and benzyl alcohol, gum, polyethylene alkylene glycol and so on.

These capsules tablets, granules and powder may contain the effective ingredient in amounts of from 0.1-80 weight % and especially in amounts of from 0.1-60 weight %. Liquid carriers such as water, physiological saline solution, sugar solution, dextrose solution, ethylene glycol, propylene glycol, glycols such as polyethylene glycol, polyoxyethylene sorbitan monoolate are desirable.

When administered non-orally by means of intramuscular injection, intravenous injection or hypodermic injection, the compounds corresponding to the above-stated general formula (2), (3-a) (3-a-1) and (3-b) are used as a germ-free solution having incorporated other soluble components such as minerals or glucose in order to obtain an isotonic solution. Appropriate solvents for an injection include sterilized water, solution of lidocaine hydrochloride (for intramuscular injection), physiological saline solution, glucose solution, any kind of fluids for an intravenous injection, and electrolyte solution (for intravenous injection). When those solutions for the injection are used, usual dosage is 0.01-20 weight % and an even more preferred dosis is 0.05-5 weight %.

In the case of liquids for oral administration, it is better to be used as suspension or syrup with 0.01-20 weight %. A carrier of these liquids is watery excipient such as perfume, syrup and micelle which are available for manufacturing, of pharmaceutic preparations.

### BRIEF DESCRIPTION OF THE DRAWINGS

Figure 1 is a graph showing the effects of "Yoshixol," 4,4-dimethyl-6-methylene-2-cyclohexen-1-one, in the following termed on NADPH, NADP, heme and cytochrome C; these effects have been measured by means of infrared spectrophotometer.

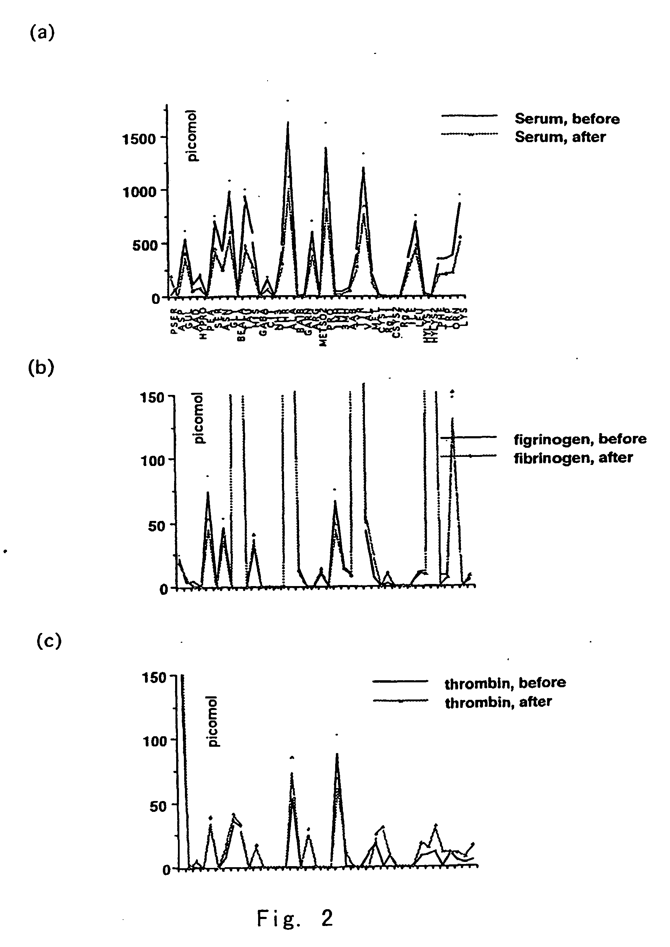

Figure 2 is a graph showing the effects of Yoshixol on amino acids composition of human blood serum, thrombin and fibrinogen.
Figure 3 (a) and 3 (b) is a graph showing the effect of Yoshixol on blood coagulation as measured by thromboelastgram.
Figure 4 is a graph showing the effect of Yoshixol on the configuration and the function of trypsin and bovine albumin wherein the obtained digitalized data to have been converted a graphic design from stained contrast of electrophoresis.
Figure 5 (a) and 5 (b) is a graph showing the effect of Yoshixol on serum antibodies for blood type judgment of ABO blood types, wherein the obtained digitalized data have been converted to a graphic design from a magnitude of aggregated reaction.
Figure 6 is a graph showing an effect of Yoshixol to increase the blood pressure due to vasopressin.
Figure 7 (a), 7 (b) and 7(c) is a graph showing the effect of Yoshixol on configuration of proteins of bovine albumin and blood type anti-A blood serum, anti-B blood serum wherein the obtained digitalized data have been converted to a graphic design from stained contrast of electrophoresis.
Figure 8 (a), 8 (b) and 8 (c) is a graph showing the antimicrobacterial effect of Yoshixol on methitilin resistance staphylococcus aureus (MRSA), E. Coli or Candida albicans.
Figure 9 is a picture prepared by means of a scanning electron microscopy, and demonstrating a typical morphological aspect of the cell death of MRSA immediately after treatment with Yoshixol.
Figure 10 is a picture prepared by means of a scanning electron microscopy and demonstrating a typical morphological aspect of the cell death of E. coli immediately after treatment with Yoshixol.
Figure 11 is a picture prepared by means of a scanning electron microscopy and demonstrating a typical morphological aspect of the cell death of acid-fast bacilli immediately after treatment with Yoshixol.
Figure 12 is a picture prepared by means of a scanning electron microscopy and demonstrating a typical morphological aspect of the cell death of Candida albicans immediately after treatment with Yoshixol.
Figure 13 is a picture prepared by means of a scanning electron microscopy, and demonstrating a typical morphological aspect of the cell death of pseudomonas aeruginosa immediately after treatment with Yoshixol.
Figure 14 is a graph showing the effect of Yoshixol on a number of plaques formed by bacteriophages infected to E. Coli.
Figure 15 is a graph showing the effect of Yoshixol on chicken myeloblastosis virus (AMV) reverse transcriptase.
Figure 16 (a) and 16 (b) a representation of histological preperations showing the effect of Yoshixol on cultured keratinocytes, observed by a phase microscopy.
Figure 17 (a) and 17 (b) is a representation of the histological preparation showing the effect of Yoshixol on cultured keratinocytes, observed by an transmission electron microscopy.
Figure 18 is a graph showing the survival rate of HeLa cells which have been treated with Yoshixol.
Figure 19 is a picture prepared by means of a scanning electron microscopy and demonstrating a typical morphological aspect of the cell death of HeLa cells immediately after treatment with Yoshixol.
Figure 20 (a) and 20 (b) is a representation of histological preparations showing morphological changes of blood erythrocyte due to Yoshixol, observed by means of a scanning electron microscopy.
Figure 21 is the representation of a histological preperation showing canine skin after treatment with Yoshixol, and a further histological preparation wherein rabbit skin was transplanted to a dog as recipient and intravenously treated with Yoshixol after the transplantation.
Figure 22 is a picture showing a thrombolytic effect of Yoshixol on fresh thrombus.
Figure 23 is a graph showing the effect of Yoshixol on change in amount of total proteins and serum albumin concentration when Yoshixol was administered orally to a dog.
Figure 24 is a graph showing the effect of Yoshixol on change in amount of serum globulin concentration and ratio of albumin vs globulin when Yoshixol was orally administered to a dog.
Figure 25 is a graph showing the effect of Yoshixol on change in total cholesterol and concentration of serum triglyceride when Yoshixol was orally administered to a dog.
Figure 26 is a graph showing the effect of Yoshixol on change in serum nonproteins nitrogen and serum creatinine concentration when Yoshixol was orally administered to a dog.
Figure 27 is a graph showing the effect of Yoshixol on change in serum creatinine concentration when Yoshixol was orally administered to a dog.
Figure 28 is the representation of a histological preparation showing the effect of Yoshixol on flagellum of bovine spermatozoa, observed by means of a scanning electron microscopy.
Figure 29 is the representation of a histological preparation showing the effect of Yoshixol on flagellum of bovine spermatozoa, observed by means of a transmission electron microscopy.
Figure 30 is a graph showing a calorimetry effect of Yoshixol on palmitic acid, measured by means of a digital scanning calorimeter.
Figure 31 is a graph showing a calorimetry effect of Yoshixol on polyethylene glycol 1000, measured by means of a digital scanning calorimeter.
Figure 32 is a graph showing a calorimetry effect of Yoshixol on polystyrene 280,000, measured by means of a digital scanning calorimeter.
Figure 33 is a graph which showing calorimetry effects of Yoshixol on methyl methacrylate and ethyl methacrylate, as measured by means of a digital scanning calorimeter.
Figure 34 is a graph showing calorimetry effects of Yoshixol on isobutyl methacrylate and poly (vinyl chloride), as measured by means of a digital scanning calorimeter.
Figure 35 is a graph showing a calorimetry effect of Yoshixol on polyethylene glycol 4000 and poly (methylacrylate), as measured by means of a digital scanning calorimeter.
Figure 36 is a graph showing a thermal mechanical effect of Yoshixol on poly (vinyl chloride), as measured by means of a thermal mechanical analyzer.
Figure 37 shows the results of electrophoresis and demonstrating the effect of Yoshixol on newly synthesized dimers with 7 base pairs.
Figure 38 shows the results of electrophoresis and demonstrating the effect of Yoshixol on PCR of snake DNA by using a newly synthesized dimer of 7 base pairs as a primer.
Figure 39 is a graph showing the effect of Yoshixol on change in absorbance of ethylene bromide, as measured by means of a spectrophotometer.

### BEST MODE FOR CARRYING OUT THE INVENTION

4,4-dimethyl-6-methylene-2-cyclohexen-1-one (this compound is termed as Yoshixol) has been prepared as one of a concrete and representative compound which shows reasonable biological effects and which may be easily synthesized due to its simple chemical structure. Representative experiments have been performed in order to demonstrate the effects of this Yoshixol so as to show the effectiveness of the present invention.

Yoshixol was prepared according to the following method.

44,4 ml of a hexane solution of n-butyllithium (70 mmol, 1.62 mol/L) were added drop-wise to solution of diisopropylamine (7.27g, 7mmol) in anhydrous tetrahydrofuran (THF) at -78°C. The obtained solution was heated to 0°C and stirred for 1 hr at this temperature. The thus prepared lithium diisopopylamide solution was again cooled to -78°C, and 4,4-dimethyl-2-cyclohexen-1-one (7.5g, 60 mmol) was continously added dropwise under stirring within 1 hr. Thereafter, gaseous formaldehyde, generated by thermal decomposition of paraformaldehyde (5g), was passed through the solution by means of a gentle stream of dry nitrogen gas and the reaction mixture was stirred for 2 hr. After standing over night at room temperature, 1N hydrochloric acid was added until the solution reached a weak acid pH. The solvent was removed and the residue was extracted with ether. After drying over anhydrous sodium sulfate and removal of the solvent, a mixture of 4,4-dimethyl-6-methylene-2-cyclohexen-1-one and 4,4-dimethyl-6-hydroxymethyl- 2-cyclohexen-1-one was obtained (7.03g). This mixture (3.0g) was treated in refluxing benzene for 2 hr in the presence of a catalytic amount of anhydrous p-toluenesulfonic acid and molecular sieve 3A (4g). The obtained solution was neutralized with 1N sodium bicarbonate and washed with brine. After drying over anhydrous sodium sulfate and removal of the solvent, crude 4,4-dimethyl-6-methylene-2-cyclohexen-1-one (Yoshixol) was obtained (2.35 g, overall yield 68%). A sample for analytical purposes was obtained after purification by column chromatography or kugelrohr distillation. The spectroscopical analysis of Yoshixol provides the following data: IR(neat): 1670(C=O), 1620(C=C) cm-1. 1H NMR (60 MHZ, CCL4): d1.15 (s,6H,C(CH3)2), 2.57 (bs,2H,CH2), 5.20, 5.93 (2m,2H,CH2=), 5.87 (d,J=10,1H,2-H), 6.63 (d, J=10Hz, 3-H).

### Stability of Yoshixol solution under light

The just obtained Yoshixol was maintained within a sealed glas tube under room light for 6 months, and changes in colour, viscosity and odor were observed. As a result, colour viscosity and odor did not change and were identical with the initial properties.

### Reaction of Yoshixol with ribose, glycerin, cellulose and polyethylene vinyl

Each 1 ml of glycerin, cellulose, polyethylene vinyl and ribose (1 mol) was mixed with 4 µl of Yoshixol in a test tube. Then, fluidability and transparence of the reaction mixture in each sample tube were investigated at room temperature (28 °C), at elevated temperature (80°C) and at reduced temperature (4°C). At room temperature, transparence and fluidability of each sample in test tube increased. When each sample was cooled, an increase in hardness and cloudy appearance was observed. In contrast, at high temperature, an increase in fluidability and transparence of each sample to a greater extent than above two conditions, was observed. This result is interpreted that Yoshixol has polymerization and/or depolymerization effect.

### Effect of Yoshixol on NADPH, heme and cytochrome C

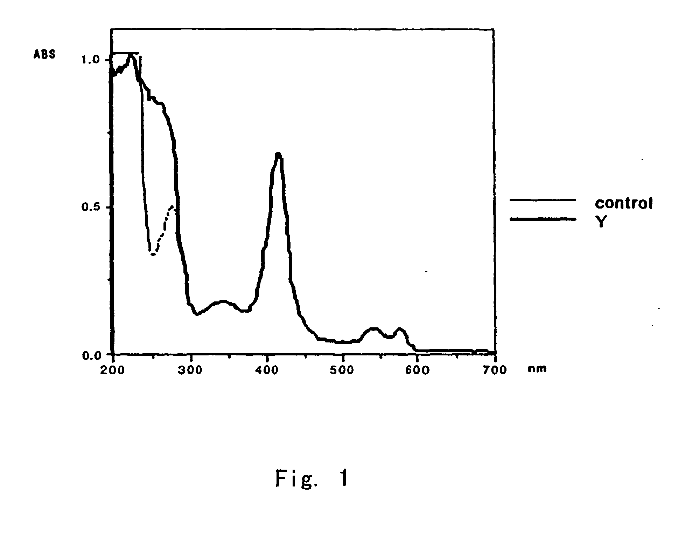

Changes in IR-absorption of NADPH (at 340 nm), of cytochrome C (at 415, 520, 550 nm), of heme (at 415 nm) and of aromatic amino acids (at 280 nm) in 1 µl of defibrinated human blood serum were measured before and after treatment with 4 µl of Yoshixol by an infrared spectrophotometer. IR-absorption of NADPH, cytochrome C and heme did not alter after treatment with Yoshixol, but characteristic peak of IR-absorption of amino acids disappeared and shifted to lower wave lengths (range of aromatic amines). This result is interpreted that Yoshixol has an effect of forming new aromatic amines not only due to dehydrogenation but due to reduction and/or hydrogen binding. Figure 1 shows that an extremely high and monophasic absorption peak appeared of 280 nm resulting from disappearance of biphasic peak,

### Effect of Yoshixol on composition of amino acids in human blood serum, fibrinogen and thrombin

Changes in composition of amino acids were analyzed by use of 1 ml of human blood serum, 1 ml of human fibrinogen (concentration of 160 mg/dl), 1 ml of human thrombin before and after treatment with Yoshixol (4 µl). After treatment with Yoshixol, the total amino acids content in the human blood serum decreased by 41%. Further the treatment altered each composition so that as an example the content of phosphoserine increased about 20 times from a level of 7.2 picomole to a level of 164.8 picomole. The content of the total amino acids in fibrinogen did not change by treatment with Yoshixol, but glutamic acid and hydroxyl serine which were existed before treatment disappeared after the treatment, and the content of cystathione in fibrinogen increased to a more than 5 times level. In addition, in a thrombin sample treated with Yoshixol, glutamic acid, taurine, methionine and aminoisobutylic acid each not observed before the treatment were newly formed. This result is interpreted that Yoshixol has a cross-linking or coupling effect such as obtained by desulfurization reaction and has an effect to change the molecular configuration of proteins and amino acids molecules to another molecular configuration thereof.

### "Effect of Yoshixol on human blood coagulation and fibrin formation"

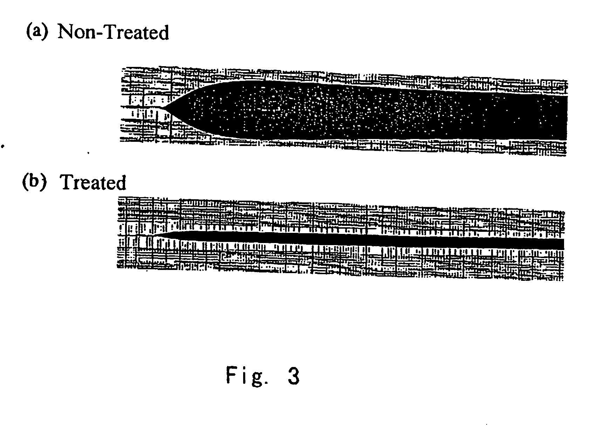

Effect of Yoshixol (4 µl) on human blood coagulation and fibrin formation (thrombin was added to fibrinogen) were investigated by means of thromboelastgram (Hellige GMBH, West Germany). Also, the magnitude of a fibrin network formed at human blood coagulation was investigated by a means of scanning electron microscope. Coagulation of human blood without treatment with Yoshixol was started within 1 to 2 min so that maximum level of coagulation occurred approximately within 15 min, followed by gradual decrease due to an activation of the fibrinolytic system. Immediately after adding thrombin to fibrinogen, the viscosity of the sample started to increase and reached a maximum level within 5 to 6 minutes. As observed, this maximum level was maintained for periods of 6 hours (as demonstrated with Figure 3a). However, when Yoshixol (4 µl) was added to 0.4 ml of whole blood or fibrinogen (160 mg/dl), the onset time of blood coagulation and fibrin formation was delayed to 4 to 5 minutes and maximum aggregation time was observed within 6 to 8 minutes. After the treatment the maximum level of coagulation was inhibited to 90% of the control period. (as demonstrated with Figure 3b). And, when thrombin treated with Yoshixol was added to fibrinogen, not any aggregation reaction could be found on thromboelastgram. Moreover, morphological investigations showed substantial blood coagulation, rouleaux formation of erythrocytes and formation of fibrin net in the group not treated with Yoshixol; however, respective formations could not be observed in the group treated with Yoshixol. The result is interpreted that Yoshixol has a strong anticoagulating effect (anti-thrombin effect) and antifibrinolytic effect.

### Effects of Yoshixol on change in function and configuration of trypsin and thrombin

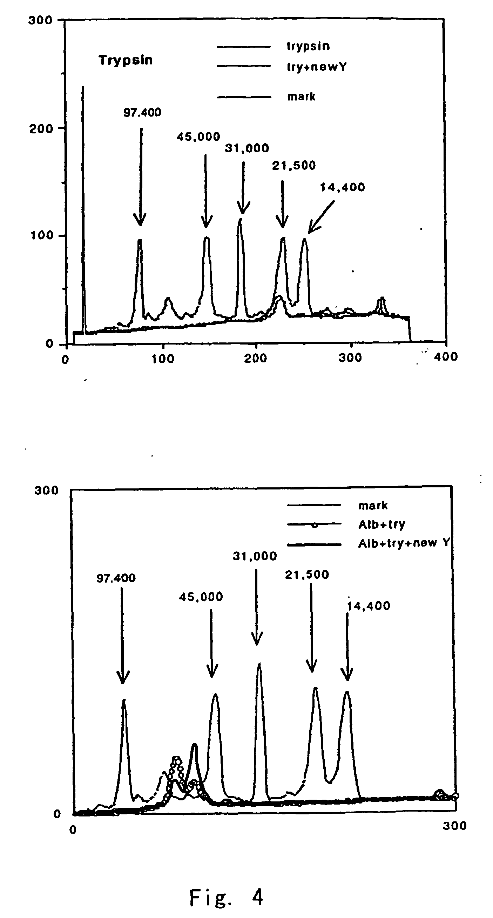

Physiological effects of trypsin (Wakou Jyunyaku Ltd.) and thrombin (Behlinger Manheim-Yamanouchi Co.) were investigated by means of electrophoresis. When trypsin was added to bovine albumin (Sigma Co. Saint Louis, Missouri, USA) or myoglobin (Sigma Co. Saint Louis, Missouri, USA), primary albumin structure of proteins was altered by the proteolytic action of trypsin. When thrombin was added to human fibrinogen (Behlinger Manheim-Yamanouchi Co.), a primary fibrinogen structure of proteins could not be found. Primary trypsin or fibrinogen structures of proteins did not show any electrophoretic changes even after treatment with Yoshixol (4 µl) and, the usual physiological reaction of trypsin or fibrinogen with bovine albumin, myoglobin and fibrinogen could not be observed (as demonstrated with Figure 4). This result is interpreted that Yoshixol has a blocking property to typical trypsin or thrombin functions, may be due to change of multi-dimensional structure, but not of due a change of primary structure of trypsin or thrombin.

### Effect of Yoshixol on serum antibody functions in different blood types (A, B and O)

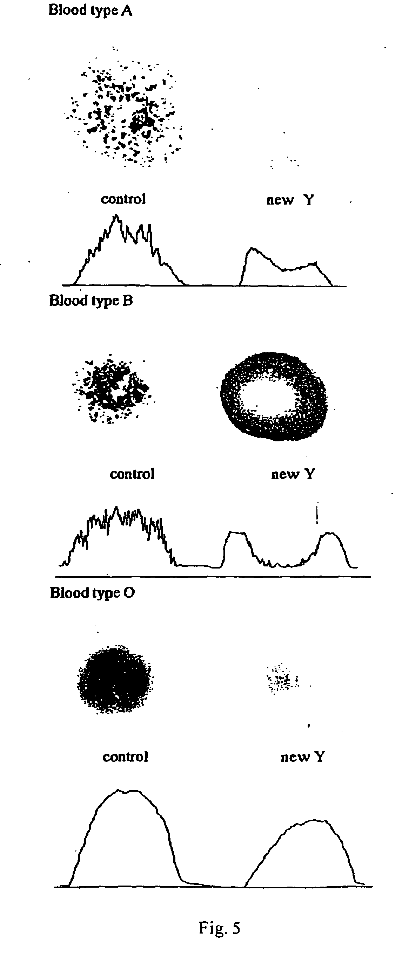

A typical antibody has a Y-shaped structure of Y consisting of two pairs of light chain and heavy chain; this fundamental structure is maintained by S-S bonds. Serum antibody reaction in different blood types (A, B and O) is historically important in order to understand various kinds of antigen-antibody reactions. In this respect, an effect of Yoshixol (4 µl) was investigated on standard human blood of A type, of B type and of O type (400 µl, respectively). When the antiserum of each blood type was not treated with Yoshixol, the usual results were obtained, that means: anti-A blood serum caused a blood aggregation when added to human A type blood; anti-B blood serum caused a blood aggregation when added to human B type blood; and both kinds of anti serums did not cause blood aggregation when added to human O type blood; as demonstrated with Figure 5a). But, when the same treatment was performed with each Yoshixol (4 µl) each treated antiserum not aggregation of sample blood of each blood type, could be observed and usual judgment of blood type (A or B or O) was impossible as demonstrated with Figure 5b). However, when the primary structure of A or B or C blood type antiserum was investigated by means of electrophoretic analysis, not any change in the primary structure of antiserum could be found, even after treatment will Yoshixol. This result is interpreted that Yoshixol has an inhibiting or blocking effect on the antibody function which effect does more interfere with the multi-dimensional structure of the antibody than with the primary structure of the antibody.

### Effect of Yoshixol on physiological function of vasopressin and insulin

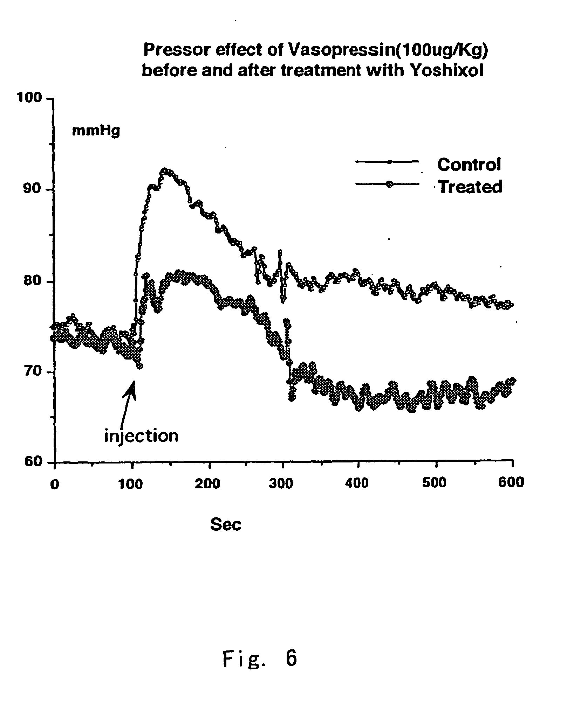

Each vasopressin and insulin has a physiological effect such as a increase of blood pressure and a decrease of blood sugar level, respectively. An effect of Yoshixol on these physiological functions was investigated by in-vivo injection of each hormone treated or non-treated with Yoshixol in rabbit. When non-treated vasopressin (100 ng/Kg, Sigma Co. Saint Louis, Missouri, USA) was injected intravenously, blood pressure increased by 15 to 25 mmHg and this increased level was maintained for about 25 minutes. But, vasopressin (100 ng/Kg) treated with Yoshixol (4 µl) caused an increase in blood pressure of only about 5 mmHg (as demonstrated with Figure 6). An injection of non-treated insulin (5 units/Kg, Novo Ltd.) caused a maximal decrease of blood sugar level of 45 mg/dl. When insulin treated with Yoshixol (4 µl) was injected, the decrease of blood sugar level was only 12 mg/dl. These results are interpreted that Yoshixol las an inhibiting or blocking effect on bioactive low molecule peptides and hormones the functional specificity thereof is due to certain amino acid sequences.

### Effect of Yoshixol on primary structure of proteins

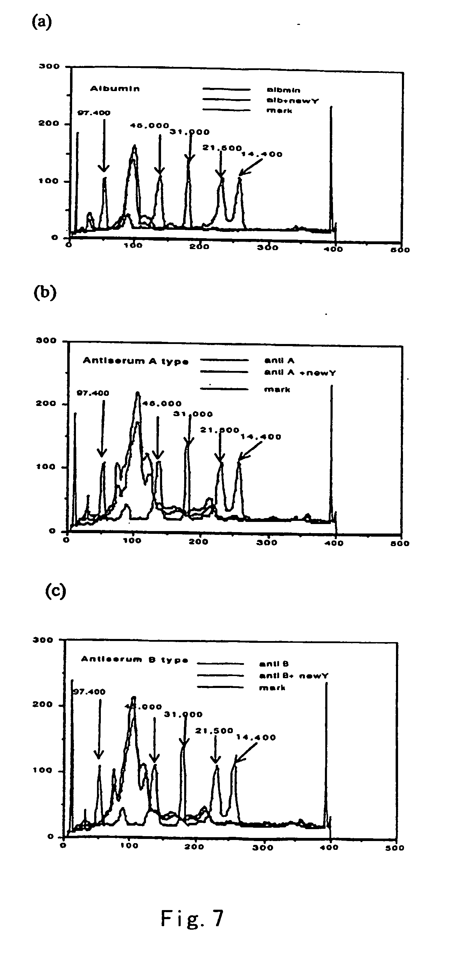

Changes in molecular weight of the following macromolecular proteins (1 molar solution) before and after treatment with Yoshixol (4 µl) were investigated after not denaturation by electrophoretic analysis of non-SDS wide page. In each case 1 ml solution of human defibrinated serum, bovine albumin, human fibrinogen, myoglobin, anti-A blood type serum and anti-B blood type serum (Green Cross Co.) were tested. Even after treatment with Yoshixol, the distribution of the molecular weight on electrophoretic analysis was identical to the molecular weight of the non-treated macromolecules proteins (as demonstrated with Figure 7). This result shows that Yoshixol does not directly change a primary structure of a biological proteins having a macromolecular structure.

### Concerning anti-microbacterial effects

### Effect on methitilin resistance staphylococcus aureus, (MRSA)

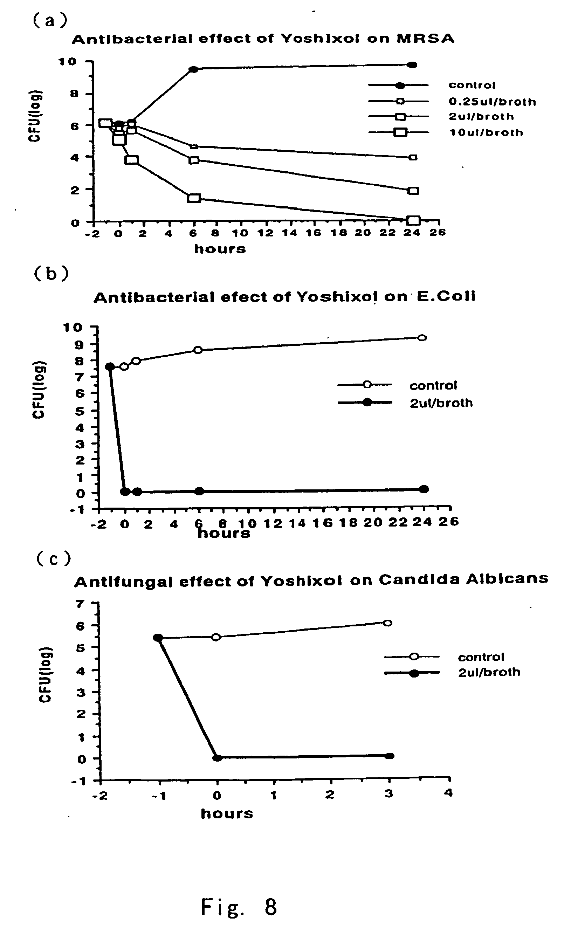

Effect of Yoshixol on MRSA was investigated by using the original strain (stock no. SCK18) which was isolated from sepsis patient and which was confirmed to cause severe circulatory shock in test animals such as mouse, rat, rabbit and dog. A brain-heart infusion agar served as culture medium. Yoshixol was added in amounts between 0.25 µl and 10 µl per 1 ml of culture medium, Colony formation unit (CFU) was measured after a 24 h lasting incubation at 37°C; the initial CFU was 10⁸. CFU in Yoshixol non-treated group as control group increased to 10¹⁰ during a 24 h lasting culture; however, CFU in Yoshixol treated group (0.25 µl Yoshixol per 1 ml culture medium) decreased to 10⁴. Additionally, at dosages of 2 µl and 10 µl of Yoshixol per 1 ml of culture medium CFU were 10² and zero respectively (as demonstrated with Figure 8a). The result is interpreted that Yoshixol has a strong bactericidal effect on MRSA which has acquired resistance to other antibiotics in gram positive bacteria.

### Effect on E. Coli

Effect of Yoshixol was investigated by using E. Coli (E. coli strain no. W3110). A brain-heart infusion agar served as culture medium. After addition of Yoshixol (2 µl per 1 ml of culture medium), a colony formation unit (CFU) was measured after a 24 h lasting incubation at 37°C; the initial CFU was 10⁸. CFU in the Yoshixol non-treated group as control group increased to 10¹⁰ during a 24 h lasting culture; however, CFU in Yoshixol treated group (2 µl per 1 ml of culture medium) decreased to zero after a 1 h lasting culture, and was still zero after 24 hours (as demonstrated with 8b). The result is interpreted that Yoshixol has an extremely strong bactericidal effect on E. coli which is gram negative bacteria.

### Effect on acid-fast bacteria

Effect of Yoshixol was investigated by using atypical mycobacteria (Mycobacterium Rapid Grower). The size of a inhibition zone of proliferation on brain-heart infusion agar was measured. The formation of a inhibition zone of proliferation occurred at 0.2 µl of Yoshixol per 1 ml of the culture medium and revealed a diameter of 22 mm at 2 µl. Accordingly, Yoshixol has a strong bactericidal effect on atypical mycobacteria.

### Concerning an antifungal effect of Yoshixol

2 µl Yoshixol were added to 1 ml of culture medium containing Candida Albicans (10⁶ CFU/ml) and Sabouraud broth (5 ml). In the Yoshixol non-treated group, CFU did not change after a 3h lasting incubation and tended to increase. However, in Yoshixol treated group, CFU was zero after 1 h lasting incubation. This level of zero was maintained even after 3 further hours incubation. Accordingly, Yoshixol has an strong antifungal effect.

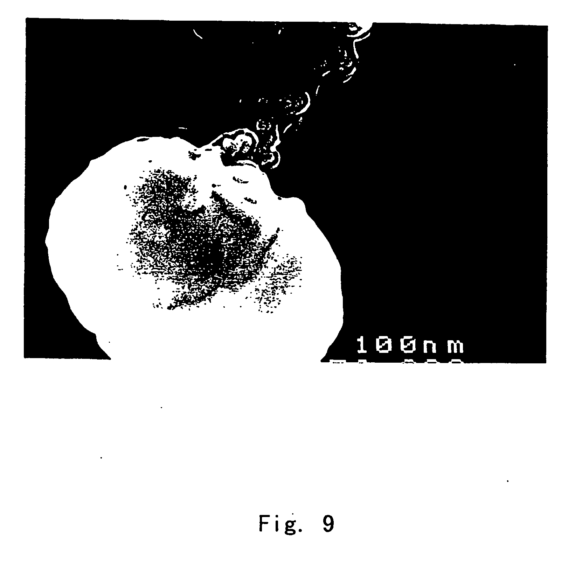

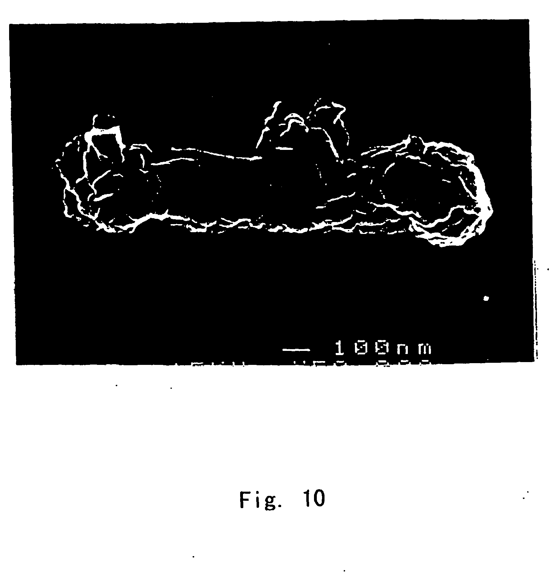

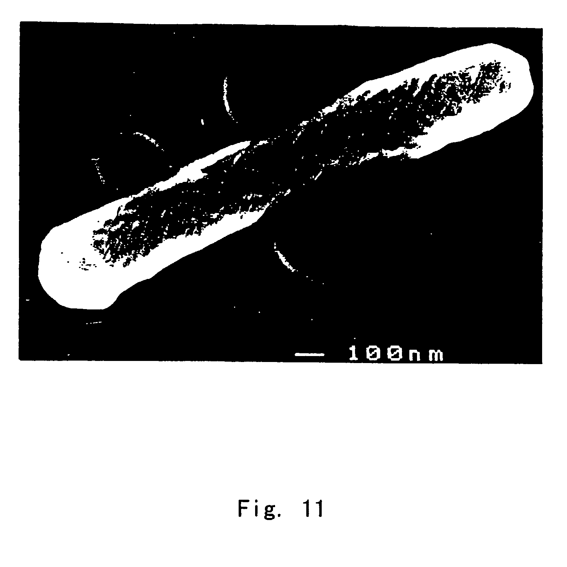

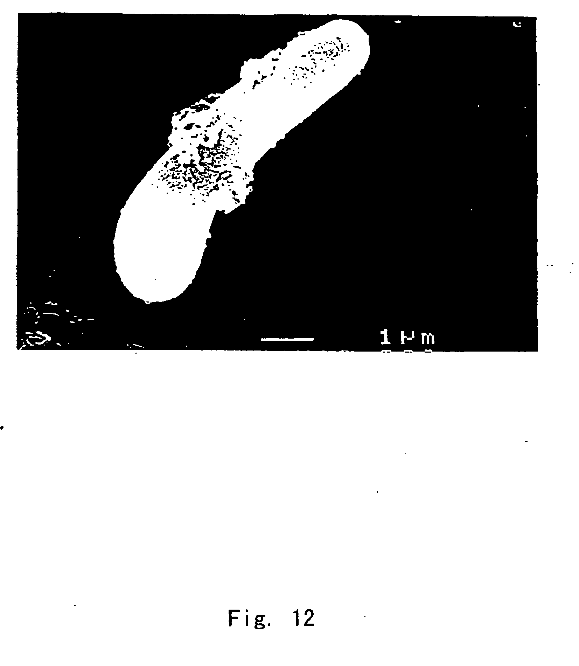

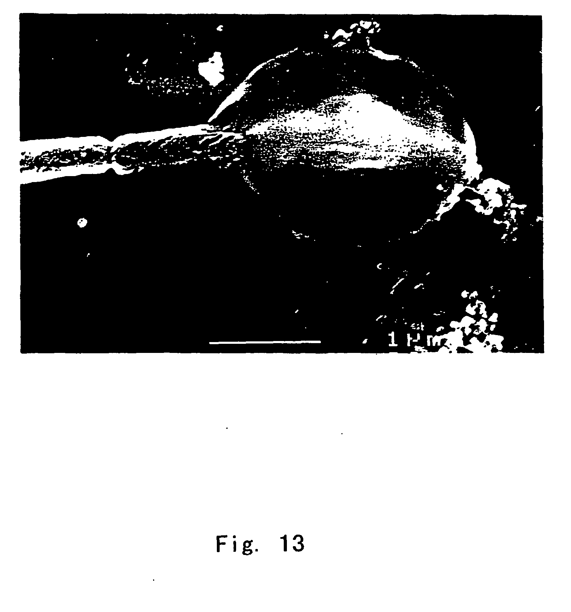

Those antibacterial effects are related to histological aspects of cell death. Thus, in the case of MRSA, the investigation by means of a scanning electron microscope showed characteristic histological images after Yoshixol treatment. A group of MRSA bacilli was separated from an individual bacterial cell, and small particles having a size of 10 to 50 nm were sprayed out in an explosive like manner form the surface structure of the individual cell (Figure 9). At the final stage of cell death, even smaller particles than the above stated particles were dispersed in a concentric circle, like fireworks. Such characteristic features were also observed by means of a transmission electron microscopic recording. Scanning electron microscopic observation of E. Coli showed that the surface of E. coli has lost its smoothness, and small particles of about 10 to 50 nm have been formed; some expanded prominences appeared on the surface (Figure 10). Further in the case of E. coli, an adhesive group of bacilli was separated, and the final stage provided destroyed small particles. Even in cases of acid-fast bacteria (Figure 11) and Candida albicans (Figure 12) similar histological features were observed after the treatment with Yoshixol. The histological observation of pseudomonas aeruginosa after treatment with Yoshixol showed that the bacilli were swelled like a balloon and were finally ruptured so that bacilli components formed small particles Figure 13). Accordingly, Yoshixol has a disinfective and antimicrobacterial effect, and its mechanism for cell death is different form the antibacterial and bactericidal mechanism as generated by conventional drugs resulting in denaturation, necrosis and/or coagulation. The characteristic mechanism which Yoshixol exerted on microbacteria is to inhibit an adhesion between individual bacteria and to form small particles by destroying the bacilli components in a manner like erupting, explosing and/or ballooning depending of the morphogenesis of each bacilli. These histological findings are identical with results obtained by zeiosis or apoptosis. Thus, it appears possible to apply Yoshixol as an effective antibacterial and/or bactericidal agent which does not induce a variability and drug resistance

### Concerning to a disinfection sterilizing effect of Yoshixol

An antimicrobacterial effect of evaporating components of Yoshixol, was studied 50 µl Yoshixol were distributed in the whole space of the schale. The bacilli of methitiline resistance staphylococcus aureus, E. Coli, Candida albicans and acid-fast bacteria (some of the same strains as mentioned above) were cultured in ordinary gelatin agar, in BHI (brain heart infusion) culture medium, in heart infusion culture medium and in Sabouraud culture medium. The prolifelation of each bacteria during a 24 h incubation period was measured. However, a direct contact between Yoshixol and the bacteria dessiminated culture medium was avoided. A piece of filter paper has been dipped in to an adequate amount of Yoshixol, and placed in such a manner that the position of culture medium was upside and the soaked paper was placed on the base of the schale. Subsequently, a proliferation of each bacteria mentioned above did not occur due to evaporating components of Yoshixol from the base of the schale in a state of constant-temperature at 37 °C, but not due to a direct contact diffusion. This result is interpreted that a volatilizating and/or evaporating component of Yoshixol provides a strong bactericidal and/or sterilizing effect even by use via room air in the living space.

### Concerning effect of Yoshixol on formation of nitric oxide

As recently reported nitric oxide (NO) generated in the living body may generate anticancer effect, bactericidal effect, inhibitory effect of antigen- antibody reaction and cardiovascular effect. Wether NO is involved in the above demonstrated Yoshixol effects, such as effect on blood aggregation, effect on fibrin formation and antibacterial effect on MRSA, was investigated by using NG-methyl-L-arginine (NMLA) which served as blocking agent against formation of NO. The common use of 4 µl of NMLA (1 mol or solution) and Yoshixol, decreased the blood aggregation effect, the fibrin formation effect and the bactericidal effect of Yoshixol by 10 to 20% as compared to the effect provided by Yoshixol alone in absence of NMLA. This result is interpreted that Yoshixol has a supporting effect on the in vivo NO formation. The additional amount of NO due to the Yoshixol effect contributes to about 10 to 20 % of anticancer effect, bactericidal effect, anti-viral effect and effect on antigen-antibody reaction of Yoshixol.

### Effect of Yoshixol on bacteriophage

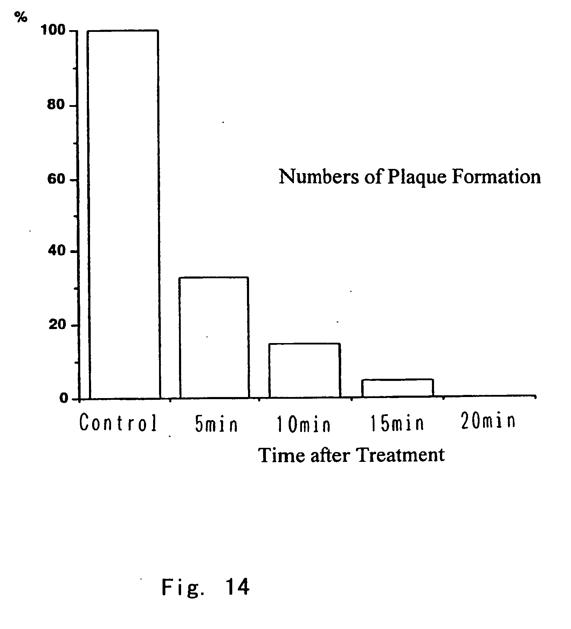

Yoshixol (4 µl) was added to 1 ml phage solution (E79 double-chained DNA phage) containing about 10⁷ phages per 1 ml; the preparation was well mixed. Then, aliquote 10 µl amounts of the mixture were transferred into a test tube and maintained for a 5 min, or 10 min, or 20 min, or 30 min reaction of Yoshixol with the pages solution. Thereafter, the reaction was stopped by quickly adding 10 ml of medium. Then, each 100 µl of the diluted phages solution was transferred into a test tube provided with a upper plate made of warmed gelatine agar, which was mixed with 100 µl of bacteria indicating solution (pseudomonas aeruginosa) cultured for one night. Then, the adjusted solution of the phages in the test tube was dropped on the agar plate and, a homogeneous surface of the upper agar plate was obtained by smoothly rotating the plate on the table. After resting on the table for about 10 min when multilayer gelatine agar became sufficiently solid, the samples were transferred into an incubator maintained at 37°C. After one night incubation, the number of plaques were counted. As a result, the numbers of plaques in the 5 min-treated phage solution, in the 10 min-treated phage solution and in the 15 min-treated phage solution decreased to 33%, 15% and 6% of the plaques formation in the non-treated phage solution, respectively. Moreover, when the phage solution was treated with Yoshixol over 20 min, plaques formation could not be found (Figure 14).

### Concerning antiviral effect of Yoshixol

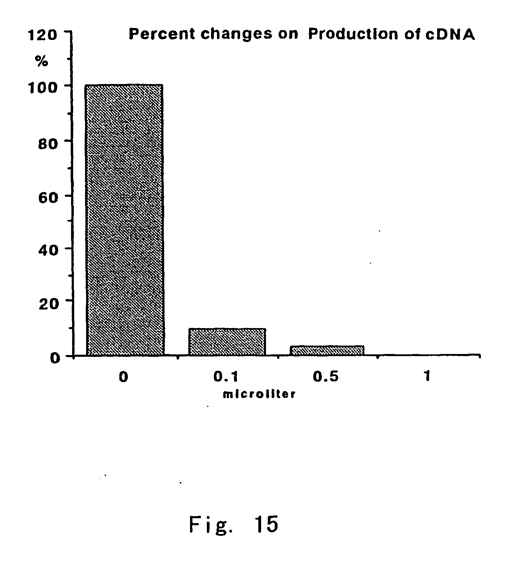

Each, structures of single strand DNA, double strand DNA and mRNA of E. coli bacteriophage (1ml of 1 mol or solution), were prepared as Yoshixol non-treated sample and as Yoshixol treated sample (4 µl Yoshixol) and were investigated by means of scanning and transmission electron microscope. In addition, changes in proliferation and in morphological aspects of E. Coli were investigated by means of scanning and transmission electron microscope); E. coli, has been infected with single strand DNA, or with double strand DNA or with mRNA; the infected E. coli were investigated before and after treatment of each phage with Yoshixol. Here, 4 µl Yoshixol were added to 1 ml of each phage solution adjusted to 1 mol or concentration. As a result, a characteristic helical and multi-dimensional structure of the single strand DNA, of the double strand DNA and of the mRNA of E. Coli bacteriophage changed to a simple structure after treatment with Yoshixol and a distance of the chain markedly separated. The histological investigation of E. coli, showed an adhesion of the phage on the bacterial surface and/or intrabacterial existence was observed in the Yoshixol non-treated group, but not in the Yoshixol treated group. In addition, an effect of Yoshixol on chicken myeloblastosis virus (AMV) reverse transcriptase (Gibco Co. Getesburg, Maryland, USA) was investigated by measuring the amount of cDNA synthesis, which was amplified by PCR of RNA(5 µg) and AMV reverse transcriptase (25 units). Here, 0.01 µl Yoshixol were added to 25 units sample of the AMV reverse transcriptase. The expected formation of cDNA was found in the non-treated group, however, the amount of formed cDNA decreased to 10% of the expected amount, due to the Yoshixol treatment (Figure 15). This result is interpreted that Yoshixol has an inhibitory effect on the self prolifelation ability within host cells, destroying virus actions, and has an inhibitory effect on the adhesion of host cells, and has an effect which causes a change in multi-dimensional structure of genes with virus oneself.

### Concerning anti-cancer effect of Yoshixol

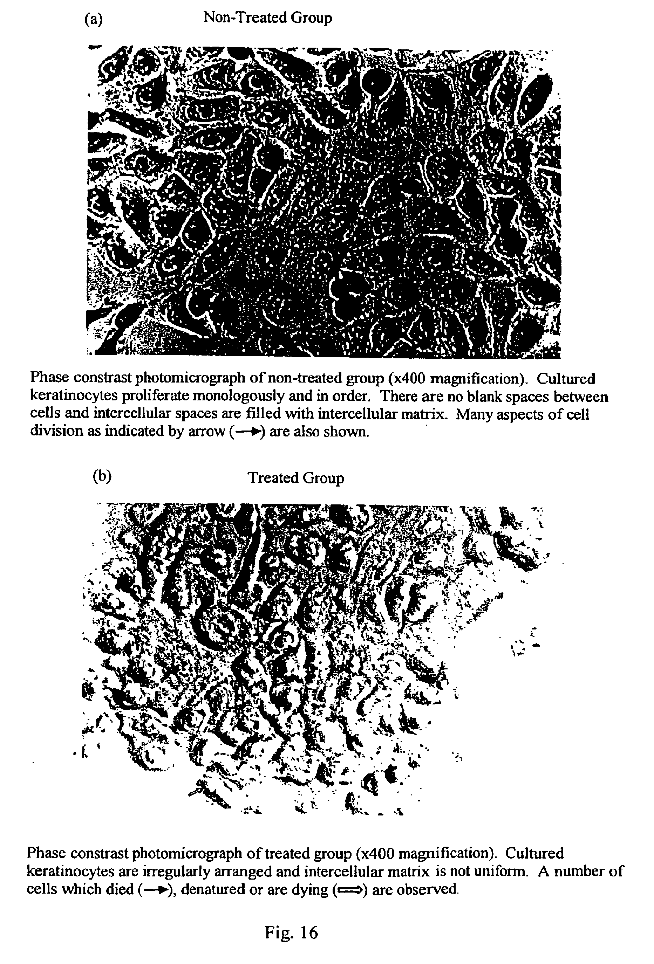

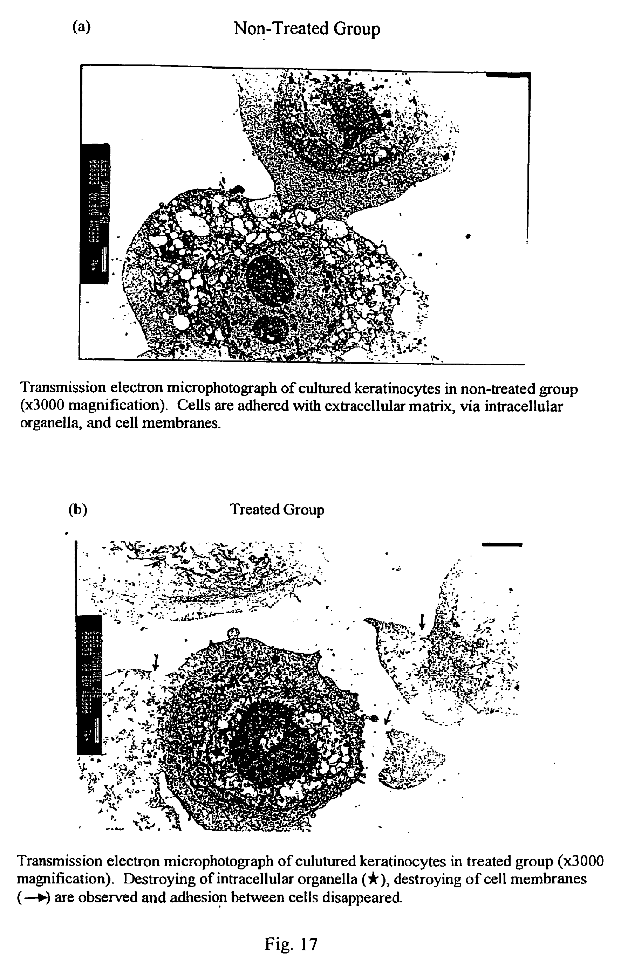

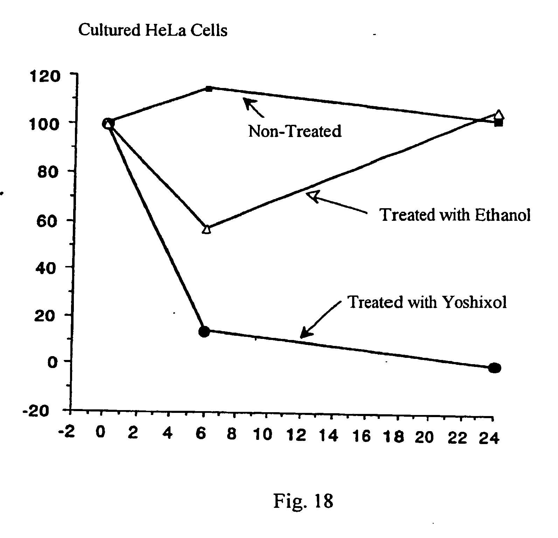

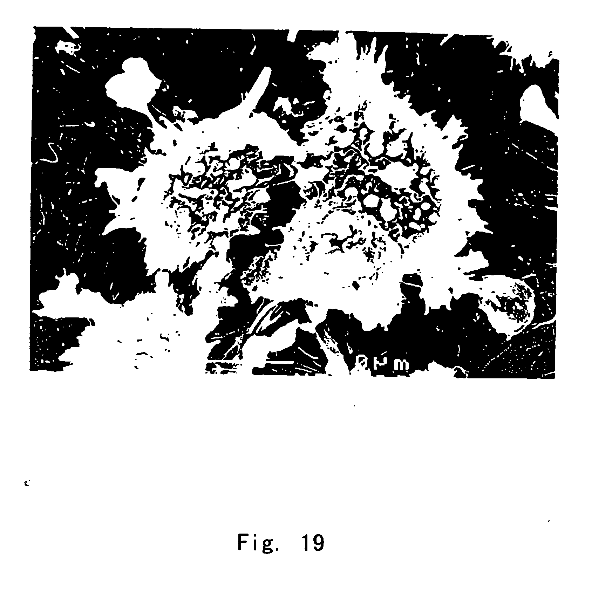

Adhesive factors between cells play an important role on the prolifelation of malignant tumor cells and on the formation of metastases. Intracellular structure of cultured keratinocytes are used to investigate the adhesive factors between cells. The keratinocytes have been isolated from a human skin and cultured. The structure between cells and cellular aspect of keratinocytes (5 days passed after culturing of second generation) were investigated by means of phase microscope (Olympus Co., IMT-2), transmission type microscope (JEOL, Ltd. JEM 1200, EXII) and scanning type microscope (JEOL, Ltd. JSM-6000F). In the Yoshixol non-treated group the cultured cells proliferated monologously and in order like a stone wall. Normal aspects of intracellular organella and mitosis were found. No blank spaces between cells, and no filling with intracellular matrix could be found (Figure 16a, 17a). In contrast, the cultured cells of the Yoshixol treated group (4 µl of Yoshixol) showed diverse irregularities. Many cells showed destroyed cell membrane and destroyed intracellular organella (Golji apparatus, rough endoplasmic reticulum, cytoskeleton consisting of tubulin), and the coupling between cells has been seperated to an irregular order. In addition, the extracellular matrix has been dispersed in an irregular form from cells forming particles of various sizes. (Figure 16b, 17b). Similar observations were found in cultured HeLa cells (American Type Culture Collection, ATCC No. CCL2, Maryland, USA, referred Figure 18) and cultured mouse hepatoma cells. This result is interpreted that Yoshixol may suppress cell division and prolifelation, and may block adhesion between cells. Further, Yoshixol has an inhibitory effect on cell prolifelation and on the formation of metastases such as tumor cells. Additionally, the found histological aspects suggest that the cells destroyed due to Yoshixol are far from those cells destroyed by necrosis in cell death and reported of above (Figure 19); here, the destroyed cells are more close to the picture reported as natural cell death or apoptosis (these morphologic images were investigated in various bacteria mentioned above). Further, these findings show that small particles (10 to 100 nm) destroyed from cell composition were cleaned up by phagocytosis of macrophages and/or lymphocytes, resulting in reuptaking those particles and being recycled into the natural living organism.

### Concerning preservative effect of Yoshixol on organs and tissues

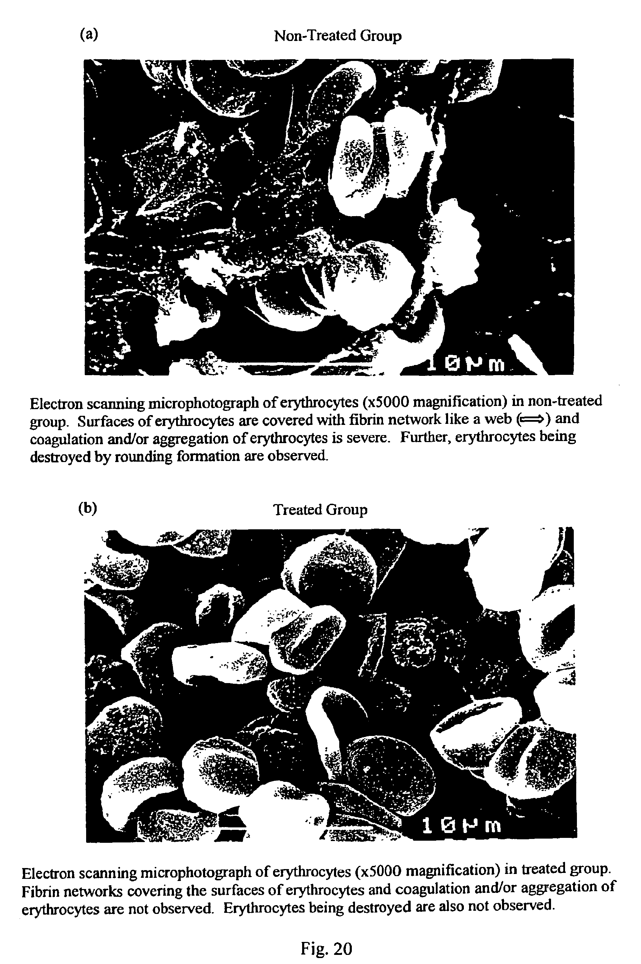

Each, 5 ml of human blood from a cubital vein, was transferred into a 10 ml vacuum tube. The vacuum tubes were divided into two group; one group is the Yoshixol nontreated group and the other group is the Yoshixol treated group (4 µl Yoshixol). Subsequently to a storing at room temperature for 30 min, or for 1 h or for 1 month the changes in morphological aspects of the stored blood, were observed by means of a transmission type electron microscope and by means of a scanning electron microscope. When the blood sample was not treated with Yoshixol, coagulated masses were found 30 minutes after the sampling and, normal aspects of erythrocytes could not be found. Contrarely, the sample mass appeared like a destroyed and aggregated mass. However, erythrocytes which were treated with Yoshixol did not show an aggregation with fibrin networks. Many stomatocytes (lip-like erythrocyte) and echinocyte were observed apparently due to a lack of pyruvate kinase. However, cell membrane and intracellular organella were preserved normally over 1 month lasting storage Figure 20). This result is interpreted that blood treated with Yoshixol may be stored even at room temperature and that Yoshixol has an preservative effect for organs and tissues which are functional units of body.

### Concerning inhibitory effect of Yoshixol to the rejection reaction of transplanted heterogeneous skin

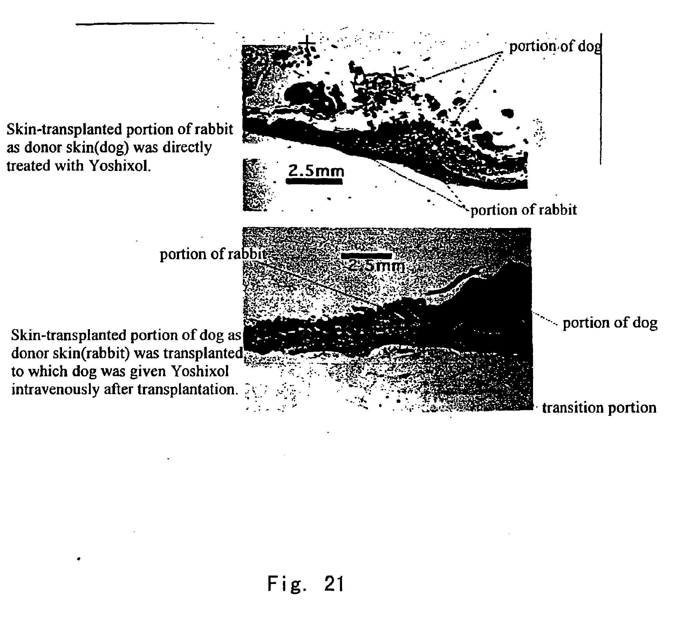

Transplanted skin was macroscopically and microscopically observed for 3 months. In one test, a piece of the entire skin layer (diameter 3 cm) from the back region of a rabbit was transplanted to the back region of an adult mongrel dog. In another test, a piece of the entire skin layer (diameter 3 cm) from the back region of an adult mongrel dog was transplanted to the back region of rabbit. At first, each piece of skin from rabbit or dog was treated with 5 ml of physiological saline with 10 µl of the test solution for 2 minutes and was then transplanted to each recipient by the surgical suture. Two kinds of the test solution were prepared for the above treatment; a control solution, consisting of 2ml of polyoxyethylene (20) sorbitan monoolate 1 (Wakou Jyunyaku Co.) which is identical to Tween 80 (ICI Ltd.) and 88 ml of physiological saline, and a Yoshixol solution, obtained by adding 10 ml of Yoshixol to the fore-mentioned control solution. Further, 10 µl of Yoshixol was directly applied on the transplanted wound for 3 days after implantation. Desquamation of each transplanted skin and treated with the control solution started on the 3rd to 5th day after implantation; the transplanted wound made an extremely dirty impression with tissue necrosis and inflammation reaction. After one week, the transplanted skin showed mummification and was completely dropped out from the recipient 10 days after the implantation. Then, the implantated wound of the recipient was cured with a self regenerated skin of each recipient after 3 months. On the other hand, dipping piece of transplanted skin into the Yoshixol solution, made the treated skin soft and move thicker. The suture procedure with surgical needle could be easily done. The transplanted skin did not show a desquamation and deciduation within a period of 2 weeks after the implantation; not any inflammation reaction could be found within a period of 1 month after the implantation. The transplanted wound appeared to be covered with chitin-like and lustered collagen-like substances so that the wound was cured to a level which could not find a boundary with a skin of recipient (upper panel of Figure 21). These results show that Yoshixol can inhibit a rejection reaction of the transplanted tissue by pretreatment of tissues or organs of donor with Yoshixol when a heterogeneous transplantation of tissues or organs or skin is performed. In another test, an entire skin layer (diameter 3 cm) of rabbit was transplanted and sutured to the back of a dog as the recipient. Thereafter, the dog was observed during a period of 3 months after the implantation. The rabbit skin did not receive the pretreatment with Yoshixol. The recipient dog received intravenously 10 µl Yoshixol/kg per day for one week, but did not receive any kinds of conventional antibiotics and/or immunosuppressants. It was found that the transplanted skin was implanted on the back of dog over 1 month without any rejection reaction and bacterial infection. An outer layer of the transplanted skin was faded 3 month after the implantation similar to stripping a thin membrane. But, though an appearance of hair was not found in a wounded skin, well epithelialization was found macroscopically and microscopically (lower panel of Figure 21). In addition, by this intravenous administration of Yoshixol, adult mongrel dog also became vigor, youthful and showed a good appetite in comparison with behaviors before the treatment. This result showed that Yoshixol can inhibit a rejection reaction of the transplanted tissue and can improve the physical status due to the intravenous administration, and can inhibit infections and can improve an implantation effect.

### Concerning thrombolytic effect of Yoshixol

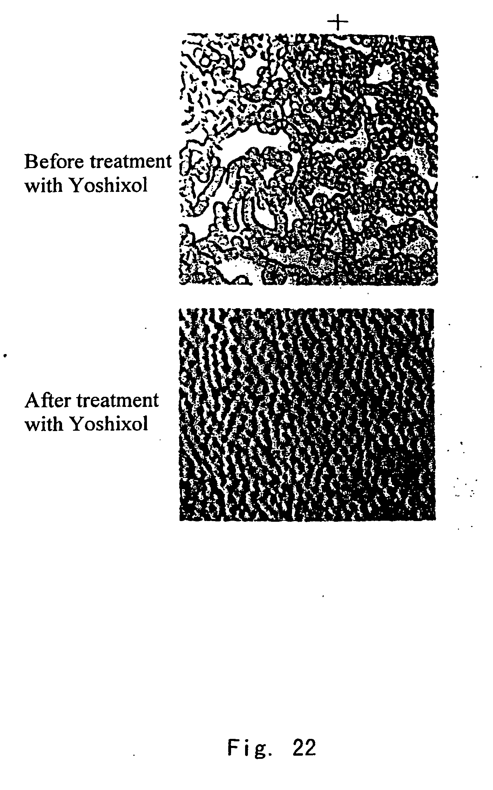

1 ml of human blood or canine blood were sampled by a syringe and transferred into the Petori-schale (grainer lab. GmHB) in order to observe thrombus formation and rouleaux formation under a phase contrasted microscope (IMT-2, Olympus Co.). Then, 1 µl of Yoshixol was added in the blood sample when all visual fields could not be discriminated as a normal individual erythrocyte by thrombus formation. Then, a thrombolytic process of the thrombus was investigated and the process was recorded to a video tape via a collar video camera (CCD-IRIS, Sony Co.). The video pictures were analyzed as a liquid phenomena by an original software of dynamic state analysis. When Yoshixol was added in the sample, the thrombus which has consisted with a mass has completely individualized to each erythrocyte via a reversed process of the thrombus formation. And, while original form of erythrocyte was recovered, each erythrocyte was marvelously dispersed from thrombus. In addition, hemolysis due to swelling and rupturing each erythrocyte did not occur. Each position of the individual erythrocyte did not disturb a position of other erythrocyte , and as a whole there was a dynamic state with a defined order (as demonstrated with Figure 22). When analyzing this process by a dynamic image processing or microscopically, the pattern of this process showed a behavior as a dissipative mass similarly to phenomenon of liquidarization of solid materials or gelatinization phenomenon. This result is interpreted that Yoshixol has a potential to improve circulatory hindrance. Thus, Yoshixol may be applied as a thrombolytic agent as well as a inhibitory agent for thrombus formation.

### Concerning effect of Yoshixol on improving metabolism

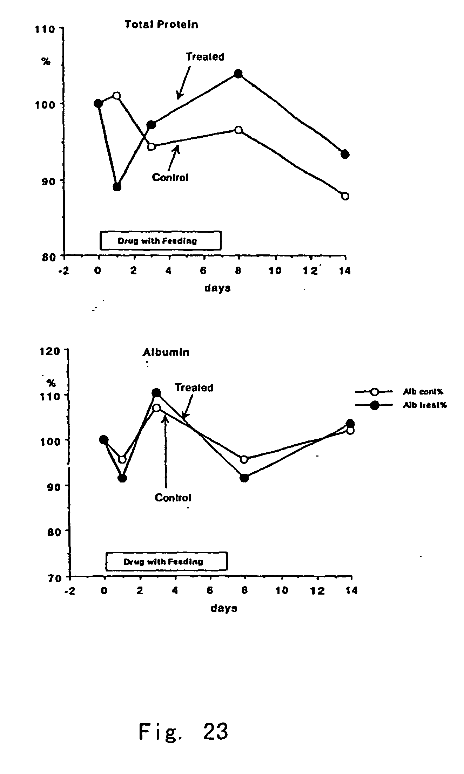

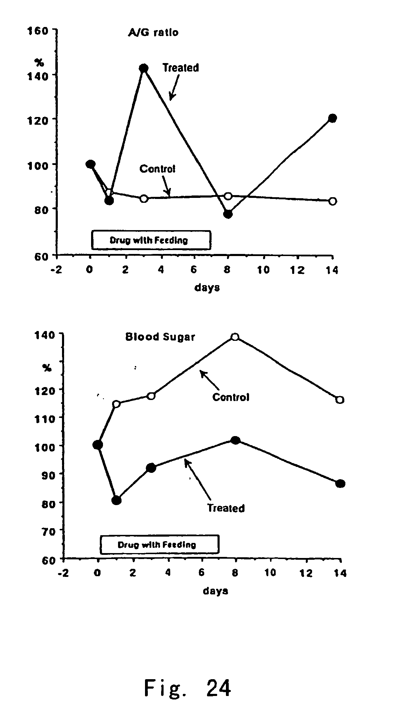

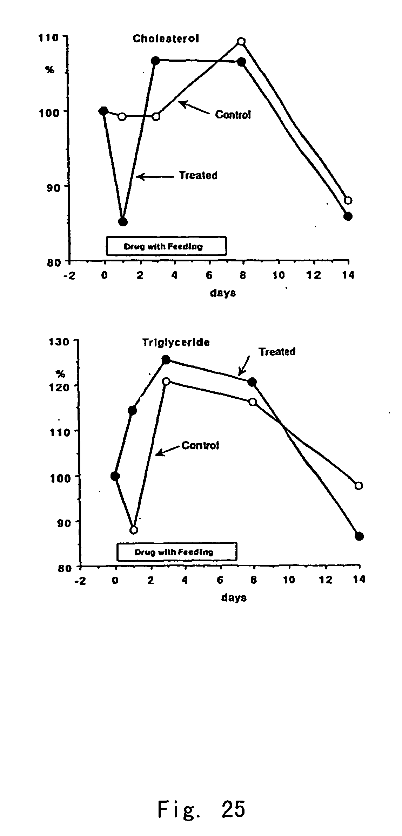

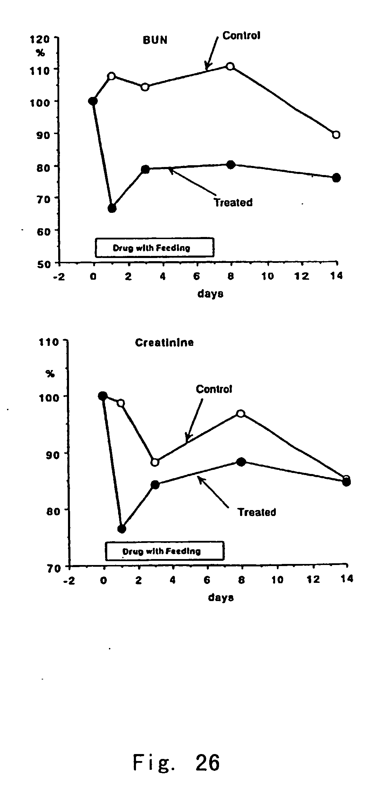

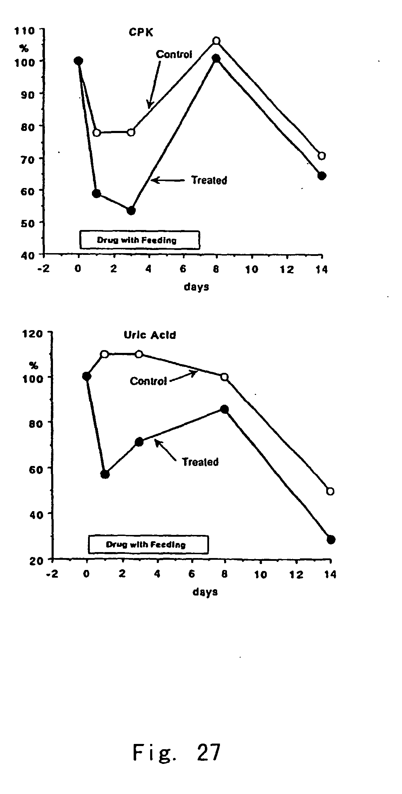

10 µl per body weight of Yoshixol was mixed with 100 g of dog foods (" dog foods < beef >", produced in New Zealand, imported by Daiei, Inc., according to by pet food fair trading committee standard). This food was given to the beagles as their feedstuff. Oral administration was continued for 1 week, and sampling of blood was performed before, 1 day after and 3 day after administration, and 1 day after and 7 days after the stop of the administration. Further, red blood cells, white blood cells and platelets were counted. Also, total proteins (Byuret method) in a blood serum, albumin (BCG method), urea nitrogen (GLDH-UV method), creatinine (enzymic method), glucose (GDH method), total bilirubin (enzymic method) are measured. GOT and GPT (JSCC sub method), TTT and ZTT (standard method of research project of liver function), cholinesterase (DMBT method), total cholesterol (PDO enzymic method), triglyceride (PDO enzymic method), uric acid (urikase PDO method), serum iron (nitroso PSAP method), creatinine phosphokinase (SCC sub method), sodium, potassium and chloride (ISE dilution), inorganic phosphorus (enzymes-UV method) and calcium (OCPC method) were also measured. As a result, any changes in numbers of red blood cells, white blood cells and platelets could not be found. Moreover, any changes in concentration of total bilirubin, GOT, GPT, TTT, ZTT, cholinesterase, serum iron, potassium, sodium, chloride, inorganic phosphorus and calcium could not be found. However, total proteins increased 3 days after administration of Yoshixol followed by recovery to a same level in the control 1 week after the stop of administration. Though albumin level did not change (as demonstrated with Figure 23), albumin/globulin ratio was elevated 3 days after administration (please refer to upper panel of Figure 24) indicating of newly producing globulin. Moreover, though blood sugar level was elevated by 15% of the control group 3 days after taking feedstuff, this elevated blood sugar level could not be found in the treated group with Yoshixol and the rebound phenomena of the glucose metabolism did not occur 1 week after the stop of administration (please refer to lower panel of Figure 24). A difference in concentration of total cholesterol and triglyceride between groups did not occur (as demonstrated with Figure 25). But, levels of nonproteins nitrogen and creatinine in the serum showed lower value (70-80% of the control) since 1 day after administration (referred Figure 26), and level of uric acid also was maintained at lower level during the administration. In addition, creatinine phosphokinase fell (referred Figure 27), and enzymes such as γ-GPT, GOT, GPT also fell. TTT and ZTT did not show abnormal values. During this administration of Yoshixol and 1 weeks after stopping administration, any abnormal behaviors, diarrhea, vomiting and bloody feces, loss of the weight and appetite loss were not observed. Moreover, even though 3 µl per body weight of Yoshixol was administered for one month to the beagles and the parameters of blood samples were observed during 3 months, a similar effect of Yoshixol to the above experiments could be obtained even though there was a difference of the time-lag and a degree. These results are interpreted that oral administration of Yoshixol influences several effects to improve metabolism and nutrition of saccharides, lipids and proteins. Thus, Yoshixol may be applied as therapeutic drug into protective agents for cellular function as well as agent for diabetes, kidney diseases, heptic diseases and hypoproteinsemia.

### Concerning softening and flexibility effect of Yoshixol on skin

A piece of skin from rabbit and dog, was dipped in 5 ml of physiological saline with 10 µl of the test solution. This test solution was prepared from 88 ml of physiological saline with 2 ml of polyoxyethylene (20) sorbitan monoolate 1 (Wakou Jyunyaku Co.) that is identical to Tween80 (ICI Co.) as the basic solution of control. As the treated solution, 10 ml of Yoshixol was added to the basic solution. When each skin sample was dipped in the test solution for approximately 1 to 2 minutes, the treated skin became soft, wetly and thicken. Surgical stitching by the needle was easily to be inserted and transfixed in the skin. Additionally, structure damages of each skin could not be found histologically. Further, the sample treated with Yoshixol proved as a fresher sample by hematoxylin eosin staining in contrast to the skin sample treated with the control solution. Moreover, a similar observation on histological pictures of each sample was obtained even when each skin sample was thawed at 20 °C after storage of each sample at 4°C in the refrigeration for one month. This result is interpreted that Yoshixol may be applied as a flexibility promoter and/or softner of the tissues and/or substances which have fiber structures such as skin and, that Yoshixol may be used as preserving agent for organs.

### Concerning inhibitory effect of Yoshixol on flagellum motility of spermatozoa

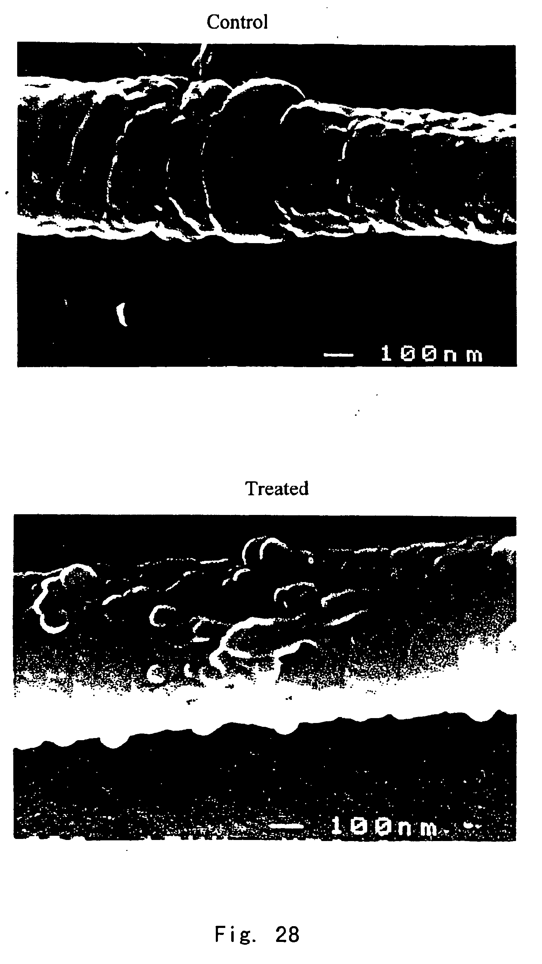

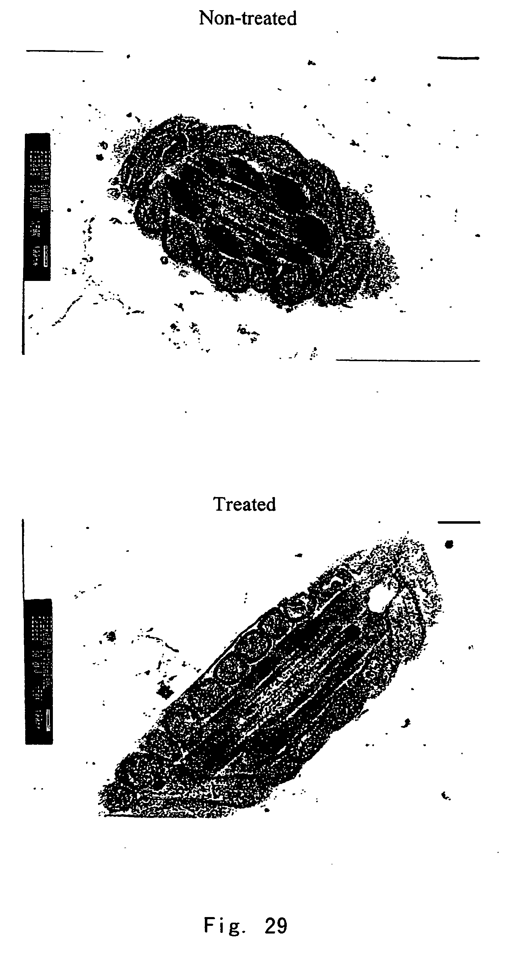

Spermatozoa (seed japanese bull ID: Sinmorihide < registration No.Zen-Wa-Kuro1114, lot No.84Y28>) which has been conserved in liquid nitrogen was thawed at 35 °C. After thawing, 1 ml of semen was transferred to Petori-schale (grainer lab. GmHB) and, flagellum motility was observed by means of a phase contrast microscope (IMT-2, Olympus Co.) and was recorded by a video film via a collar video camera (CCD-IRIS, Sony Co.) so that wave cycles and speed of the flagellum were analyzed by an original software of dynamic state analyzer. While a head of normal spermatozoa was transferred by regular movement of a flagellum at the speed of 0.3 to 1.5 mm per second, a flagellum movement was immediately inhibited after addition of 1 µl of Yoshixol, so that velocity of spermatozoa became zero. However, though a flagellum movement was completely stopped, the rotation of the head which occurs at the centriole (near junction between head and tail) was found during a few 10 seconds. Further, a speed of this rotation gradually was decelerated with progress of a time. This result is interpreted that Yoshixol is related to physical property and function property of a substance (for example, acting such as contraction protein and hydrolysis reaction of ATP as energy source) which is concerned with a flagellum movement of spermatozoa, so that Yoshixol is related greatly on a movement and signal transduction of the procaryotes and eucaryotes, and on the dynamic behavior which is an essential feature of living organism. Moreover, from the knowledge that these movements of cells are involved in microfilaments and microtubles which is constricted from the proteins assembly, a morphological change of this spermatozoa due to treatment with Yoshixol was investigated by means of transmission type (JEM1200, EXII, JEOL, Ltd.) and scanning type (JSM-6000F, JEOL, Ltd.) electron microscope. The outside of normal flagellum with regular basic structure of 9+2 is enclosed with a cell membrane and, there was the axial filament which structure is related to a movement is observed inside. The axial filament has two pairs of tubles (centrum pair) that exist mainly on the center and nine double tubles that exist in a periphery (circumscription canaliculus). The surface structure contributed to the movement was observed to make ring formation likely to the doughnuts, which are configured orderly and smoothly on the surface. But, though morphological aspect as a whole of spermatozoa was not altered by the treatment with Yoshixol, many particles with a size of 10 to 30 nm on the surface were observed likely to be blowed out, when the surface structure of flagellum was investigated by a magnification. And also, ring formation likely to a doughnut in which region is contributed to the movement was not found and, adhesion of small particles were noticeable (please refer to Figure 28). Moreover, while fundamental fiber units of a flagellum were maintained on the transmission electron microscopic observations, there were a lack of continuity of the membrane which surrounds fibers of the surface and a lack of regular alignment configuration whose fiber units (myofibrille) inside of the each unit of fiber construction. And, an appearance of high-density aggregated substance was found (as demonstrated with Figure 29). Moreover, the morphologic changes in the surface and intracellular organella of the head were not found in contrast to findings of marked changes in flagellum. These results are interpreted that Yoshixol may be applied as inhibitory agent of bacteria prolifelation for procaryotes, as antibacterial agent and/or anticancer agent for eucaryotes as well as an application as the contraceptive agent which can control and/or inhibit a motility of spermatozoa. Moreover, it looks like that Yoshixol may be applied as inhibitory agent or control agent for polymerization reaction related with morphogeneis, cell adhesion and each life events (for example, immune response).

### Effect of Yoshixol on changing a physical property with macromolecule

Changes in physical properties, for example, change of glossy or shiny and luster, accurate boundary on casting formation, smoothness and homogeneity of surface, transparence, texture minuteness, change in quantity and so on of macromolecules due to Yoshixol were investigated. Here, each 100 µl of Yoshixol per 100 g of each of the following substance were added. Then, each sample was heated within a water tank until the melting; then, the melted sample was poured into a filling teeth-marks phantom of caries which is used at the odontotherapy. After cooling to solid state and taking out from a template, the surface of each sample was observed by 2 to 5 times magnification with loupe with all substances treated with Yoshixol, a fall of melting point was found in comparison with the non-treated object. In octadecanol (Wakou Jyunyaku Ltd., Osaka) with Yoshixol, glossy, shiny and luster became better and boundary also became more clear and sharp. In stearic acid (Wakou Jyunyaku Ltd., Osaka) with Yoshixol, an appearance of transparence, smooth of a surface and texture minuteness is characteristic. In lauric acid (Wakou Jyunyaku Ltd., Osaka) with Yoshixol, smoothness and transparence appeared. In dodecanol (lauryl alcohol) (Wakou Jyunyaku Ltd., Osaka) with Yoshixol, melting became markedly and casting form could not be kept by room temperature. In palmitic acid (Wakou Jyunyaku Ltd., Osaka) with Yoshixol, a soft feeling appeared. In myristic acid (Wakou Jyunyaku Ltd., Osaka) with Yoshixol, clearness of boundary and homogeneity appeared, and also in tetradecanol (miristyl alcohol) (Wakou Jyunyaku Ltd., Osaka) with Yoshixol, clearness of boundary and homogeneity appeared. In hexadecanol (Wakou Jyunyaku Ltd., Osaka) with Yoshixol, it was a characteristic that texture minuteness and transparence fell. In decanoic acid (Wakou Jyunyaku Ltd., Osaka) with Yoshixol, a rough surface and a small prominence appeared though luster and boundary clearness appeared. In polyethylene glycol 1000 (Wakou Jyunyaku Ltd., Osaka) with Yoshixol, clearness of boundary and smoothness get worse. In polyethylene glycol 1540 (Wakou Jyunyaku Ltd., Osaka) with Yoshixol, transparence, smoothness and homogeneity gets better. In polyethylene glycol 2000 (Wakou Jyunyaku Ltd., Osaka), polyethylene glycol 4000 (Wakou Jyunyaku Ltd., Osaka) and polyethylene glycol 6000 (Wakou Jyunyaku Ltd., Osaka), an increment effect of texture minuteness, transparence and expansibility was found after Yoshixol addition. In addition, luster, clearness of boundary, homogeneity of a surface, transparence and texture minuteness were found in N-isopropylacrylamide (Aldrich Co. Milwaukee, Wisconsin, USA) treated with Yoshixol. Moreover, in order to investigate an effect of Yoshixol on polymers as macromolecules, acrylate polymers were tested as one of a concrete example. Standard grade of lauryl methacrylate 471, methyl methacrylate 48, ethyl methacrylate 126, isobutyl methacrylate 140 and butyl methacrylate 320 (polymer kit, acrylate polymer standard 18336-9, Aldrich Co. Milwaukee, Wisconsin, USA) were used and were observed at room temperature. When 150 µl of Yoshixol was added to 1 g of each acrylate macromolecules, all substances become liquid and glass-like transparent and, powdered and crystal configuration disappeared so that these were changed in the homogeneous substances. In addition, when 100 µl of Yoshixol is added to the above lauryl methacrylate 471, fluidability increased immediately. Floating viscidity appeared after 2 weeks and, there was separation of solid component with translucent and fluidable component one month after adding Yoshixol although the volume of sample was decreased in the group without Yoshixol. In methyl methacrylate 48, a dried powder state disappeared immediately after addition of Yoshixol and, fluidability increased and was likely to a rice cake so that it became paste-like after 2 weeks and adhered on the wall of tube, and volume of this sample was increased after 1 month. Dried powder-like aspect disappeared even in ethyl methacrylate 126 immediately after addition of Yoshixol and viscidity with lumpy appeared immediately after addition of Yoshixol so that after 2 weeks a paste-like material adhered in the wall of tube. More transparence was increased after 1 month. Dried powder-like aspect disappeared even in isobutyl methacrylate 140 and, viscidity with lumpy transparence appeared so that viscosity was increased and it became paste-like after 2 weeks.Though the sample which adhered on the wall of tube was transparent, an unreacted part of the substrate had a color in a powdered state before treatment with Yoshixol. In butyl methacrylate 320, a powdered state disappeared and viscidity, lumpy and transparence were increased after after addition of Yoshixol. The sample adhered paste-like on the wall of tube. Though there is a difference of transparence according to the adhesive state after 2 weeks, a transparent part of the sample had a vitrified transparence. So, the sample changed to less viscidity and to a more homogeneous glass-like mass with transparence after 1 month, and the quantity of the sample also increased. In addition, changes in the physical property of other polymers in acrylate group were investigated by using standard grade polymers (polymer kit, polyacrylate standard 18338 - 5, Aldrich Co. Wisconsin, Milwaukee, USA). The kit contained poly(2-ethylhexyl acrylate), poly (methylacrylate), poly(octadecyl acrylate), poly (ethyl acrylate) and poly (butyl acrylate). Then, 500 mg of each sample was treated with 200 µl of Yoshixol at room temperature. An increase in fluidability occurred in poly(2-ethylhexyl acrylate) immediately after treatment with Yoshixol, however, color of the sample did not change. Fluidability and capacity were further increased after 2 weeks, and also the refraction of light was changed. In addition, fluidability was increased after 1 month in addition, and a capacity also was increased by twice. In poly (methylacrylate) treated with Yoshixol, an increase in fluidability and a cubic capacity was observed, and optical transparence also became better. Fluidability has been enhanced after 1 month, and a capacity increased nearely 2 to 3 times. In poly(octadecyl acrylate) with Yoshixol, a dried powder state disappeared, and the sample became homogeneous and changed to a lumpy mass. In poly (ethyl acrylate) treated with Yoshixol, fluidability was increased markedly and the mass became fluidable, and a marked increase in capacity occurred so that fluidability of the sample was increased and volume of the sample was gained by 3 times after 1 month. In poly (butyl acrylate) treated with Yoshixol, fluidability also was increased. In addition, the fluidability of the sample was further increased after 2 weeks and the sample became even more fluidable. A volume of the sample was increased by almost 2 to 3 times as well as an increment of fluidability after 1 month. Moreover, changes in the physical property of other polymers were investigated by using standard grade polymers (polymer kit 18337-7, Aldrich Co. Wisconsin, Milwaukee, USA). The kit contained poly(dimethyl siloxan), poly (vinyl acetate), poly (methyl methacrylate), poly (vinyl chloride) and polycarbonate resin. Then, 500 mg of each sample was treated with 200 µl of Yoshixol at room temperature. In poly(dimethyl siloxan) treated with Yoshixol, though Yoshixol was difficult to be mixed with the sample just after the treatment and it remains on the top as a liquid layer, an increase in a cubic capacity and fluidability occurred after 2 weeks. A viscosity and a quantity of the sample were gained after 1 month, however, optical transmission was decreased. In poly (vinyl acetate) treated with Yoshixol, viscidity was increased and the sample adhered on the wall of tube though a fluid component was observed on the bottom of the tube. However, fluid component in the tube disappeared after 2 weeks so that the sample changed to a viscous solution having transparence and an increment of transparence was observed. In addition, transparence of the sample became very stable after 1 month. In poly (methyl methacrylate) treated with Yoshixol, the sample appeared frosted glass-like and crystal like after 1 month and changed to a massive homogeneously state showing transparence immediately after Yoshixol addition. In poly (vinyl chloride) treated with Yoshixol, a dried powder state of the control sample disappeared and the sample changed to a non-uniform mass with small granules. A marked change was not observed when the sample was placed at room temperature. In polycarbonate resin treated with Yoshixol, a powder-like granule disappeared immediately after addition of Yoshixol and the sample became adhesive. Moreover, when 100 µl of Yoshixol are added to 200 mg of N-isopropylacrylamide (Aldrich Co. Milwaukee, Wisconsin, USA), Yoshixol was permeated into a crystal, likely when sugar indulges in water. Subsequently, after 2 to 3 minutes, a transparent substance was formed at the bottom of the tube and changed to a sherbet-like substance after 4 hours. Further, the sample changed after 2 weeks to a frosted glass-like appearance having less transparency.

Moreover, 100 µl of Yoshixol was added to 200 mg of polyethylene glycol phenylether acrylate (Aldrich Co. Milwaukee, Wisconsin, USA), polyethylene 125, 000 (Aldrich Co. Milwaukee, Wisconsin, USA) and polyethylene low density (Aldrich Co. Milwaukee, Wisconsin, USA) as another macromolecule of the polyethylene group. Each sample was observed at room temperature. In polyethylene glycol phenylether acrylate treated with Yoshixol, an increase in fluidability was observed just after the treatment. As time passed, an increase in optical transparence and volume of the sample was observed. Moreover, in polyethylene 125,000 treated with Yoshixol, an adhesion between granules occurred so that each granule of the sample became adhered after 2 weeks though a grained form still remained. In addition, in low density polyethylene treated with Yoshixol, the sample changed to a bigger granular from original powder-like state. Moreover, in order to investigate an effect of Yoshixol (100 µl) on macromolecules in polystyrene group, 200 mg of polystyrene 45,000 (Aldrich Co. Milwaukee, Wisconsin, USA), polystyrene 280,000 (Aldrich Co. Milwaukee, Wisconsin, USA) and polystyrene standard (Aldrich Co. Milwaukee, Wisconsin, USA) were used. When Yoshixol is added on polystyrene 45,000, powder-like granules of polystyrene 45,000 disappeared, and viscidity of the sample appeared, and the sample became transparent and a crystal structure of the sample was lost. These properties did not alter even after 1 month. Powder-like granules disappeared in polystyrene 280,000 after treatment with Yoshixol so that particle configuration of the sample was lost parallel to an appearance of viscidity. Optical transparence became better likely as a transparent glass even when 1 month has been passed. A similar change in physical property was observed in standard polystyrene. Moreover, when 100 µl of Yoshixol was added to 200 mg of hydroxylated polyethylene vinyl alcohol (Aldrich Co. Milwaukee, Wisconsin, USA), the sample changed to a lumpy mass showing a non-uniform and easy breakage from originally dried powder-like state. When time passed, the sample changed again to a dried powder-like lumpy mass, and adhered to the wall of tube. These results are interpreted that Yoshixol may improve the physical properties of macromolecules.

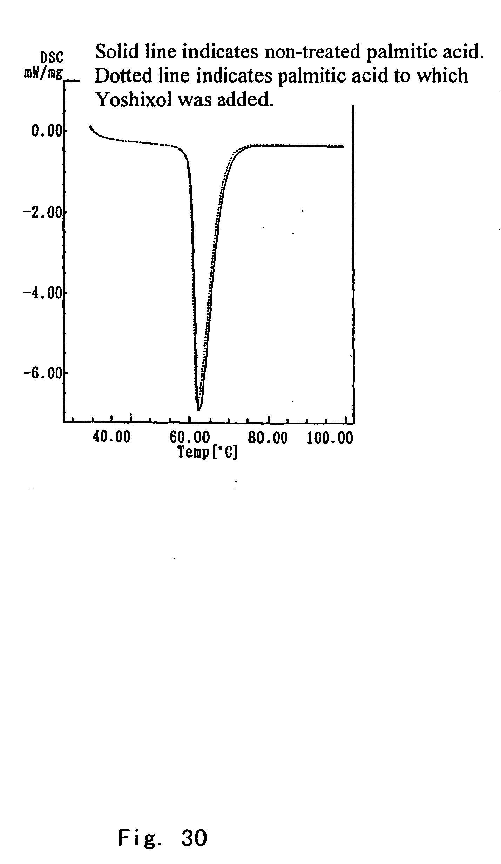

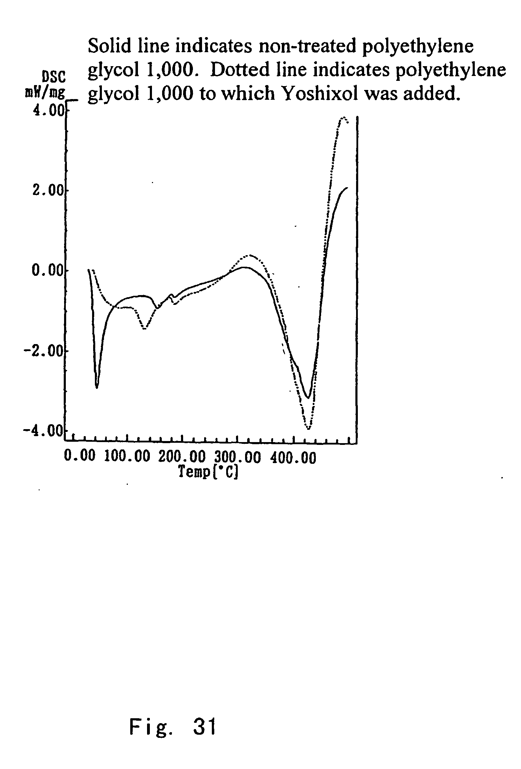

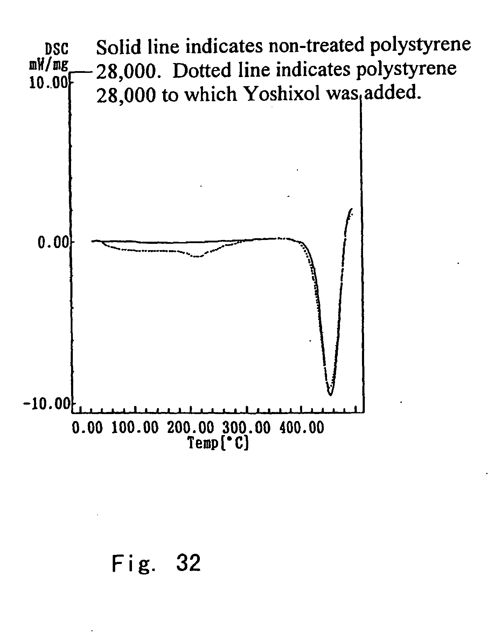

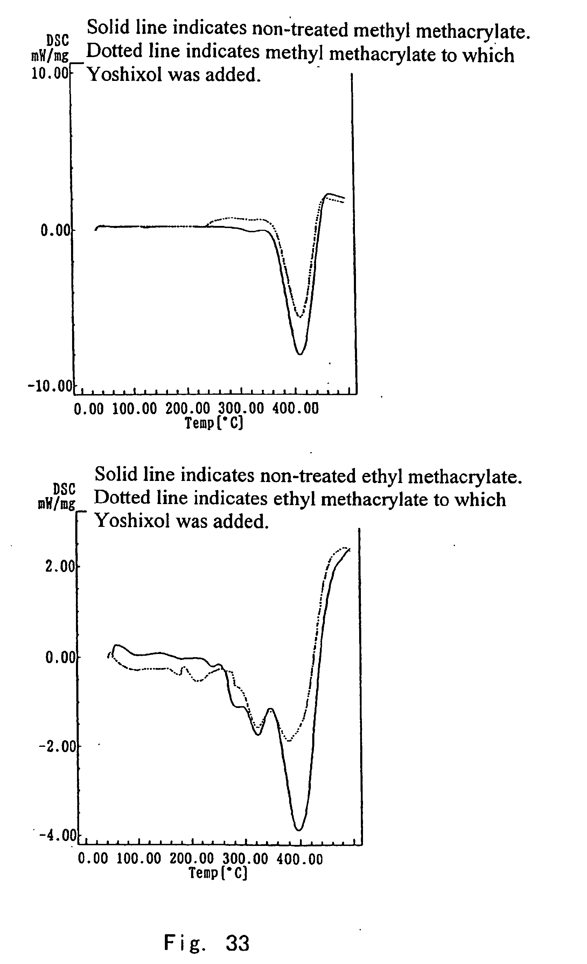

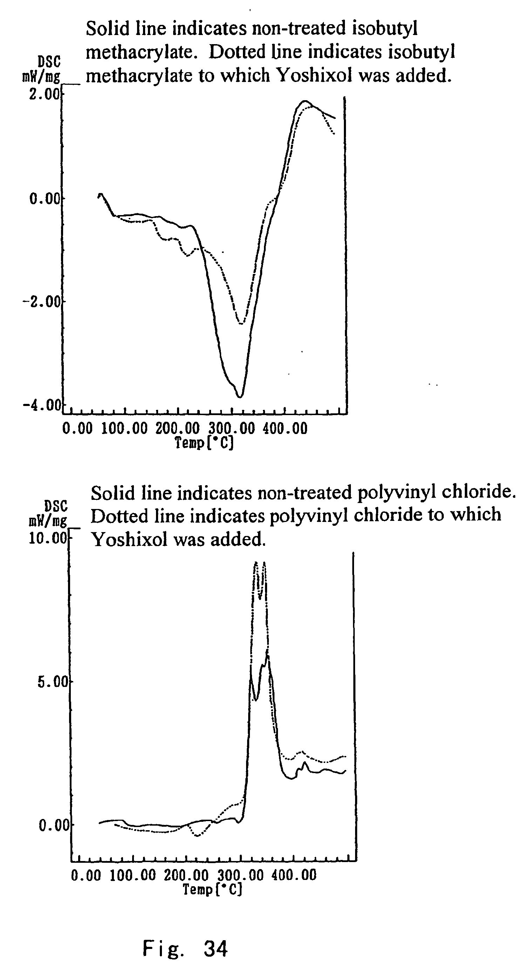

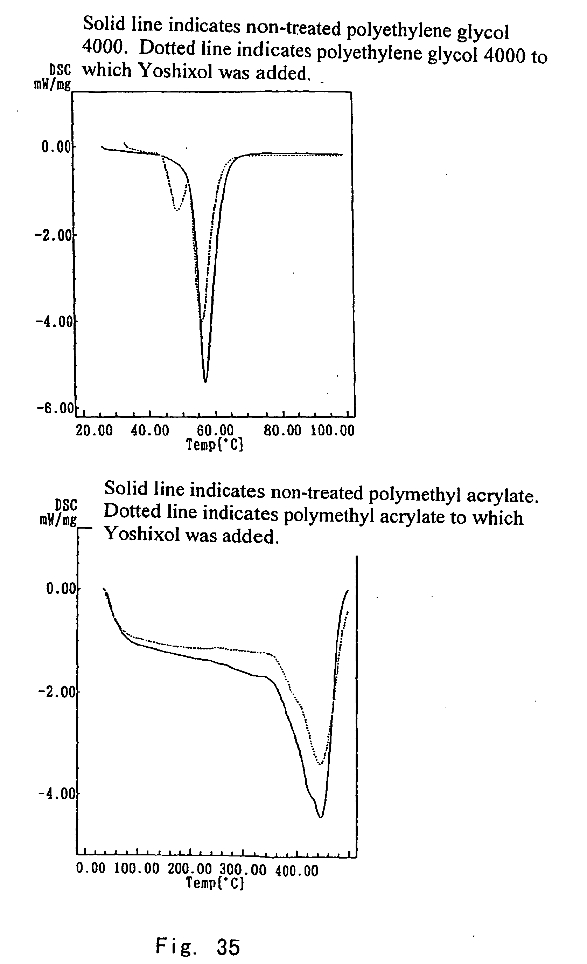

Moreover, since the above-mentioned investigations were mainly carried out by macroscopic observations, in addition, microscopic observations were performed to investigate changes in physical properties of each macromolecule. Here, changes in physical properties of macromolecules were measured by means of a digital scanning calorimeter (DSC-50, Shimadzu Seisakusho Ltd.). Data from the digital scanning calorimeter are displayed as a curve which is converted a temperature deviation signal (ΔT) in comparison with a standard substance (at present measurement, alumina was used) against time or a sample temperature so that the area of the part enclosed between a base line and a peak is proportional to a thermal energy required to fuse the sample. Since the thermal energy supplied to the sample at a constant pressure is identical with a increased amount of enthalpy in the sample, decrement effect of sample enthalpy (decrement of heat capacity at constant volume and condensation rate) is displayed as a peak decreasing when phenomena such as change of the enthalpy against temperature in case of a primary phase transition of a crystal and a fusion of sample will occure. Therefore, peak area is regarded as a saltation quantity of enthalpy. Any change in a heat capacity measured by digital scanning calorimeter could not be observed in stearic acid and lauric acid, both, before and after treatment. Though a slight fall of melting point was observed in myristic acid and palmitic acid (100 µl of Yoshixol per each 100 g, as shown with Figure 30), a large change in heat capacity was not found. On the other hand, a decrease in enthalpy between 20 and 60°C on polyethylene glycol 1000 disappeared after Yoshixol addition so that a phase having thermal capacity between 120 and 160°C changed to a decrement reaction of enthalpy please refer to Figure 31). In polyethylene glycol 4000 treated with Yoshixol, a fall of melting point was observed. When 100 µl of Yoshixol were added to 200 mg of polystyrene 280,000, a phase of a decrement reaction of enthalpy around 30 °C and 280°C appeared (please refer to Figure 32). When 200 µl of Yoshixol were added to 500 mg of methyl methacrylate, a decrement reaction of enthalpy between 230°C and 340°C appeared though melting point did not change. Moreover, when 200 µl of Yoshixol were added to 500 mg of ethyl methacrylate, a decreased and increased reaction of enthalpy was inhibited in total please (refer to Figure 33). Even when 200 µl of Yoshixol were added to 500 mg of isobutyl methacrylate, two peaks of an increased reaction of enthalpy between 60°C and 250°C appeared newly and, an inhibition of maximal increase in enthalpy around 320°C was found. In addition, a phase transition phenomenon was observed in macromolecules of the acrylate group such as lauryl methacrylate and poly (methylacrylate) by treatment with Yoshixol. In 500 mg of poly (vinyl chloride) treated with 200 µl of Yoshixol, the enthalpy around 300°C to 380°C decreased about 2 times in comparison with the control (Please refer to Figure 34). When 100 µl of Yoshixol was added to 100 g of polyethylene glycol 4000, a new peak was found which shows an increase enthalpy between around 44°C and 50°C. When 200 µl of Yoshixol was added to 500 mg of poly (methylacrylate), an increase of enthalpy in a range between 60°C and 360°C was inhibited (as demonstrated with Figure 35).

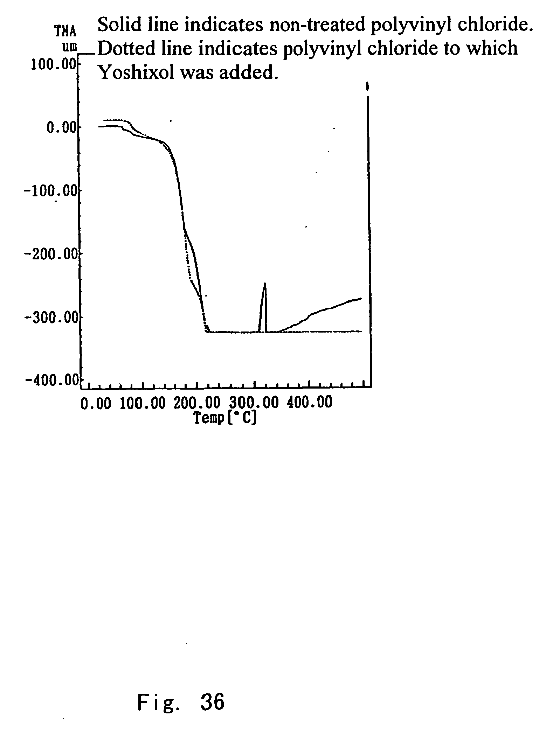

Moreover, when expansibility of poly (vinyl chloride) was investigated by means of a heat machinery analysis equipment (TMA-50, Shimadzu Seisakusho Ltd.), an increase of volume in the control sample was found at a temperature around 315°C, however, an increas of volume in 500 mg of poly (vinyl chloride) treated with 200 µl of Yoshixol could not be found and a decrease in a condensation rate was found (please refer to figure 36). These results are interpreted that Yoshixol has a capacity to improve a physical property of molecules and macromolecules, namely a thermodynamic property (for example, energy storage capacitance and internal energy state, change of structure due to change in entropy).

### Effect of Yoshixol on changes in molecular weight of a new synthesized dimer with seven base pair

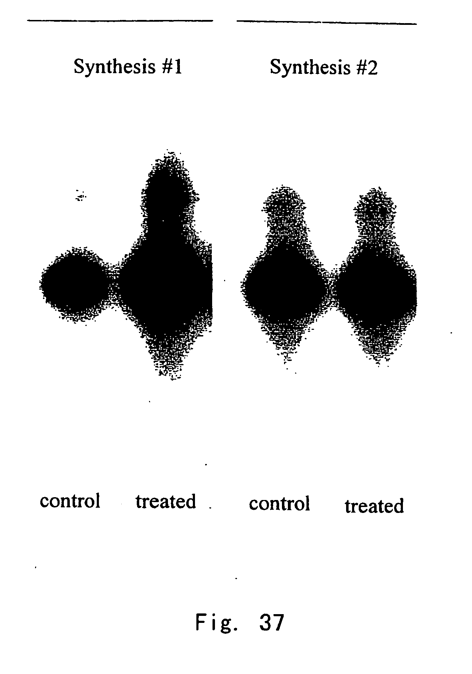

DNA or RNA synthesis device (392-25 type, Perkin-Elmer Co.) was used and 7 base alignment (CTTCGGA) and (CTTCGGG) new synthesis dimer (5'>CTTCGGACTTCGGA<3') and (5'>CTTCGGGCTTCGGG<3') were synthesized. Then, an effect of Yoshixol on a change in molecular weight of the dimer was investigated. This pellet was dissolved on 50 µl of tris EDTA, and OD260 was measurement by 100 times attenuation so that equality of concentration was reached (5 ng/µl) by tris EDTA and distilled water. Further, 4 µl of an adjusted synthesis dimer was labeled at 5'-terminal end of the dimer with 4 µl of ATP which was labeled by P32. In addition, after processing the dimer with 1 µl of polynucleokinase (TaKaRa, Tokyo) for 30 minutes by 37°C, the solution was heated at 70 °C for 5 minutes. Afterward, 65 µl of tris EDTA, 1 µl of glycogen and 190 µl of chilled ethanol were added and were mixed. Further, the mixture was centrifuged over 10 minutes by 16,000 cpm. After removing the supernant, the pellet was dried. Again, this pellet was dissolved in 50 µl of tris EDTA and, 1 µl of the radioactivity comprising solution was mixed with urea (15 g), acrylamide (5.7 g), bisacrylamide (0.3 g), tris boric acid EDTA (3 ml), 10% ammoniumpersulfate (0.1 ml), N, N, N, N-tetramethyldiamine (15 µl) and distilled water, to reach a total volume of 30 ml. Then, 20% gels were made, and an electrophoresis with constant voltage of 10 watt was performed to be exposed on a film. Changes in molecular weight with the dimers were investigated by the sample which consisted of 2 µl of the solution of final tris EDTA with 2 µl of Yoshixol. As the control sample, 2 µl of distilled water was added to the solution. Then, each test sample was given 6 µl of the stop solution. Change in molecular weight of the synthesized Yoshixol treated dimers did not differ from the untreated sample (please refer to Figure 37). This result is interpreted that Yoshixol does not change a distribution of molecular weights with new synthesized dimers (5'>CTTCGGACTTCGGA<3') and (5'>CTTCGGGCTTCGGG<3') and does not hange at least a primary structure.

### PCR effect of Yoshixol on DNA template of a new synthesis dimer with base pair

Effect of Yoshixol on PCR was investigated by DNA template which was extracted from snake (blue-green snake, captured at Matsumoto city, Nagano). The new synthesized dimer of (5'>CTTCGGGCTTCGGG<3') having 7 base-pair alignment (CTTCGGG) as mentioned above was used as a primer. PCR reaction was effected by use of DNA thermal cycler (PJ-2000) made in PERKIN ELMER CETUS company. The following six combinations were prepared for the test samples. Two kinds of samples which consist of 5 µl of primer (100 pico mole/µl) with 5 µl of Yoshixol (P+) and without Yoshixol (P-). Two kinds of samples which consist of 5 µl of snake DNA (500 ng/µl) with 5 µl of Yoshixol (D+) and without Yoshixol (D-). Two kinds of samples which consist of 1 µl of polymerase enzyme (Recombinant Taq DNA Polymerase, No. R001A, TaKaRa Shuzo Co., Otsu city: 5 unit/pl) with 1 µl of Yoshixol (Pm+) and without Yoshixol (Pm-). After each sample was placed for 10 minutes at room temperature, each sample was diluted in distilled water. Further, each sample was adjusted in a primer solution of 10 picomole/µl, DNA solution of 50 ng/µl and a polymerase enzymes solution of 0.5 unit/µl. Then, the following combinations were prepared. On PCR, a primer solution of 5 ml which is diluted mentioned above, 5 ml of DNA solution, 5 µl of buffer solution for PCR reaction, 0.25 µl of polymerase enzymes solution and 4 µl of dNTP-mixed solution were added in distilled water to reach a total volume of 50 µl. The following five groups of samples are provided: First, each sample without Yoshixol addition, as the control (P-, D-, Pm-). Second, each sample wherein only a primer has been treated with Yoshixol (P+, D-, Pm-). Third, each sample wherein only DNA has been treated with Yoshixol (P-, D+, Pm-). Fourth each sample wherein only polymerase enzyme has been treated with Yoshixol (P-, D-, Pm+).

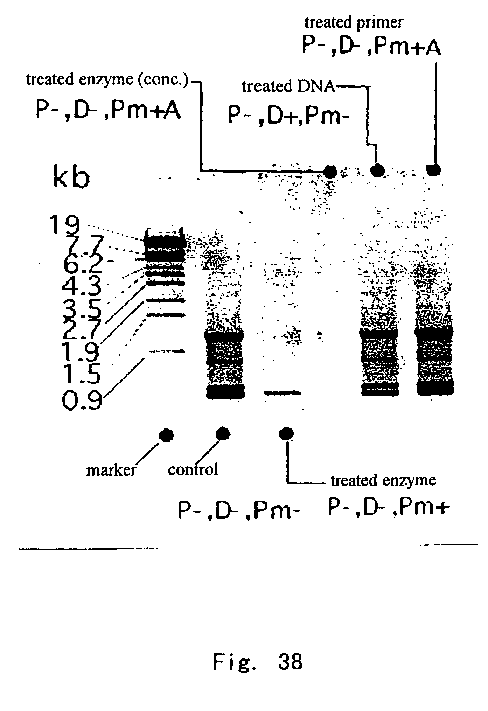

Fifth, each sample wherein the concentration of Yoshixol added to the polymerase is increased to 100 times of P-, D-, Pm+ series mentioned above (P-, D-, Pm+A). In each combination, the amount of cDNA synthesis was amplified by a PCR method and was measured. On the group of non-treated samples P-, D-, Pm- 4 bounds between a molecular weight of 0.5 and 1.2kb were found. Further, the bounds in P-, D-, Pm- did not differ from those bounds found in P+, D-, Pm- and P-, D+, Pm-. But, in the group of P-, D-, Pm+, only one bound at the lowest molecular weight within 4 bounds could be found. In addition, in the group of P-, D-, Pm+A not any kind of bounds could be found (please refer to Figure 38). This result is interpreted that Yoshixol controls or inhibits functional generation of polymerase enzyme which is related to transcription and/or amplification of the base-pair alignment generated by DNA template which consists of many base-pair alignments.

### Effect of Yoshixol on a change and a modulation in absorbance of wavelength of pigment molecules

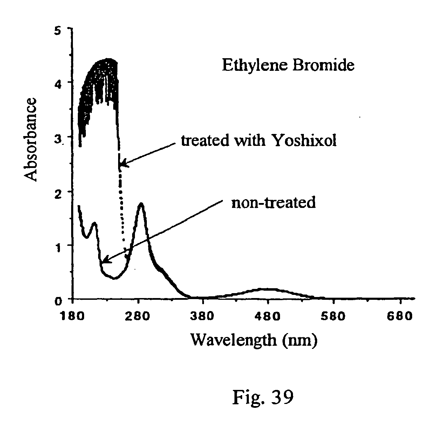

An effect of Yoshixol on a change and a modulation of wavelength of pigment molecules, such as eosin-5-iodoacetamide (Molecular Probes Inc., USA), evans blue (Wakou Jyunyaku Ltd., Osaka) and ethidium bromide (Molecular Probes Inc., USA) was investigated. After addition of Yoshixol (10 µl) to 1 ml of eosin-5-iodoacetamide solution (10 mg/l ml), 1 ml of evans blue solution (1 mol) and 1 ml of ethidium bromide solution (10 mg/ml), the absorbance was measured by means of a spectrophotometer (BIO SPECTRO, Beckman Co. USA). The fundamental wave length band of each pigment did not change with or without Yoshixol addition. Though a quantitative and qualitative change of the fundamental peak wavelength could not be found a wave length band below 260 nm showed a great change. Especially, the color of Yoshixol treated ethidium bromide changed to less transparency from a transparent dark and red color. Such color after the treatment is observed by naked eyes as almost a white and pink color just like a ripe peach. In this case, a major difference was found quantitatively and qualitatively in a wave length band between 270 and 200 nm which was measured by means of a spectrophotometer (please refer to Figure 39). This result is interpreted that Yoshixol can modulate a wave length band of pigmentes.

### Effect of Yoshixol on surface-active agents and/or surfactants

Moreover, effects of Yoshixol (50 µl) on surface-active agents and/or surfactants were investigated; here samples of 20 ml of each detergent of commercially available kitchen use quality (for example, CHERMING, Lion Co.: NATERA, Lion Co.: MOA, Kao Co.), were used mainly consisting of alkyl ether sulfate ester sodium, fatty acid alkanolamido and polyoxyethylene alkyl ether. Further effects of Yoshixol (50 µl) on shampoos were investigated; here samples of 20 ml of each commercially available shampoo for hair washing (for example, PANTEN, Maxfacter Co.: LAX STYLING, Japan Riever Co.: ESSENTIAL STYLING, Kao Co.), were used mainly consisting of lauryl sulphate, paraven, cetyl alcohol, edetic acid, propylene glycol, polyoxyethylene laurylether sulphate. Each Yoshixol treated detergent and shampoo showed a better foaming effect, a better bubbling effect with sensitive texture, a less adhesive property and a more bright colored; and a soil of oiliness could be easily wasted out with a less amount of water. In addition, though a large difference between each Yoshixol treated and non-treated detergent and a shampoo could not be found, the viscosity of each Yoshixol treated detergent and a shampoo decreased in comparison with the non-treated sample when measured at 30°C by means of a viscosimeter (Vismetron VEA-L, Shibaura System Co.) (Please refer to Table 1). This result is interpreted that adding Yoshixol to a commercially available detergent and shampoo may improve the surface active effect and the overall detergent and a shampoo, property.

**Table 1 :**

| Change in Viscosity (CP) of Each Detergent Before and After Treatment with Yoshixol | | | |
|---|---|---|---|
| | MORE | CHARMING | NATERA |
| Before | 66.9 | 62.2 | 70.2 |
| After | 62.9 | 53.3 | 63.5 |

### Effect on each fatty acid of Yoshixol

Each a soapy preparation was prepared by adding 5 g of lauric acid (Wakou Jyunyaku Co., Osaka), 5 g of myristic acid (Wakou Jyunyaku Co., Osaka), 5 g of palmitic acid (Wakou Jyunyaku Co., Osaka), 1 g of linolic acid (Wakou Jyunyaku Co., Osaka), 5 g of stearic acid (Wakou Jyunyaku Co., Osaka) and 1 g of oleic acid (Wakou Jyunyaku Co., Osaka) into 50 ml of sodium hydroxide solution (1 N) (neutralization method). Then, the effect of adding Yoshixol was investigated. A soapy preparation provided with 50 µl of Yoshixol showed a better foaming effect, scarced, lavaged and less adhesive property in comparison with the non-treated preparation. Even when maintained in open state in a room over 6 months, a brown change of color did not occur in comparison with a non-treated preparation. This result is interpreted that Yoshixol is useful as antioxidant for a soap which is made from fatty acids, vegetable oil or animal oil, and that Yoshixol may improve soap properties.

### Concerning acute and chronic toxicity of Yoshixol

A solution obtained by mixing 50 µl/Kg of Yoshixol with 1 ml of 20% glucose solution was administered intravenously in unanesthetized animals (rabbit and dog). After intravenous injection, transitional increase in respiration rate, increase in blood pressure and heart rate and active movements of an extremity (not convulsion) were observed for 1 to 2 minutes after administration. Although an increase in a respiration rate and blood pressure remained during the observation period of 2 hours, an abnormal behavior could not be found. On examination of blood, an hyperchromic anemia at the maximum level occurred 3 days after administration followed by a recovery to the normal state after 1 week. A change in platelets was not observed and, the number of white blood cells increased 3 days after administration followed by a recovery to normal level at 1 week after administration. Though these animals were observed over 1 month, not any abnormal behavior could be found. Also, after they were sucrified by intravenous injection of potassium chloride solution under deep anesthesia after 1 month, not any pathological findings were observed macroscopically and microscopically in the vital organs. This result is interpreted that Yoshixol has a low toxic in vivo effect, and less side effects.

Further, similar experiments have been performed with 4,4-dimethyl-2-cyclohexen-1-one that is the compound according to general formula (3-b) wherein all the substituents R3, R4, R5 and R6 are representing hydrogen. As a result, this substance provided similar results as provided obtained with Yoshixol and mentioned above. In addition, a higher dosage was required to obtain in 9 qualitative manner an identical effect to the above-mentioned effect of Yoshixol. Accordingly, the concentration of 4,4-dimethyl-2-cyclohexen-1-one has to be increased to the about 30 to 100 times dosage of Yoshixol on the biological samples, and to the about 10 to 50 times dosage of Yoshixol with respect to the lower molecular substance and the macromolecular substance of non-living samples.

### EXAMPLES

### Formulation example 1:

The following substances are mixed in order to provide a cream having an antiphlogistic effect: Yoshixol (0.3 ml), citric acid-1-hydrate (0.5 ml), polyethylene pyrene glycol (4.5 ml), distilled water (67.7 ml), cetyl alcohol (4.0 ml), stearic acid (10.0 ml), hard paraffin (2.0 g), myristic acid octlydodecyl (5.0 ml), myristic acid isopropyl (5.0 ml), glycerylmonoolate (0.5 ml). Thereafter, this mixture is heated to about 80 °C to dissolve the components, and to reach an emulsification to provide a vanishing cream (O/W emulsion).

### Formulation example 2:

Yoshixol is added to a liquid paraffin. Following sufficient mixing, the mixture is added to a plastibase to provide an ointment (ointment with oiliness) which consists of 0.3 weight % of Yoshixol.

## Claims

1. Use of a substituted 4,4-dimethyl-2-cyclohexene-1-one compound represented by the following formula (2) for the manufacture of a preparation to be used in the treatment as an antibacterial agent, an antifungal agent, an antiviral agent, a bactericidal and/or sterilizing agent, an anticancer agent and/or antitumor agent, an anticoagulant and/or antifibrinolytic agent, a thrombolytic agent, a metabolism (lipids, sugar, proteins)- improving agent, a promoting agent for wound healing epithelialization-promoting agent (including hair restoration effect), against arteriosclerosis , against antigen-antibody reaction, and/or to be used in an organ and tissue preservative, an antiseptic and/or preservative, a labeled reagent that has a labeled substance in at least one substituent, which labeled substance indicates a targeted position of generating function of molecule, a reductant, a free radical scavenger, a desulfurization agent, a depolymerization agent, an agent for improving functional and/or physical properties of surfactants, a spermatocidal agent or contraceptive agent for external use, a conformation altering agent of saccharide chains, a phase transition agent or a phase transition-improving agent, an improving agent of microphase separation structure, a plasticizer or plasticity and/or elasticity-improving agent, a copolymerization agent or a copolymerization-improving agent, a polymerization regulator or an improving agent of polymerization adjustment, a stabilizer or a stabilization-improving agent, an antioxidant or an oxidation-preventing agent, an improving agent of crystallized materials and/or amorphous materials, a fluidability-improving agent, a softener or an improving agent of softeners and/or flexibility improving agent, a modulation and/or improving agent fluorescent wavelength and/or excitation wavelength pigments, coating materials, paints or colorants, an improving agent of functional and/or physical properties of low molecular substances, an improving agent of physical properties and functions of macromolecular substances, and/or a physical property-improving agent of macromolecular composite materials and functional macromolecular composite materials; 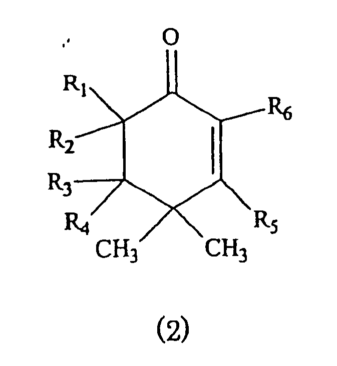 wherein
(i) R1 and R2 both together represent a methylene group and/or R1, R2, R3, R4, R5 and R6 represent independently hydrogen atom; halogen atom; C1-C6 alkyl group; amidino group; C3-C8 cycloalkyl group; C1-C6 alkoxy C1-C6 alkyl group; aryl group; allyl group; aralkyl group in which one or more C1-C6 alkyl groups are bound to an aromatic ring selected from the group consisting of benzene, naphthalene and anthracene ring; C1-C6 alkylene group; benzoyl group; cinnamyl group; cinnamoyl group or furoyl group;
(ii) one or more of R1, R2, R3 and R4, and/or one or more of R5 and R6 may be substituted or non-substituted cyclopentyl group; substituted or non-substituted cyclohexyl group; or substituted or non-substituted naphthyl group;
(iii) R5 and R6 may form a ring by binding with another condensation polycyclic hydrocarbon compound or heterocyclic compound;
(iv) one or more of R3, R4, R5 and R6 may be substituted by one or more of substituents selected from the group consisting of halogen atom, cyano group, protected or non-protected carboxyl group, protected or non-protected hydroxyl group, protected or non-protected amino group, C1-C6 alkyl group, C1-C6 alkoxy group, C1-C7 alkoxy carbonyl group, aryl group, C3-C6 cycloalkyl group, C1-C6 acylamion group, C1-C6 acyloxy group, C2-C6 alkenyl group, C1-C6 trihalogenoalkyl group, C1-C6 alkylamino group, and C1-C6 dialkylamino group;
(v) R2 and/or R5 may be substituted by one or more substituents selected from the group consisting of halogen atom, C1-C6 alkyl group, protected or non-protected carboxyl group, protected or non-protected hydroxyl group, protected or non-protected amino group, protected or non-protected C1-C6 alkylamino group, protected or non-protected C1-C6 aminoalkyl group, protected or non-protected C1-C6 alkylamino C1-C6 alkyl group, protected or non-protected hydroxyalkyl group, and C3-C6 cycloalkylamino group;
wherein
(vi) when one or more of R3, R4, R5 and R6 are alkyl groups, terminal end(s) of the alkyl group(s) may be substituted by C3-C8 cycloalkyl group);
said aryl group in (i) and (iv) is phenyl, tolyl, xylyl or naphthyl group; said substituted cyclopentyl group in (ii) is cyclopentylamino group or cyclopentylcarbinol gropu; said substituted cyclohexyl group in (ii) is cyclohexylamino group, cyclohexylaldehyde group or cyclohexyl acetic acid group; and said substituted naphthyl group in (ii) is naphthylamion group or naphthylamion sulfonic acid group; and
said condensation polycyclic hydrocarbon compound in (iii) is pentalene, indene, naphthalene, azulene, heptalene, biphenylene, indacene, acenaphthylene, fluorene, phenalene, phenanthrene, anthracene, pentacene, hexacene, dibenzophenanthrene, 1H-cyclopentacyclooctene or benzocyclooctene, and the heterocyclic compound may be furan, thiophene, pyrrole, Y-pyran, Y-thiopyran, pyridine, thiazole, imidazole pyrimidine, indole or quinoline.

2. Use of a substituted 4,4-dimethyl-2-cyclohexene-1-one compound represented by the following formula (3-a) for the manufacture of a preparation to be used in the treatment as an antibacterial agent, an antifungal agent, an antiviral agent, a bactericidal and/or sterilizing agent, an anticancer agent and/or antitumor agent, an anticoagulant and/or antifibrinolytic agent, a thrombolytic agent, a metabolism (lipids, sugar; proteins)- improving agent, a promoting agent for wound healing epithelialization-promoting agent (including hair restoration effect), against arteriosclerosis , against antigen-antibody reaction, and/or to be used in an organ and tissue preservative, an antiseptic and/or preservative, a labeled reagent that has a labeled substance in at least one substituent, which labeled substance indicates a targeted position of generating function of molecule, a reductant, a free radical scavenger, a desulfurization agent, a depolymerization agent, an agent for improving functional and/or physical properties of surfactants, a spermatocidal agent or contraceptive agent for external use; a conformation altering agent of saccharide chains, a phase transition agent or a phase transition-improving agent, an improving agent of microphase separation structure, a plasticizer or plasticity and/or elasticity-improving agent, a copolymerization agent or a copolymerization-improving agent, a polymerization regulator or an improving agent of polymerization adjustment, a stabilizer or a stabilization-improving agent, an antioxidant or an oxidation-preventing agent, an improving agent of crystallized materials and/or amorphous materials, a fluidability improving agent, a softener or an improving agent of softeners and/or flexibility improving agent, a modulation and/or improving agent fluorescent wavelength and/or excitation wavelength pigments, coating materials, paints or colorants, an improving agent of functional and/or physical properties of low molecular substances, an improving agent of physical properties and functions of macromolecular substances, and/or a physical property-improving agent of macromolecular composite materials and functional macromolecular composite materials. 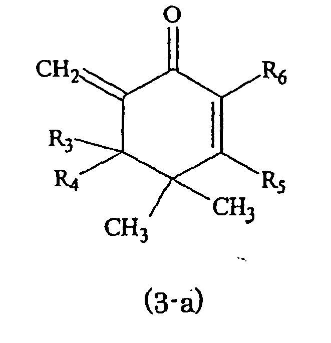 wherein
(i) R3, R4, R5 and R6 represent independently hydrogen atom; halogen atom; C1-C6 alkyl group; amidino group; C3-C8 cycloalkyl group; C1-C6 alkoxy C1-C6 alkyl group; aryl group; allyl group; aralkyl group in which one or more C1-C6 alkyl groups are bound to an aromatic ring selected from the group consisting of benzene, naphthalene and anthracene ring; C1-C6 alkylene group; benzoyl group; cinnamyl group; cinnamoyl group or furoyl group;
(ii) one or more of R3 and R4, and/or one or more of R5 and R6 may be substituted or non-substituted cyclopentyl group; substituted or non-substituted cyclohexyl group; or substituted or non-substituted naphthyl group;
(iii) R5 and R6 may form a ring by binding with another condensation polycyclic hydrocarbon compound or heterocyclic compound;
(iv) one or more of R3, R4, R5 and R6 may be substituted by one or more of substituents selected from the group consisting of halogen atom, cyano group, protected or non-protected carboxyl group, protected or non-protected hydroxyl group, protected or non-protected amino group, C1-C6 alkyl group, C1-C6 alkoxy group, C1-C7 alkoxy carbonyl group, aryl group, C3-C6 cycloalkyl group, C1-C6 acylamion group, C1-C6 acyloxy group, C2-C6 alkenyl group, C1-C6 trihalogenoalkyl group, C1-C6 alkylamino group, and C1-C6 dialkylamino group;
(v) R5 may be substituted by one or more substituents selected from the group consisting of halogen atom, C1-C6 alkyl group, protected or non-protected carboxyl group, protected or non-protected hydroxyl group, protected or non-protected amino group, protected or non-protected C1-C6 alkylamino group, protected or non-protected C1-C6 aminoalkyl group, protected or non-protected C1-C6 alkylamino C1-C6 alkyl group, protected or non-protected hydroxyalkyl group, and C3-C6 cycloalkylamino group;
wherein
(vi) when one or more of R3, R4, R5 and R6 are alkyl groups, terminal end(s) of the alkyl group(s) may be substituted by C3-C8 cycloalkyl group);
said aryl group in (i) and (iv) is phenyl, tolyl, xylyl or naphthyl group; said substituted cyclopentyl group in (ii) is cyclopentylamino group or cyclopentylcarbinol gropu; said substituted cyclohexyl group in (ii) is cyclohexylamino group, cyclohexylaldehyde group or cyclohexyl acetic acid group; and said substituted naphthyl group in (ii) is naphthylamion group or naphthylamion sulfonic acid group; and
said condensation polycyclic hydrocarbon compound in (iii) is pentalene, indene, naphthalene, azulene, heptalene, biphenylene, indacene, acenaphthylene, fluorene, phenalene, phenanthrene, anthracene, pentacene, hexacene, dibenzophenanthrene, 1H-cyclopentacyclooctene or benzocyclooctene, and the heterocyclic compound may be furan, thiophene, pyrrole, γ-pyran, γ-thiopyran, pyridine, thiazole, imidazole pyrimidine, indole or quinoline.

3. Use of a substituted 4,4-dimethyl-2-cyclohexene-1-one compound represented by the following formula (3-a-1) for the manufacture of a preparation to be used in the treatment as an antibacterial agent, an antifungal agent, an antiviral agent, a bactericidal and/or sterilizing agent, an anticancer agent and/or antitumor agent, an anticoagulant and/or antifibrinolytic agent, a thrombolytic agent, a metabolism (lipids, sugar, proteins)- improving agent, a promoting agent for wound healing epithelialization-promoting agent (including hair restoration effect), against arteriosclerosis , against antigen-antibody reaction, and/or to be used in an organ and tissue preservative, an antiseptic and/or preservative, a labeled reagent that has a labeled substance in at least one substituent, which labeled substance indicates a targeted position of generating function of molecule, a reductant, a free radical scavenger, a desulfurization agent, a depolymerization agent, an agent for improving functional and/or physical properties of surfactants, a spermatocidal agent or contraceptive agent for external use, a conformation altering agent of saccharide chains, a phase transition agent or a phase transition-improving agent, an improving agent of microphase separation structure, a plasticizer or plasticity and/or elasticity-improving agent, a copolymerization agent or a copolymerization-improving agent, a polymerization regulator or an improving agent of polymerization adjustment, a stabilizer or a stabilization-improving agent, an antioxidant or an oxidation-preventing agent, an improving agent of crystallized materials and/or amorphous materials, a fluidability-improving agent, a softener or an improving agent of softeners and/or flexibility improving agent, a modulation and/or improving agent fluorescent wavelength and/or excitation wavelength pigments, coating materials, paints or colorants, an improving agent of functional and/or physical properties of low molecular substances, an improving agent of physical properties and functions of macromolecular substances, and/or a physical property-improving agent of macromolecular composite materials and functional macromolecular composite materials.

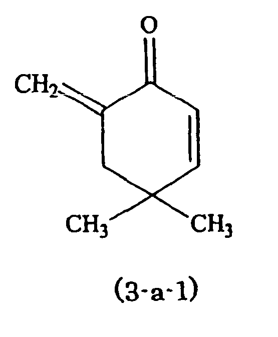

4. Use of a substituted 4,4-dimethyl-2-cyclohexene-1-one compound represented by the following formula (3-b) for the manufacture of a preparation to be used in the treatment as an antibacterial agent, an antifungal agent, an antiviral agent, a bactericidal and/or sterilizing agent, an anticancer agent and/or antitumor agent, an anticoagulant and/or antifibrinolytic agent, a thrombolytic agent, a metabolism (lipids, sugar, proteins)- improving agent, a promoting agent for wound healing epithelialization-promoting agent (including hair restoration effect), against arteriosclerosis , against antigen-antibody reaction, and/or to be used in an organ and tissue preservative, an antiseptic and/or preservative, a labeled reagent that has a labeled substance in at least one substituent, which labeled substance indicates a targeted position of generating function of molecule, a reductant, a free radical scavenger, a desulfurization agent, a depolymerization agent, an agent for improving functional and/or physical properties of surfactants, a spermatocidal agent or contraceptive agent for external use, a conformation altering agent of saccharide chains, a phase transition agent or a phase transition-improving agent, an improving agent of microphase separation structure, a plasticizer or plasticity and/or elasticity-improving agent, a copolymerization agent or a copolymerization-improving agent, a polymerization regulator or an improving agent of polymerization adjustment, a stabilizer or a stabilization-improving agent, an antioxidant or an oxidation-preventing agent, an improving agent of crystallized materials and/or amorphous materials, a fluidability-improving agent, a softener or an improving agent of softeners and/or flexibility improving agent, a modulation and/or improving agent fluorescent wavelength and/or excitation wavelength pigments, coating materials, paints or colorants, an improving agent of functional and/or physical properties of low molecular substances, an improving agent of physical properties and functions of macromolecular substances, and/or a physical property-improving agent of macromolecular composite materials and functional macromolecular composite materials: 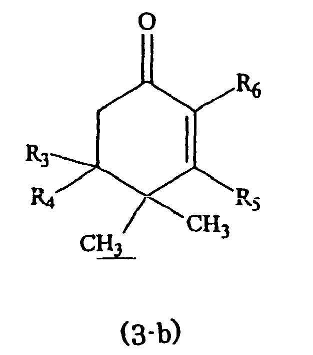 wherein
(i) R3, R4, R5 and R6 represent independently hydrogen atom; halogen atom; C1-C6 alkyl group; amidino group; C3-C8 cycloalkyl group; C1-C6 alkoxy C1-C6 alkyl group; aryl group; allyl group; aralkyl group in which one or more C1-C6 alkyl groups are bound to an aromatic ring selected from the group consisting of benzene, naphthalene and anthracene ring; C1-C6 alkylene group; benzoyl group; cinnamyl group; cinnamoyl group or furoyl group;
(ii) one or more of R3 and R4, and/or one or more of R5 and R6 may be substituted or non-substituted cyclopentyl group; substituted or non-substituted cyclohexyl group; or substituted or non-substituted naphthyl group;
(iii) R5 and R6 may form a ring by binding with another condensation polycyclic hydrocarbon compound or heterocyclic compound;
(iv) one or more of R3, R4, R5 and R6 may be substituted by one or more of substituents selected from the group consisting of halogen atom, cyano group, protected or non-protected carboxyl group, protected or non-protected hydroxyl group, protected or non-protected amino group, C1-C6 alkyl group, C1-C6 alkoxy group, C1-C7 alkoxy carbonyl group, aryl group, C3-C6 cycloalkyl group, C1-C6 acylamion group, C1-C6 acyloxy group, C2-C6 alkenyl group, C1-C6 trihalogenoalkyl group, C1-C6 alkylamino group, and C1-C6 dialkylamino group;
(v) R5 may be substituted by one or more substituents selected from the group consisting of halogen atom, C1-C6 alkyl group, protected or non-protected carboxyl group, protected or non-protected hydroxyl group, protected or non-protected amino group, protected or non-protected C1-C6 alkylamino group, protected or non-protected C1-C6 aminoalkyl group, protected or non-protected C1-C6 alkylamino C1-C6 alkyl group, protected or non-protected hydroxyalkyl group, and C3-C6 cycloalkylamino group;
wherein
(vi) when one or more of R3, R4, R5 and R6 are alkyl groups, terminal end(s) of the alkyl group(s) may be substituted by C3-C8 cycloalkyl group);
said aryl group in (i) and (iv) is phenyl, tolyl, xylyl or naphthyl group; said substituted cyclopentyl group in (ii) is cyclopentylamino group or cyclopentylcarbinol gropu; said substituted cyclohexyl group in (ii) is cyclohexylamino group, cyclohexylaldehyde group or cyclohexyl acetic acid group; and said substituted naphthyl group in (ii) is naphthylamion group or naphthylamion sulfonic acid group; and
said condensation polycyclic hydrocarbon compound in (iii) is pentalene, indene, naphthalene, azulene, heptalene, biphenylene, indacene, acenaphthylene, fluorene, phenalene, phenanthrene, anthracene, pentacene, hexacene, dibenzophenanthrene, 1H-cyclopentacyclooctene or benzocyclooctene, and the heterocyclic compound may be furan, thiophene, pyrrole, Y-pyran, Y-thiopyran, pyridine, thiazole, imidazole pyrimidine, indole or quinoline.

## Patentansprüche

1. Verwendung
einer, der nachstehenden allgemeinen Formel (2) entsprechenden substituierten 4,4-Dimethyl-2-cyclohexen-1-on-Verbindung zur Herstellung eines Präparates/Mittels,
das bei einer Behandlung benutzt wird als:
- ein antibakterielles Mittel;
- ein, gegen Pilzbefall wirksames Mittel;
- ein, gegen ein Virus wirkendes Mittel;
- ein bakterizides und/oder sterilisierendes Mittel;
- ein, gegen Krebs und/oder gegen Tumor(en) wirksames Mittel;
- ein gerinnungshemmendes und/oder die Fibrinbildung hemmendes Mittel;
- ein, einen Thrombuszerfall bewirkendes Mittel;
- ein, den (Fett-, Zucker-, Protein-)Stoffwechsel verbesserndes Mittel;
- ein, die Wundheilung und Epithelbildung (einschließlich des Haarwachstum wiederherstellendes) förderndes Mittel;
- ein Mittel gegen Arteriosklerose, ein, die Antigen-Antikörper-Bildungsreaktion beeinflussendes Mittel und/oder ein, bei Transplantationen das Organ und das Gewebe schützendes Mittel;
- ein Antiseptikum und/oder Schutzmittel;
- ein Markierungsmittel, das an wenigstens einer Substiuentenstelle mit einer (Isotopen-)Markierungssubstanz versehen ist, die eine Zielposition für ein, diese Zielposition suchendes Molekül markiert;
- ein Reduktionsmittel;
- ein, freie Radikale unschädlich machendes Mittel;
- ein Entschwefelungsmittel;
- ein Depolymerisierungsmittel;
- ein Mittel zur Verbesserung funktionaler und/oder physikalischer Eigenschaften von oberflächenaktiven Stoffen bzw. Tensiden;
- ein spermazides Mittel und/oder empfängnisverhütendes Mittel, je zur äußerlichen Anwendung;
- ein, die Konformation von Saccharid-Ketten veränderndes Mittel;
- ein Phasenumwandlungsmittel oder ein, einen Phasenübergang oder eine Phasentransformation verbesserndes Mittel;
- ein Mittel zur Verbesserung der Trennung von Mikrophasenstrukturen;
- ein Weichmacher oder ein, die plastischen und/oder elastischen Eigenschaften verbesserndes Mittel;
- ein Copolymerisationsmittel oder ein, die Copolymerisation verbesserndes Mittel;
- ein Polymerisationsregulierungsmittel oder ein Mittel zur Verbesserung der Polymerisationsgradeinstellung;
- ein Stabilisierungsmittel oder ein, die Stabilisierung verbesserndes Mittel;
- ein Antioxidantium bzw. Oxidationsschutzmittel oder ein Oxidationsvorgänge verhinderndes Mittel;
- ein Verbesserungsmittel für kristalline und/oder amorphe Materialien;
- ein, das Fließverhalten bzw. die Fluidität verbesserndes Mittel;
- ein Erweichungsmittel, Enthärtungsmittel oder Weichspüler, oder ein Verbesserungsmittel für Erweichungsmittel, Enthärtungsmittel und Weichspüler und/oder ein, die Biegsamkeit, Dehnbarkeit, Anpassungsfähigkeit und Elastizität verbesserndes Mittel;
- ein Modulationssteuermittel und/oder ein, die Fluoreszenzstrahlungswellenlänge und/oder die Erregungsstrahlungswellenlänge von Pigmenten, von Anstrich- und Beschichtungsmitteln, von Farben, von Anstrichstoffen und von Färbemitteln verbesserndes Mittel;
- ein, die funktionalen und/oder physikalischen Eigenschaften von niedermolekularen Substanzen verbesserndes Mittel;
- ein, die physikalischen Eigenschaften und Funktionen von makromolekularen Substanzen verbesserndes Mittel; und/oder
- ein, die physikalischen Eigenschaften verbesserndes Mittel für makromolekulare Verbundwerkstoffe, Kompositmaterialien und für funktionale makromolekulare Verbundwerkstoffe und Kompositmaterialien 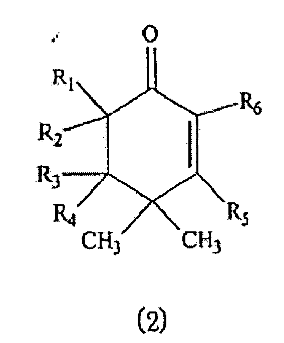
wobei:
(I) Beide, R1 und R2 zusammen stehen für eine Methylengruppe; und/oder R1, R2, R3, R4, R5 und R6 stehen - unabhängig voneinander - für ein Wasserstoffatom, für ein Halogenatom, für eine C₁₋₆-Alkylgruppe, für eine Amidinogruppe, für eine C₃₋₈-Cycloalkylgruppe, für eine C₁₋₆₋Alkoxy-C₁₋₆-alkylgruppe, für eine Arylgruppe, für eine Allylgruppe, für eine Aralkylgruppe, in der eine oder mehrere C₁₋₆-Alkylgruppe(n) an einen aromatischen Ring gebunden sind, der seinerseits ausgewählt ist aus einem Benzol-, Naphthalin- oder Anthracen-Ring, oder für eine C₁₋₆₋Alkylengruppe, für eine Benzoylgruppe, für eine Cinnamylgruppe, für eine Cinnamoylgruppe oder für eine Furoylgruppe;
(II) ein oder mehrere Substituent(en) R1, R2, R3 und R4 und/oder ein oder mehrere Substituent(en) R5 und R6 können eine substituierte oder nicht-substituierte Cyclopentylgruppe, eine substituierte oder nicht-substituierte Cyclohexylgruppen oder eine substituierte oder nicht substituierte Naphthylgruppen sein;
(III) R5 und R6 können durch Verknüpfung mit einer anderen kondensierten polycyclischen Kohlenwasserstoffverbindung oder mit einer heterocyclischen Verbindung einen Ring bilden;
(IV) ein oder mehrere Substiuent(en) R3, R4, R5 und R6 können mit einem oder mehreren Substituent(en) substituiert sein, der/die ihrerseits ausgewählt sind aus einer Gruppe, die umfasst: ein Halogenatom, eine Cyanogruppe, eine geschützte oder ungeschützte Carboxylgruppe, eine geschützte oder ungeschützte Hydroxylgruppe, eine geschützte oder ungeschützte Aminogruppe, eine C₁₋₆-Alkylgruppe, eine C₁₋₆₋Alkoxygruppe, eine C₁₋₇Alkoxy-carbonylgruppe, eine Arylgruppe, eine C₃₋₆-Cycloalkylgruppe, eine C₁₋₆-Acylaminogruppe, eine C₁₋₆₋Acyloxygruppe, eine C₂₋₆-Alkenylgruppe, eine C₁₋₆-Trihalogen-alkylgruppe, eine C₁₋₆-Alkylaminogruppe und eine C₁₋₆₋Dialkylaminogruppe;
(V) R2 und/oder R5 können substituiert sein mit einem oder mehreren Substituent(en), der/die ihrerseits ausgewählt sind aus einer Gruppe, die umfasst: ein Halogenatom, eine C₁₋₆-Alkylgruppe, eine geschützte oder ungeschützte Carboxylgruppe, eine geschützte oder ungeschützte Hydroxylgruppe, eine geschützte oder ungeschützte Aminogruppe, eine geschützte oder ungeschützte C₁₋₆-Alkylaminogruppe, eine geschützte oder ungeschützte C₁₋₆-Aminoalkylgruppe, eine geschützte oder ungeschützte C₁₋₆-Alkylamino-C₁₋₆-Alkylgruppe, eine geschützte oder ungeschützte Hydroxyalkylgruppe und eine C₃₋₆-Cycloalkylaminogruppe;
(VI) weiterhin mit den folgenden Maßgaben:
sofern es sich bei einem oder mehreren Substituent(en) R3, R4, R5 und R6 um Alkylgruppen handelt, können das/die endständige(n) Ende(n) der Alkylgruppe(n) ihrerseits mit C₃₋₈-Cycloalkylgruppen substituiert sein;
die in den vorstehenden Absätzen (i) und (iv) genannte Arylgruppe ist eine Phenyl-, Tolyl-, Xylyl- oder Napthyl-gruppe;
die im vorstehenden Absatz (ii) genannte substituierte Cyclopentylgruppe ist eine Cyclopentylaminogruppe oder eine Cyclopentylcarbinolgruppe;
die im vorstehenden Absatz (ii) genannte substituierte Cyclohexylgruppe ist eine Cyclohexylaminogruppe, eine Cyclohexylaldehydgruppe oder eine Cyclohexyl-essigsäure-gruppe;
die im vorstehenden Absatz (ii) genannte substiuierte Napthylgruppe ist eine Naphthylaminogruppe oder eine Napthylamino-sulfonsäuregruppe;
die im vorstehenden Absatz (iii) genannte kondensierte polycyclische Kohlenwasserstoffverbindung ist Pentalen, Inden, Naphthalin, Azulen, Heptalen, Biphenylen, Indacen, Acenapthylen, Fluoren, Phenalen, Phenanthren, Anthracen, Pentacen, Hexacen, Dibenzophenanthren, 1-H-Cyclopentacycloocten oder Benzocycloocten; und
die im vorstehenden Absatz (iii) genannte heterocyclische Verbindung kann Furan, Thiophen, Pyrrol, γ-Pyran, γ-Thiopyran, Pyridin, Thiazol, Imidazol, Pyrimidin, Indol oder Chinolin sein.

2. Verwendung
einer, der nachstehenden allgemeinen Formel (3-a) entsprechenden
substituierten 4,4-Dimethyl-2-cyclohexen-1-on-Verbindung zur Herstellung eines Präparates/Mittels,
das bei einer Behandlung benutzt wird als:
- ein antibakterielles Mittel;
- ein, gegen Pilzbefall wirksames Mittel;
- ein, gegen ein Virus wirkendes Mittel;
- ein bakterizides und/oder sterilisierendes Mittel;
- ein, gegen Krebs und/oder gegen Tumor(en) wirksames Mittel;
- ein gerinnungshemmendes und/oder die Fibrinbildung hemmendes Mittel;
- ein, einen Thrombuszerfall bewirkendes Mittel;
- ein, den (Fett-, Zucker-, Protein-)Stoffwechsel verbesserndes Mittel;
- ein, die Wundheilung und Epithelbildung (einschließlich des Haarwachstum wiederherstellendes) förderndes Mittel;
- ein Mittel gegen Arteriosklerose, ein, die Antigen-Antikörper-Bildungsreaktion beeinflussendes Mittel und/oder ein, bei Transplantationen das Organ und das Gewebe schützendes Mittel;
- ein Antiseptikum und/oder Schutzmittel;
- ein Markierungsmittel, das an wenigstens einer Substiuentenstelle mit einer (Isotopen-)Markierungssubstanz versehen ist, die eine Zielposition für ein, diese Zielposition suchendes Molekül markiert;
- ein Reduktionsmittel;
- ein, freie Radikale unschädlich machendes Mittel;
- ein Entschwefelungsmittel;
- ein Depolymerisierungsmittel;
- ein Mittel zur Verbesserung funktionaler und/oder physikalischer Eigenschaften von oberflächenaktiven Stoffen bzw. Tensiden;
- ein spermazides Mittel und/oder empfängnisverhütendes Mittel, je zur äußerlichen Anwendung;
- ein, die Konformation von Saccharid-Ketten veränderndes Mittel;
- ein Phasenumwandlungsmittel oder ein, einen Phasenübergang oder eine Phasentransformation verbesserndes Mittel;
- ein Mittel zur Verbesserung der Trennung von Mikrophasenstrukturen;
- ein Weichmacher oder ein, die plastischen und/oder elastischen Eigenschaften verbesserndes Mittel;
- ein Copolymerisationsmittel oder ein, die Copolymerisation verbesserndes Mittel;
- ein Polymerisationsregulierungsmittel oder ein Mittel zur Verbesserung der Polymerisationsgradeinstellung;
- ein Stabilisierungsmittel oder ein, die Stabilisierung verbesserndes Mittel;
- ein Antioxidantium bzw. Oxidationsschutzmittel oder ein Oxidationsvorgänge verhinderndes Mittel;
- ein Verbesserungsmittel für kristalline und/oder amorphe Materialien;
- ein, das Fließverhalten bzw. die Fluidität verbesserndes Mittel;
- ein Erweichungsmittel, Enthärtungsmittel oder Weichspüler, oder ein Verbesserungsmittel für Erweichungsmittel, Enthärtungsmittel und Weichspüler und/oder ein, die Biegsamkeit, Dehnbarkeit, Anpassungsfähigkeit und Elastizität verbesserndes Mittel;
- ein Modulationssteuermittel und/oder ein, die Fluoreszenzstrahlungswellenlänge und/oder die Erregungsstrahlungswellenlänge von Pigmenten, von Anstrich- und Beschichtungsmitteln, von Farben, von Anstrichstoffen und von Färbemitteln verbesserndes Mittel;
- ein, die funktionalen und/oder physikalischen Eigenschaften von niedermolekularen Substanzen verbesserndes Mittel;
- ein, die physikalischen Eigenschaften und Funktionen von makromolekularen Substanzen verbesserndes Mittel; und/oder
- ein, die physikalischen Eigenschaften verbesserndes Mittel für makromolekulare Verbundwerkstoffe, Kompositmaterialien und für funktionale makromolekulare Verbundwerkstoffe und Kompositmaterialien 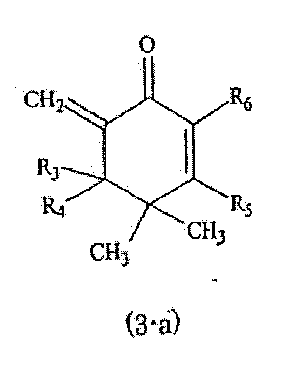
wobei:
(I) R3, R4, R5 und R6 stehen - unabhängig voneinander - für ein Wasserstoffatom, für ein Halogenatom, für eine C₁₋₆-Alkylgruppe, für eine Amidinogruppe, für eine C₃₋₈-Cycloalkylgruppe, für eine C₁₋₆₋Alkoxy-C₁₋₆-alkylgruppe, für eine Arylgruppe, für eine Allylgruppe, für eine Aralkylgruppe, in der eine oder mehrere C₁₋₆-Alkylgruppe(n) an einen aromatischen Ring gebunden sind, der seinerseits ausgewählt ist aus einem Benzol-, Naphthalin- oder Anthracen-Ring, oder für eine C₁₋₆₋Alkylengruppe, für eine Benzoylgruppe, für eine Cinnamylgruppe, für eine Cinnamoylgruppe oder für eine Furoylgruppe;
(II) ein oder mehrere Substituent(en) R3 und R4 und/oder ein oder mehrere Substituent(en) R5 und R6 können eine substituierte oder nicht-substituierte Cyclopentylgruppe, eine substituierte oder nicht-substituierte Cyclohexylgruppen oder eine substituierte oder nicht substituierte Naphthylgruppen sein;
(III) R5 und R6 können durch Verknüpfung mit einer anderen kondensierten polycyclischen Kohlenwasserstoffverbindung oder mit einer heterocyclischen Verbindung einen Ring bilden;
(IV) ein oder mehrere Substiuent(en) R3, R4, R5 und R6 können mit einem oder mehreren Substituent(en) substituiert sein, der/die ihrerseits ausgewählt sind aus einer Gruppe, die umfasst: ein Halogenatom, eine Cyanogruppe, eine geschützte oder ungeschützte Carboxylgruppe, eine geschützte oder ungeschützte Hydroxylgruppe, eine geschützte oder ungeschützte Aminogruppe, eine C₁₋₆-Alkylgruppe, eine C₁₋₆₋Alkoxygruppe, eine C₁₋₇-Alkoxy-carbonylgruppe, eine Arylgruppe, eine C₃₋₆-Cycloalkylgruppe, eine C₁₋₆-Acylaminogruppe, eine C₁₋₆₋Acyloxygruppe, eine C₂₋₆-Alkenylgruppe, eine C₁₋₆-Trihalogen-alkylgruppe, eine C₁₋₆-Alkylaminogruppe und eine C₁₋₆₋Dialkylaminogruppe;
(V) R5 kann substituiert sein mit einem oder mehreren Substituent(en), der/die ihrerseits ausgewählt sind aus einer Gruppe, die umfasst: ein Halogenatom, eine C₁₋₆-Alkylgruppe, eine geschützte oder ungeschützte Carboxylgruppe, eine geschützte oder ungeschützte Hydroxylgruppe, eine geschützte oder ungeschützte Aminogruppe, eine geschützte oder ungeschützte C₁₋₆-Alkylaminogruppe, eine geschützte oder ungeschützte C₁₋₆-Aminoalkylgruppe, eine geschützte oder ungeschützte C₁₋₆Alkylamino-C₁₋₆-alkylgruppe, eine geschützte oder ungeschützte Hydroxyalkylgruppe und eine C₃₋₆-Cycloalkylaminogruppe;
(VI) weiterhin mit den folgenden Maßgaben:
sofern es sich bei einem oder mehreren Substituent(en) R3, R4, R5 und R6 um Alkylgruppen handelt, können das/die endständige(n) Ende(n) der Alkylgruppe(n) ihrerseits mit C₃₋₈-Cycloalkylgruppen substituiert sein;
die in den vorstehenden Absätzen (i) und (iv) genannte Arylgruppe ist eine Phenyl-, Tolyl-, Xylyl- oder Napthyl-gruppe;
die im vorstehenden Absatz (ii) genannte substituierte Cyclopentylgruppe ist eine Cyclopentylaminogruppe oder eine Cyclopentylcarbinolgruppe;
die im vorstehenden Absatz (ii) genannte substituierte Cyclohexylgruppe ist eine Cyclohexylaminogruppe, eine Cyclohexylaldehydgruppe oder eine Cyclohexyl-essigsäure-gruppe;
die im vorstehenden Absatz (ii) genannte substiuierte Napthylgruppe ist eine Naphthylaminogruppe oder eine Napthylamino-sulfonsäuregruppe;
die im vorstehenden Absatz (iii) genannte kondensierte polycyclische Kohlenwasserstoffverbindung ist Pentalen, Inden, Naphthalin, Azulen, Heptalen, Biphenylen, Indacen, Acenapthylen, Fluoren, Phenalen, Phenanthren, Anthracen, Pentacen, Hexacen, Dibenzophenanthren, 1-H-Cyclopentacycloocten oder Benzocycloocten; und
die im vorstehenden Absatz (iii) genannte heterocyclische Verbindung kann Furan, Thiophen, Pyrrol, γ-Pyran, γ-Thiopyran, Pyridin, Thiazol, Imidazol, Pyrimidin, Indol oder Chinolin sein.

3. Verwendung
einer, der nachstehenden allgemeinen Formel (3-a-1) entsprechenden
substituierten 4,4-Dimethyl-2-cyclohexen-1-on-Verbindung zur Herstellung eines Präparates/Mittels,
das bei einer Behandlung benutzt wird als:
- ein antibakterielles Mittel;
- ein, gegen Pilzbefall wirksames Mittel;
- ein, gegen ein Virus wirkendes Mittel;
- ein bakterizides und/oder sterilisierendes Mittel;
- ein, gegen Krebs und/oder gegen Tumor(en) wirksames Mittel;
- ein gerinnungshemmendes und/oder die Fibrinbildung hemmendes Mittel;
- ein, einen Thrombuszerfall bewirkendes Mittel;
- ein, den (Fett-, Zucker-, Protein-)Stoffwechsel verbesserndes Mittel;
- ein, die Wundheilung und Epithelbildung (einschließlich des Haarwachstum wiederherstellendes) förderndes Mittel;
- ein Mittel gegen Arteriosklerose, ein, die Antigen-Antikörper-Bildungsreaktion beeinflussendes Mittel und/oder ein, bei Transplantationen das Organ und das Gewebe schützendes Mittel;
- ein Antiseptikum und/oder Schutzmittel;
- ein Markierungsmittel, das an wenigstens einer Substiuentenstelle mit einer (Isotopen-)Markierungssubstanz versehen ist, die eine Zielposition für ein, diese Zielposition suchendes Molekül markiert;
- ein Reduktionsmittel;
- ein, freie Radikale unschädlich machendes Mittel;
- ein Entschwefelungsmittel;
- ein Depolymerisierungsmittel;
- ein Mittel zur Verbesserung funktionaler und/oder physikalischer Eigenschaften von oberflächenaktiven Stoffen bzw. Tensiden;
- ein spermazides Mittel und/oder empfängnisverhütendes Mittel, je zur äußerlichen Anwendung;
- ein, die Konformation von Saccharid-Ketten veränderndes Mittel;
- ein Phasenumwandlungsmittel oder ein, einen Phasenübergang oder eine Phasentransformation verbesserndes Mittel;
- ein Mittel zur Verbesserung der Trennung von Mikrophasenstrukturen;
- ein Weichmacher oder ein, die plastischen und/oder elastischen Eigenschaften verbesserndes Mittel;
- ein Copolymerisationsmittel oder ein, die Copolymerisation verbesserndes Mittel;
- ein Polymerisationsregulierungsmittel oder ein Mittel zur Verbesserung der Polymerisationsgradeinstellung;
- ein Stabilisierungsmittel oder ein, die Stabilisierung verbesserndes Mittel;
- ein Antioxidantium bzw. Oxidationsschutzmittel oder ein Oxidationsvorgänge verhinderndes Mittel;
- ein Verbesserungsmittel für kristalline und/oder amorphe Materialien;
- ein, das Fließverhalten bzw. die Fluidität verbesserndes Mittel;
- ein Erweichungsmittel, Enthärtungsmittel oder Weichspüler, oder ein Verbesserungsmittel für Erweichungsmittel, Enthärtungsmittel und Weichspüler und/oder ein, die Biegsamkeit, Dehnbarkeit, Anpassungsfähigkeit und Elastizität verbesserndes Mittel;
- ein Modulationssteuermittel und/oder ein, die Fluoreszenzstrahlungswellenlänge und/oder die Erregungsstrahlungswellenlänge von Pigmenten, von Anstrich- und Beschichtungsmitteln, von Farben, von Anstrichstoffen und von Färbemitteln verbesserndes Mittel;
- ein, die funktionalen und/oder physikalischen Eigenschaften von niedermolekularen Substanzen verbesserndes Mittel;
- ein, die physikalischen Eigenschaften und Funktionen von makromolekularen Substanzen verbesserndes Mittel; und/oder
- ein, die physikalischen Eigenschaften verbesserndes Mittel für makromolekulare Verbundwerkstoffe, Kompositmaterialien und für funktionale makromolekulare Verbundwerkstoffe und Kompositmaterialien

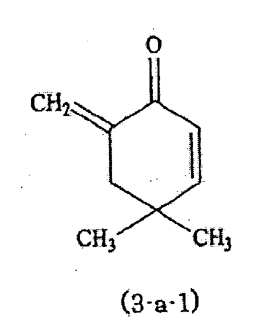

4. Verwendung
einer, der nachstehenden allgemeinen Formel (3-b) entsprechenden
substituierten 4,4-Dimethyl-2-cyclohexen-1-on-Verbindung zur Herstellung eines Präparates/Mittels,
das bei einer Behandlung benutzt wird als:
- ein antibakterielles Mittel;
- ein, gegen Pilzbefall wirksames Mittel;
- ein, gegen ein Virus wirkendes Mittel;
- ein bakterizides und/oder sterilisierendes Mittel;
- ein, gegen Krebs und/oder gegen Tumor(en) wirksames Mittel;
- ein gerinnungshemmendes und/oder die Fibrinbildung hemmendes Mittel;
- ein, einen Thrombuszerfall bewirkendes Mittel;
- ein, den (Fett-, Zucker-, Protein-)Stoffwechsel verbesserndes Mittel;
- ein, die Wundheilung und Epithelbildung (einschließlich des Haarwachstum wiederherstellendes) förderndes Mittel;
- ein Mittel gegen Arteriosklerose, ein, die Antigen-Antikörper-Bildungsreaktion beeinflussendes Mittel und/oder ein, bei Transplantationen das Organ und das Gewebe schützendes Mittel;
- ein Antiseptikum und/oder Schutzmittel;
- ein Markierungsmittel, das an wenigstens einer Substiuentenstelle mit einer (Isotopen-)Markierungssubstanz versehen ist, die eine Zielposition für ein, diese Zielposition suchendes Molekül markiert;
- ein Reduktionsmittel;
- ein, freie Radikale unschädlich machendes Mittel;
- ein Entschwefelungsmittel;
- ein Depolymerisierungsmittel;
- ein Mittel zur Verbesserung funktionaler und/oder physikalischer Eigenschaften von oberflächenaktiven Stoffen bzw. Tensiden;
- ein spermazides Mittel und/oder empfängnisverhütendes Mittel, je zur äußerlichen Anwendung;
- ein, die Konformation von Saccharid-Ketten veränderndes Mittel;
- ein Phasenumwandlungsmittel oder ein, einen Phasenübergang oder eine Phasentransformation verbesserndes Mittel;
- ein Mittel zur Verbesserung der Trennung von Mikrophasenstrukturen;
- ein Weichmacher oder ein, die plastischen und/oder elastischen Eigenschaften verbesserndes Mittel;
- ein Copolymerisationsmittel oder ein, die Copolymerisation verbesserndes Mittel;
- ein Polymerisationsregulierungsmittel oder ein Mittel zur Verbesserung der Polymerisationsgradeinstellung;
- ein Stabilisierungsmittel oder ein, die Stabilisierung verbesserndes Mittel;
- ein Antioxidantium bzw. Oxidationsschutzmittel oder ein Oxidationsvorgänge verhinderndes Mittel;
- ein Verbesserungsmittel für kristalline und/oder amorphe Materialien;
- ein, das Fließverhalten bzw. die Fluidität verbesserndes Mittel;
- ein Erweichungsmittel, Enthärtungsmittel oder Weichspüler, oder ein Verbesserungsmittel für Erweichungsmittel, Enthärtungsmittel und Weichspüler und/oder ein, die Biegsamkeit, Dehnbarkeit, Anpassungsfähigkeit und Elastizität verbesserndes Mittel;
- ein Modulationssteuermittel und/oder ein, die Fluoreszenzstrahlungswellenlänge und/oder die Erregungsstrahlungswellenlänge von Pigmenten, von Anstrich- und Beschichtungsmitteln, von Farben, von Anstrichstoffen und von Färbemitteln verbesserndes Mittel;
- ein, die funktionalen und/oder physikalischen Eigenschaften von niedermolekularen Substanzen verbesserndes Mittel;
- ein, die physikalischen Eigenschaften und Funktionen von makromolekularen Substanzen verbesserndes Mittel; und/oder
- ein, die physikalischen Eigenschaften verbesserndes Mittel für makromolekulare Verbundwerkstoffe, Kompositmaterialien und für funktionale makromolekulare Verbundwerkstoffe und Kompositmaterialien 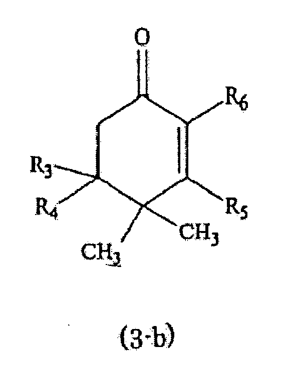
wobei:
(I) R3, R4, R5 und R6 stehen - unabhängig voneinander - für ein Wasserstoffatom, für ein Halogenatom, für eine C₁₋₆-Alkylgruppe, für eine Amidinogruppe, für eine C₃₋₈-Cycloalkylgruppe, für eine C₁₋₆₋Alkoxy-C₁₋₆-alkylgruppe, für eine Arylgruppe, für eine Allylgruppe, für eine Aralkylgruppe, in der eine oder mehrere C₁₋₆-Alkylgruppe(n) an einen aromatischen Ring gebunden sind, der seinerseits ausgewählt ist aus einem Benzol-, Naphthalin- oder Anthracen-Ring, oder für eine C₁₋₆₋Alkylengruppe, für eine Benzoylgruppe, für eine Cinnamylgruppe, für eine Cinnamoylgruppe oder für eine Furoylgruppe;
(II) ein oder mehrere Substituent(en) R3 und R4 und/oder ein oder mehrere Substituent(en) R5 und R6 können eine substituierte oder nicht-substituierte Cyclopentylgruppe, eine substituierte oder nicht-substituierte Cyclohexylgruppen oder eine substituierte oder nicht substituierte Naphthylgruppen sein;
(III) R5 und R6 können durch Verknüpfung mit einer anderen kondensierten polycyclischen Kohlenwasserstoffverbindung oder mit einer heterocyclischen Verbindung einen Ring bilden;
(IV) ein oder mehrere Substiuent(en) R3, R4, R5 und R6 können mit einem oder mehreren Substituent(en) substituiert sein, der/die ihrerseits ausgewählt sind aus einer Gruppe, die umfasst: ein Halogenatom, eine Cyanogruppe, eine geschützte oder ungeschützte Carboxylgruppe, eine geschützte oder ungeschützte Hydroxylgruppe, eine geschützte oder ungeschützte Aminogruppe, eine C₁₋₆-Alkylgruppe, eine C₁₋₆₋Alkoxygruppe, eine C₁₋₇-Alkoxy-carbonylgruppe, eine Arylgruppe, eine C₃₋₆-Cycloalkylgruppe, eine C₁₋₆-Acylaminogruppe, eine C₁₋₆₋Acyloxygruppe, eine C₂₋₆-Alkenylgruppe, eine C₁₋₆-Trihalogen-alkylgruppe, eine C₁₋₆-Alkylaminogruppe und eine C₁₋₆₋Dialkylaminogruppe;
(V) R5 kann substituiert sein mit einem oder mehreren Substituent(en), der/die ihrerseits ausgewählt sind aus einer Gruppe, die umfasst: ein Halogenatom, eine C₁₋₆-Alkylgruppe, eine geschützte oder ungeschützte Carboxylgruppe, eine geschützte oder ungeschützte Hydroxylgruppe, eine geschützte oder ungeschützte Aminogruppe, eine geschützte oder ungeschützte C₁₋₆-Alkylaminogruppe, eine geschützte oder ungeschützte C₁₋₆-Aminoalkylgruppe, eine geschützte oder ungeschützte C₁₋₆-Alkylamino-C₁₋₆-alkylgruppe, eine geschützte oder ungeschützte Hydroxyalkylgruppe und eine C₃₋₆-Cycloalkylaminogruppe;
(VI) weiterhin mit den folgenden Maßgaben:
sofern es sich bei einem oder mehreren Substituent(en) R3, R4, R5 und R6 um Alkylgruppen handelt, können das/die endständige(n) Ende(n) der Alkylgruppe(n) ihrerseits mit C₃₋₈-Cycloalkylgruppen substituiert sein;
die in den vorstehenden Absätzen (i) und (iv) genannte Arylgruppe ist eine Phenyl-, Tolyl-, Xylyl- oder Napthyl-gruppe;
die im vorstehenden Absatz (ii) genannte substituierte Cyclopentylgruppe ist eine Cyclopentylaminogruppe oder eine Cyclopentylcarbinolgruppe;
die im vorstehenden Absatz (ii) genannte substituierte Cyclohexylgruppe ist eine Cyclohexylaminogruppe, eine Cyclohexylaldehydgruppe oder eine Cyclohexyl-essigsäure-gruppe;
die im vorstehenden Absatz (ii) genannte substiuierte Napthylgruppe ist eine Naphthylaminogruppe oder eine Napthylamino-sulfonsäuregruppe;
die im vorstehenden Absatz (iii) genannte kondensierte polycyclische Kohlenwasserstoffverbindung ist Pentalen, Inden, Naphthalin, Azulen, Heptalen, Biphenylen, Indacen, Acenapthylen, Fluoren, Phenalen, Phenanthren, Anthracen, Pentacen, Hexacen, Dibenzophenanthren, 1-H-Cyclopentacycloocten oder Benzocycloocten; und
die im vorstehenden Absatz (iii) genannte heterocyclische Verbindung kann Furan, Thiophen, Pyrrol, γ-Pyran, γ-Thiopyran, Pyridin, Thiazol, Imidazol, Pyrimidin, Indol oder Chinolin sein.

## Revendications

1. L'utilisation
d'un composé substitué de 4,4-diméthyle-2-cyclohexène-1-one de la formule générale suivante (2)
pour la production d'une préparation
utilisée pour le traitement comme
- agent antibactérien ;
- agent antifongique ;
- agent antiviral ;
- agent bactéricide et/ou stérilisant
- agent anticancer et/ou antitumeur ;
- agent anticoagulant et/ou antifibrinolytique ;
- agent thrombolytique ;
- agent améliorant le métabolisme (lipides, glucides, protéines) ;
- agent favorisant la guérison de plaies et la croissance de l'épithélium y compris la restauration des cheveux ;
- agent agissant contre l'artériosclérose et comme agent agissant contre la réaction antigène-anticorps et/ou comme agent protecteur de l'organe et du tissu dans les greffes ;
- agent antiseptique et/ou protecteur ;
- agent marqueur dont au moins un substituant comprend un marqueur (isotope) affichant la position cible d'une molécule recherchant cette cible;
- agent réducteur ;
- agent absorbant les radicaux libres ;
- agent désulfurant ;
- agent dépolymérisant ;
- agent améliorant les caractéristiques fonctionnelles et/ou physiques de surfactifs respectivement de tensioactifs ;
- spermicide et/ou contraceptif, chacun étant administré par voie cutanée ;
- agent modifiant la conformation de chaînes de saccharides ;
- agent de transition de phase ou comme agent améliorant la transition de phase ;
- agent améliorant la séparation de structures de microphase ;
- agent plastifiant ou améliorant la plasticité et/ou l'élasticité :
- agent de copolymérisation ou améliorant la copolymérisation ;
- agent réglant la polymérisation ou comme agent améliorant le réglage du degré de polymérisation ;
- agent de stabilisation ou comme agent améliorant la stabilisation ;
- un agent antioxydant ou comme agent prévenant ou empêchant l'oxydation ;
- agent améliorant les matériaux cristallins et/ou amorphes ;
- agent améliorant la fluidité ;
- agent adoucissant ou assouplissant ou comme agent améliorant des adoucisseurs ou assouplissants et/ou comme agent améliorant la flexibilité ;
- agent de modulation et/ou comme agent améliorant la longueur d'onde de fluorescence et/ou la longueur d'onde d'excitation de pigments, de peintures, de colorants et d'enduits ;
- agent améliorant les caractéristiques fonctionnelles et/ou physiques de substances de faible poids moléculaire ;
- agent améliorant les caractéristiques physiques et les fonctions de substances macromoléculaires ; et/ou comme
- agent améliorant les caractéristiques physiques de matériaux composites macromoléculaires, de matières premières composites et de matériaux composites macromoléculaires et matières premières composites fonctionnels ; 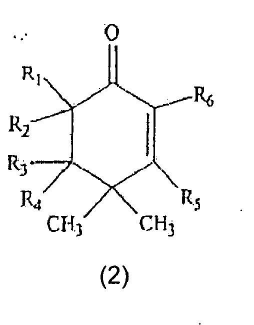
dans laquelle
(I) les deux R1 et R2 ensemble représentent un groupe méthylène ; et/ou R1, R2, R3, R4, R5 et R6 représentent indépendamment l'un de l'autre un atome hydrogène, un atome halogène, un groupe C₁₋₆-alkyle, un groupe amidino, un groupe C₃₋₈-cycloalkyle, un groupe C₁₋₆-alkoxy-C₁₋₆₋alkyle, un groupe aryle, un groupe allyle, un groupe aralkyle, dans lequel un ou plusieurs groupe(s) C₁₋₆-alkyle est/sont lié(s) à un cycle aromatique ce dernier étant choisi parmi un cycle de benzène, de naphtalène ou d'anthracène, ou un groupe C₁₋₆-alkylène, un groupe benzoyle, un groupe cinnamyle, un groupe cinnamoyle ou un groupe furoyle ;
(II) un ou plusieurs substituant(s) R1, R2, R3 et R4 et/ou un ou plusieurs substituant(s) R5 et R6 peu(ven)t représenter un groupe cyclopentyle substitué ou non substitué, un groupe cyclohexyle substitué ou non substitué ou un groupe naphtyle substitué ou non substitué ;
(III) R5 et R6 peuvent former un cycle par liaison avec un autre composé hydrocarbure polycyclique condensé ou avec un composé hétérocyclique.
(IV) un ou plusieurs substituant(s) R3, R4, R5 et R6 peu(ven)t être substitué(s) par un ou plusieurs substituant(s), le(s)quel(s) est/sont choisi(s) parmi un groupe comprenant : un atome halogène, un groupe cyano, un groupe carboxyle protégé ou non protégé, un groupe hydroxyle protégé ou non protégé, un groupe amino protégé ou non protégé, un groupe C₁₋₆-alkyle, un groupe C₁₋₆-alkoxy, un groupe C₁₋₇₋alkoxy-carbonyle, un groupe aryle, un groupe C₃₋₆-cycloalkyle, un groupe C₁₋₆-acylamino, un groupe C₁₋₆-acyloxy, un groupe C₂₋₆-alkenyle, un groupe C₁₋₆-trihalogéno-alkyle, un groupe C₁₋₆-alkylamino et un groupe C₁₋₆-dialkylamino ;
(V) R2 et/ou R5 peu(ven)t être substitué(s) par un ou plusieurs substituant(s), le(s)quel(s) est/sont choisi(s) parmi un groupe comprenant : un atome halogène, un groupe C₁₋₆-alkyle, un groupe carboxyle protégé ou non protégé, un groupe hydroxyle protégé ou non protégé, un groupe amino protégé ou non protégé, un groupe C₁₋₆-alkylamino protégé ou non protégé, un groupe C₁₋₆-aminoalkyle protégé ou non protégé, un groupe C₁₋₆-alkylamino-C₁₋₆-alkyle protégé ou non protégé, un groupe hydroxyalkyle protégé ou non protégé et un groupe C₃₋₆₋cycloalkylamino ;
(VI) et répondant en outre à ce qui suit :
si un ou plusieurs substituant(s) R3, R4, R5 et R6 représente(nt) un/des groupe(s) alkyle, l'extrémité / les extrémités terminale(s) du/des groupe(s) alkyle peu(ven)t être substituée(s) par des groupes C₃₋₈₋cycloalkyle ;
le groupe aryle mentionné aux paragraphes (I) et (IV) est un groupe phényle, tolyle, xylyle ou naphtyle ;
le groupe cyclopentyle substitué mentionné au paragraphe (II) est un groupe cyclopentylamino ou un groupe cyclopentylcarbinole ;
le groupe cyclohexyle substitué mentionné au paragraphe (II) est un groupe cyclohexylamino, un groupe cyclohexylaldéhyde ou un groupe d'acide acétique de cyclohexyle ; et
le groupe naphtyle substitué mentionné au paragraphe (II) est un groupe naphtylamino ou un groupe d'acide de naphtylamino-sulfonique.
le composé hydrocarbure polycyclique condensé mentionné au paragraphe (III) est le pentalène, l'indène, le naphtalène, l'azulène, l'heptalène, le biphénylène, l'indacène, l'acénaphtylène, le fluorène, le phénalène, le phénantrène, l'anthracène, le pentacène, l'hexacène, le dibenzophénanthrène, le 1-H-cyclopenta-cyclooctène ou le benzocyclooctène ; et
le composé hétérocyclique mentionné au paragraphe (III) peut être le furane, le thiophène, le pyrrole, le y-pyrane, le y-thiopyrane, la pyridine, le thiazole, l'imidazole, la pyrimidine, l'indole ou la chinoline.

2. L'utilisation
d'un composé substitué de 4,4-diméthyle-2-cyclohexène-1-one de la formule générale suivante (3a)
pour la production d'une préparation
utilisée pour le traitement comme
- agent antibactérien ;
- agent antifongique ;
- agent antiviral ;
- agent bactéricide et/ou stérilisant
- agent anticancer et/ou antitumeur ;
- agent anticoagulant et/ou antifibrinolytique ;
- agent thrombolytique ;
- agent améliorant le métabolisme (lipides, glucides, protéines) ;
- agent favorisant la guérison de plaies et la croissance de l'épithélium y compris la restauration des cheveux ;
- agent agissant contre l'artériosclérose et comme agent agissant contre la réaction antigène-anticorps et/ou comme agent protecteur de l'organe et du tissu dans les greffes ;
- agent antiseptique et/ou protecteur ;
- agent marqueur dont au moins un substituant comprend un marqueur (isotope) affichant la position cible d'une molécule recherchant cette cible ;
- agent réducteur ;
- agent absorbant les radicaux libres ;
- agent désulfurant ;
- agent dépolymérisant ;
- agent améliorant les caractéristiques fonctionnelles et/ou physiques de surfactifs respectivement de tensioactifs ;
- spermicide et/ou contraceptif, chacun étant administré par voie cutanée ;
- agent modifiant la conformation de chaînes de saccharides ;
- agent de transition de phase ou comme agent améliorant la transition de phase ;
- agent améliorant la séparation de structures de microphase ;
- agent plastifiant ou améliorant la plasticité et/ou l'élasticité :
- agent de copolymérisation ou améliorant la copolymérisation ;
- agent réglant la polymérisation ou comme agent améliorant le réglage du degré de polymérisation ;
- agent de stabilisation ou comme agent améliorant la stabilisation ;
- un agent antioxydant ou comme agent prévenant ou empêchant l'oxydation ;
- agent améliorant les matériaux cristallins et/ou amorphes ;
- agent améliorant la fluidité ;
- agent adoucissant ou assouplissant ou comme agent améliorant des adoucisseurs ou assouplissants et/ou comme agent améliorant la flexibilité ;
- agent de modulation et/ou comme agent améliorant la longueur d'onde de fluorescence et/ou la longueur d'onde d'excitation de pigments, de peintures, de colorants et d'enduits ;
- agent améliorant les caractéristiques fonctionnelles et/ou physiques de substances de faible poids moléculaire ;
- agent améliorant les caractéristiques physiques et les fonctions de substances macromoléculaires ; et/ou comme
- agent améliorant les caractéristiques physiques de matériaux composites macromoléculaires, de matières premières composites et de matériaux composites macromoléculaires et matières premières composites fonctionnels ; 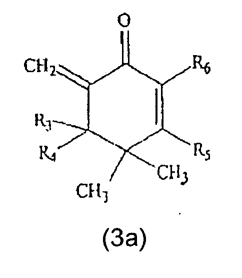
dans laquelle
(I) R3, R4, R5 et R6 représentent indépendamment l'un de l'autre un atome hydrogène, un atome halogène, un groupe C₁₋₆-alkyle, un groupe amidino, un groupe C₃₋₈-cycloalkyle, un groupe C₁₋₆-alkoxy-C₁₋₆-alkyle, un groupe aryle, un groupe allyle, un groupe aralkyle, dans lequel un ou plusieurs groupe(s) C₁₋₆-alkyle est/sont lié(s) à un cycle aromatique ce dernier étant choisi parmi un cycle de benzène, de naphtalène ou d'anthracène, ou un groupe C₁₋₆-alkylène, un groupe benzoyle, un groupe cinnamyle, un groupe cinnamoyle ou un groupe furoyle ;
II) un ou plusieurs substituant(s) R3 et R4 et/ou un ou plusieurs substituant(s) R5 et R6 peu(ven)t représenter un groupe cyclopentyle substitué ou non substitué, un groupe cyclohexyle substitué ou non substitué ou un groupe naphtyle substitué ou non substitué ;
(III) R5 et R6 peuvent former un cycle par liaison avec un autre composé hydrocarbure polycyclique condensé ou avec un composé hétérocyclique.
(IV) un ou plusieurs substituant(s) R3, R4, R5 et R6 peu(ven)t être substitué(s) par un ou plusieurs substituant(s), le(s)quel(s) est/sont choisi(s) parmi un groupe comprenant : un atome halogène, un groupe cyano, un groupe carboxyle protégé ou non protégé, un groupe hydroxyle protégé ou non protégé, un groupe amino protégé ou non protégé, un groupe C₁₋₆-alkyle, un groupe C₁₋₆-alkoxy, un groupe C₁₋₇₋alkoxy-carbonyle, un groupe aryle, un groupe C₃₋₆-cycloalkyle, un groupe C₁₋₆-acylamino, un groupe C₁₋₆-acyloxy, un groupe C₂₋₆-alkenyle, un groupe C₁₋₆-trihalogéno-alkyle, un groupe C₁₋₆-alkylamino et un groupe C₁₋₆-dialkylamino ;
(V) R5 peut être substitué par un ou plusieurs substituant(s), le(s)quel(s) est/sont choisi(s) parmi un groupe comprenant : un atome halogène, un groupe C₁₋₆-alkyle, un groupe carboxyle protégé ou non protégé, un groupe hydroxyle protégé ou non protégé, un groupe amino protégé ou non protégé, un groupe C₁₋₆-alkylamino protégé ou non protégé, un groupe C₁₋₆-aminoalkyle protégé ou non protégé, un groupe C₁₋₆₋alkylamino-C₁₋₆-alkyle protégé ou non protégé, un groupe hydroxyalkyle protégé ou non protégé et un groupe C₃₋₆-cycloalkylamino ;
(VI) et répondant en outre à ce qui suit :
si un ou plusieurs substituant(s) R3, R4, R5 et R6 représente(nt) un/des groupe(s) alkyle, l'extrémité / les extrémités terminale(s) du/des groupe(s) alkyle peu(ven)t être substituée(s) par des groupes C₃₋₈₋cycloalkyle ;
le groupe aryle mentionné aux paragraphes (I) et (IV) est un groupe phényle, tolyle, xylyle ou naphtyle ;
le groupe cyclopentyle substitué mentionné au paragraphe (II) est un groupe cyclopentylamino ou un groupe cyclopentylcarbinole ;
le groupe cyclohexyle substitué mentionné au paragraphe (II) est un groupe cyclohexylamino, un groupe cyclohexylaldéhyde ou un groupe d'acide acétique de cyclohexyle ; et
le groupe naphtyle substitué mentionné au paragraphe (II) est un groupe naphtylamino ou un groupe d'acide de naphtylamino-sulfonique.
le composé hydrocarbure polycyclique condensé mentionné au paragraphe (III) est le pentalène, l'indène, le naphtalène, l'azulène, l'heptalène, le biphénylène, l'indacène, l'acénaphtylène, le fluorène, le phénalène, le phénantrène, l'anthracène, le pentacène, l'hexacène, le dibenzophénanthrène, le 1-H-cyclopenta-cyclooctène ou le benzo-cyclooctène ; et
le composé hétérocyclique mentionné au paragraphe (III) peut être le furane, le thiophène, le pyrrole, le y-pyrane, le y-thiopyrane, la pyridine, le thiazole, l'imidazole, la pyrimidine, l'indole ou la chinoline.

3. L'utilisation
d'un composé substitué de 4,4-diméthyle-2-cyclohexène-1-one de la formule générale suivante (3a-1)
pour la production d'une préparation
utilisée pour le traitement comme
- agent antibactérien ;
- agent antifongique ;
- agent antiviral ;
- agent bactéricide et/ou stérilisant
- agent anticancer et/ou antitumeur ;
- agent anticoagulant et/ou antifibrinolytique ;
- agent thrombolytique ;
- agent améliorant le métabolisme (lipides, glucides, protéines) ;
- agent favorisant la guérison de plaies et la croissance de l'épithélium y compris la restauration des cheveux ;
- agent agissant contre l'artériosclérose et comme agent agissant contre la réaction antigène-anticorps et/ou comme agent protecteur de l'organe et du tissu dans les greffes ;
- agent antiseptique et/ou protecteur ;
- agent marqueur dont au moins un substituant comprend un marqueur (isotope) affichant la position cible d'une molécule recherchant cette cible ;
- agent réducteur ;
- agent absorbant les radicaux libres ;
- agent désulfurant ;
- agent dépolymérisant ;
- agent améliorant les caractéristiques fonctionnelles et/ou physiques de surfactifs respectivement de tensioactifs ;
- spermicide et/ou contraceptif, chacun étant administré par voie cutanée ;
- agent modifiant la conformation de chaînes de saccharides ;
- agent de transition de phase ou comme agent améliorant la transition de phase ;
- agent améliorant la séparation de structures de microphase ;
- agent plastifiant ou améliorant la plasticité et/ou l'élasticité :
- agent de copolymérisation ou améliorant la copolymérisation ;
- agent réglant la polymérisation ou comme agent améliorant le réglage du degré de polymérisation ;
- agent de stabilisation ou comme agent améliorant la stabilisation ;
- un agent antioxydant ou comme agent prévenant ou empêchant l'oxydation ;
- agent améliorant les matériaux cristallins et/ou amorphes ;
- agent améliorant la fluidité ;
- agent adoucissant ou assouplissant ou comme agent améliorant des adoucisseurs ou assouplissants et/ou comme agent améliorant la flexibilité ;
- agent de modulation et/ou comme agent améliorant la longueur d'onde de fluorescence et/ou la longueur d'onde d'excitation de pigments, de peintures, de colorants et d'enduits ;
- agent améliorant les caractéristiques fonctionnelles et/ou physiques de substances de faible poids moléculaire ;
- agent améliorant les caractéristiques physiques et les fonctions de substances macromoléculaires ; et/ou comme
- agent améliorant les caractéristiques physiques de matériaux composites macromoléculaires, de matières premières composites et de matériaux composites macromoléculaires et matières premières composites fonctionnels ;

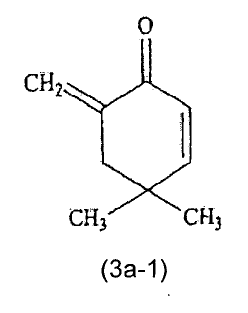

4. L'utilisation
d'un composé substitué de 4,4-diméthyle-2-cyclohexène-1-one de la formule générale suivante (3b)
pour la production d'une préparation
utilisée pour le traitement comme
- agent antibactérien ;
- agent antifongique ;
- agent antiviral ;
- agent bactéricide et/ou stérilisant
- agent anticancer et/ou antitumeur ;
- agent anticoagulant et/ou antifibrinolytique ;
- agent thrombolytique ;
- agent améliorant le métabolisme (lipides, glucides, protéines) ;
- agent favorisant la guérison de plaies et la croissance de l'épithélium y compris la restauration des cheveux ;
- agent agissant contre l'artériosclérose et comme agent agissant contre la réaction antigène-anticorps et/ou comme agent protecteur de l'organe et du tissu dans les greffes ;
- agent antiseptique et/ou protecteur ;
- agent marqueur dont au moins un substituant comprend un marqueur (isotope) affichant la position cible d'une molécule recherchant cette cible;
- agent réducteur ;
- agent absorbant les radicaux libres ;
- agent désulfurant ;
- agent dépolymérisant ;
- agent améliorant les caractéristiques fonctionnelles et/ou physiques de surfactifs respectivement de tensioactifs ;
- spermicide et/ou contraceptif, chacun étant administré par voie cutanée ;
- agent modifiant la conformation de chaînes de saccharides ;
- agent de transition de phase ou comme agent améliorant la transition de phase ;
- agent améliorant la séparation de structures de microphase ;
- agent plastifiant ou améliorant la plasticité et/ou l'élasticité :
- agent de copolymérisation ou améliorant la copolymérisation ;
- agent réglant la polymérisation ou comme agent améliorant le réglage du degré de polymérisation ;
- agent de stabilisation ou comme agent améliorant la stabilisation ;
- un agent antioxydant ou comme agent prévenant ou empêchant l'oxydation ;
- agent améliorant les matériaux cristallins et/ou amorphes ;
- agent améliorant la fluidité ;
- agent adoucissant ou assouplissant ou comme agent améliorant des adoucisseurs ou assouplissants et/ou comme agent améliorant la flexibilité ;
- agent de modulation et/ou comme agent améliorant la longueur d'onde de fluorescence et/ou la longueur d'onde d'excitation de pigments, de peintures, de colorants et d'enduits ;
- agent améliorant les caractéristiques fonctionnelles et/ou physiques de substances de faible poids moléculaire ;
- agent améliorant les caractéristiques physiques et les fonctions de substances macromoléculaires ; et/ou comme
- agent améliorant les caractéristiques physiques de matériaux composites macromoléculaires, de matières premières composites et de matériaux composites macromoléculaires et matières premières composites fonctionnels ; 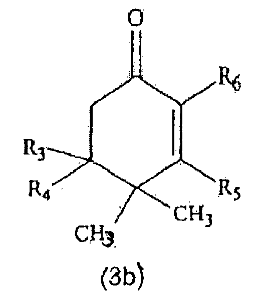
dans laquelle
(I) R3, R4, R5 et R6 représentent indépendamment l'un de l'autre un atome hydrogène, un atome halogène, un groupe C₁₋₆-alkyle, un groupe amidino, un groupe C₃₋₈-cycloalkyle, un groupe C₁₋₆-alkoxy-C₁₋₆-alkyle, un groupe aryle, un groupe allyle, un groupe aralkyle, dans lequel un ou plusieurs groupe(s) C₁₋₆-alkyle est/sont lié(s) à un cycle aromatique ce dernier étant choisi parmi un cycle de benzène, de naphtalène ou d'anthracène, ou un groupe C₁₋₆-alkylène, un groupe benzoyle, un groupe cinnamyle, un groupe cinnamoyle ou un groupe furoyle ;
(II) un ou plusieurs substituant(s) R3 et R4 et/ou un ou plusieurs substituant(s) R5 et R6 peu(ven)t représenter un groupe cyclopentyle substitué ou non substitué, un groupe cyclohexyle substitué ou non substitué ou un groupe naphtyle substitué ou non substitué ;
(III) R5 et R6 peuvent former un cycle par liaison avec un autre composé hydrocarbure polycyclique condensé ou avec un composé hétérocyclique.
(IV) un ou plusieurs substituant(s) R3, R4, R5 et R6 peu(ven)t être substitué(s) par un ou plusieurs substituant(s), le(s)quel(s) est/sont choisi(s) parmi un groupe comprenant : un atome halogène, un groupe cyano, un groupe carboxyle protégé ou non protégé, un groupe hydroxyle protégé ou non protégé, un groupe amino protégé ou non protégé, un groupe C₁₋₆-alkyle, un groupe C₁₋₆-alkoxy, un groupe C₁₋₇₋alkoxy-carbonyle, un groupe aryle, un groupe C₃₋₆-cycloalkyle, un groupe C₁₋₆-acylamino, un groupe C₁₋₆-acyloxy, un groupe C₂₋₆-alkenyle, un groupe C₁₋₆-trihalogéno-alkyle, un groupe C₁₋₆-alkylamino et un groupe C₁₋₆-dialkylamino ;
(V) R5 peut être substitué par un ou plusieurs substituant(s), le(s)quel(s) est/sont choisi(s) parmi un groupe comprenant : un atome halogène, un groupe C₁₋₆-alkyle, un groupe carboxyle protégé ou non protégé, un groupe hydroxyle protégé ou non protégé, un groupe amino protégé ou non protégé, un groupe C₁₋₆-alkylamino protégé ou non protégé, un groupe C₁₋₆-aminoalkyle protégé ou non protégé, un groupe C₁₋₆₋alkylamino-C₁₋₆-alkyle protégé ou non protégé, un groupe hydroxyalkyle protégé ou non protégé et un groupe C₃₋₆-cycloalkylamino ;
(VI) et répondant en outre à ce qui suit :
si un ou plusieurs substituant(s) R3, R4, R5 et R6 représente(nt) un/des groupe(s) alkyle, l'extrémité / les extrémités terminale(s) du/des groupe(s) alkyle peu(ven)t être substituée(s) par des groupes C₃₋₈₋cycloalkyle ;
le groupe aryle mentionné aux paragraphes (I) et (IV) est un groupe phényle, tolyle, xylyle ou naphtyle ;
le groupe cyclopentyle substitué mentionné au paragraphe (II) est un groupe cyclopentylamino ou un groupe cyclopentylcarbinole ;
le groupe cyclohexyle substitué mentionné au paragraphe (II) est un groupe cyclohexylamino, un groupe cyclohexylaldéhyde ou un groupe d'acide acétique de cyclohexyle ; et
le groupe naphtyle substitué mentionné au paragraphe (II) est un groupe naphtylamino ou un groupe d'acide de naphtylamino-sulfonique.
paragraphe (III) est le pentalène, l'indène, le naphtalène, l'azulène, l'heptalène, le biphénylène, l'indacène, l'acénaphtylène, le fluorène, le phénalène, le phénantrène, l'anthracène, le pentacène, l'hexacène, le dibenzophénanthrène, le 1-H-cyclopenta-cyclooctène ou le benzo-cyclooctène ; et
le composé hétérocyclique mentionné au paragraphe (III) peut être le furane, le thiophène, le pyrrole, le y-pyrane, le y-thiopyrane, la pyridine, le thiazole, l'imidazole, la pyrimidine, l'indole ou la chinoline.
